# EUROPÄISCHE PATENTSCHRIFT

(11) **EP 0 521 334 B1**
(45) Veröffentlichungstag und Bekanntmachung des Hinweises auf die Patenterteilung: **09.09.1998**
(21) Anmeldenummer: 92110119.2
(22) Anmeldetag: 16.06.1992
(51) Int. Cl.: C07D 207/38, C07D 209/54, C07F 9/572, A01N 43/36, A01N 57/08, A01N 57/24

(54) **Substituierte 1-H-3-Aryl-pyrrolidin-2,4-dion-Derivate, deren Herstellung und deren Verwendung als Insektizide, Akarizide und Herbizide**
Substituted 1-H-3-aryl-pyrrolidin-2,4-dione derivatives, their preparation and their use as insecticides, acaricides and herbicides
Dérivés substitués de la 1-H-3-aryl-pyrrolidine-2,4-dione, leur préparation et leur utilisation comme insecticides, acaricides et herbicides

(30) Priorität: 28.06.1991 DE 4121365
(43) Veröffentlichungstag der Anmeldung: 07.01.1993
(73) Patentinhaber: BAYER AG, 51368 Leverkusen (DE)
(72) Erfinder: Fischer, Reiner, Dr., W-4019 Monheim 2 (DE); Krüger, Bernd-Wieland, Dr., W-5060 Bergisch-Gladbach 2 (DE); Bretschneider, Thomas, Dr., W-5200 Siegburg (DE); Erdelen, Christoph, Dr., W-5653 Leichlingen (DE); Wachendorff-Neumann, Ulrike, Dr., W-4019 Monheim (DE); Lürssen, Klaus, Dr., W-5060 Bergisch Gladbach 2 (DE); Santel, Hans-Joachim, Dr., W-5090 Leverkusen (DE); Schmidt, Robert R., Dr., W-5060 Bergisch Gladbach 2 (DE)

(56) Entgegenhaltungen:
- EP-A- 355 599
- EP-A- 377 893
- EP-A- 0 415 185
- EP-A- 0 442 077
- EP-A- 0 456 063
- DE-A- 4 032 090
- US-A- 4 104 043

## Beschreibung

Die Erfindung betrifft neue substituierte 3-Aryl-pyrrolidin-2,4-dion-Derivate, mehrere Verfahren zu ihrer Herstellung und ihre Verwendung als Insektizide, Akarizide und Herbizide.

Von 3-Acyl-pyrrolidin-2,4-dionen sind pharmazeutische Eigenschaften vorbeschrieben (S. Suzuki et. al. Chem. Pharm. Bull. 15 1120 (1967)). Weiterhin wurden N-Phenylpyrrolidin-2,4-dione von R. Schmierer und H. Mildenberger Liebigs Ann. Chem. 1985 1095 synthetisiert. Eine biologische Wirksamkeit dieser Verbindungen wurde nicht beschrieben.

In EP-A-0 262 399 werden ähnlich strukturierte Verbindungen (3-Aryl-pyrrolidin-2,4-dione) offenbart, von denen jedoch keine herbizide, insektizide oder akarizide Wirkung bekannt geworden ist.

In DE-A-3 525 109 werden ähnlich strukturierte 1-H-3-Arylpyrrolidin-2,4-dione offenbart, die als Zwischenprodukte für Farbstoffsynthesen verwendet werden.

In EP-0 456 063, EP-442 077, EP-0 415 185, DE-4 032 090, EP-0 355 599 und EP-377 893 werden 3-Aryl-pyrrolidin-2,4-dion-Derivate mit insektiziden und/oder akariziden und/oder herbiziden Eigenschaften beschrieben.

Die Aufgabe der Erfindung bestand nun darin, neue insektizid und/oder akarizid und/oder herbizid wirksame Verbindungen mit gegenüber den bekannten Verbindungen verbesserter Wirkung bereitzustellen.

Es wurden nun neue substituierte 3-Aryl-pyrrolidin-2,4-dion-Derivate gefunden, die durch die Formel (I) dargestellt sind, 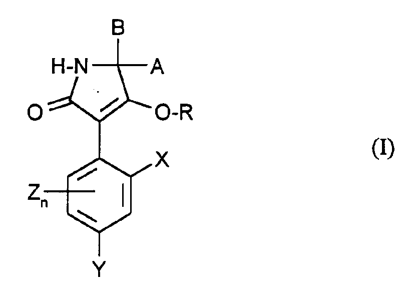 in welcher
X für C₁-C₆-Alkyl, Halogen, C₁-C₆-Alkoxy steht,
Y für Wasserstoff, C₁-C₆-Alkyl, Halogen, C₁-C₆-Alkoxy, C₁-C₃-Halogenalkyl steht,
Z für C₁-C₆-Alkyl, Halogen, C₁-C₆-Alkoxy steht,
n für eine Zahl von 0 bis 3 steht,
A für Wasserstoff oder gegebenenfalls durch Halogen substituiertes geradkettiges oder verzweigtes Ci-C12-Alkyl, C₃-C₈-Alkenyl, C₃-C₈-Alkinyl, C₁-C₁₀-Alkoxy-C₂-C₈-alkyl, C₁-c₈-Polyalkoxy-C₂-C₈-alkyl, C₁-C₁₀-Alkylthio-C₂-C₈- alkyl, Cycloalkyl mit 3 bis 8 Ringatomen, das durch Sauerstoff und/oder Schwefel unterbrochen sein kann,
B für Wasserstoff, geradkettiges oder verzweigtes C₁-C₁₂-Alkyl, C₁-C₈-Alkoxyalkyl steht,

oder worin
A und B gemeinsam mit dem Kohlenstoffatom, an das sie gebunden sind, einen 3-bis 8-gliedrigen gesättigten oder ungesättigten Ring bilden, der durch Sauerstoff und/oder Schwefel unterbrochen und durch gegebenenfalls halogeniertes Alkyl, Alkoxy, Phenyl und Halogen substituiert sein kann,
R für die Gruppen 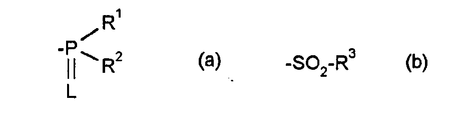 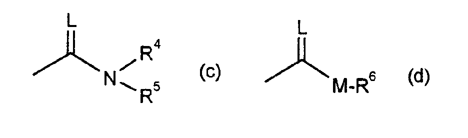 steht,
   in welchen
L und M jeweils für Sauerstoff oder Schwefel stehen und wobei L und M nicht gleichzeitig für Sauerstoff stehen,
R¹, R² und R³ unabhängig voneinander für gegebenenfalls durch Halogen substituiertes C₁-C₈-Alkyl, C₁-C₈-Alkoxy, C₁-C₈-Alkylamino, Di(C₁-C₈)-alkylamino, C₁-C₈-Alkylthio, C₂-C₅-Alkenylthio, C₂-C₅-Alkinylthio, C₃-C₇-Cycloalkylthio, für gegebenenfalls durch Halogen, Nitro, Cyano, Cᵢ-C₄-Alkoxy, C₁-C₄-Halogenalk- oxy, C₁-C₄-Alkylthio, C₁-C₄-Halogenalkylthio, C₁-C₄-Alkyl, C₁-C₄-Halogenalkyl substituiertes Phenyl, Benzyl, Phenoxy oder Phenylthio stehen,
R⁴ und R⁵ unabhängig voneinander für gegebenenfalls durch Halogen substituiertes C₁-C₂₀-Alkyl, C₁-C₂₀-Alkoxy, C₂-C₈-Alkenyl, C₁-C₂₀-Alkoxy-C₁-C₂₀-alkyl, für gegebenenfalls durch Halogen, C₁-C₂₀-Halogenalkyl, C₁-C₂₀-Alkyl oder C₁-C₂₀-Alkoxy substituiertes Phenyl, für gegebenenfalls durch Halogen, C₁-C₂ₒ-Alkyl, C₁-C₂₀-Halogenalkyl oder C₁-C₂₀-Alkoxy substituiertes Benzyl stehen oder zusammen für einen gegebenenfalls durch Sauerstoff unterbrochenen C₂-Cₛ-Alkylenring stehen,
R⁶ für gegebenenfalls durch Halogen substituiertes C₁-C₂₀-Alkyl, das durch Sauerstoff unterbrochen sein kann, für gegebenenfalls durch Halogen, C₁-C₂₀-Halogenalkyl, C₁-C₂₀-Alkoxy substituiertes Phenyl, für gegebenenfalls durch Halogen, C₁-C₂₀-Halogenalkyl oder C₁-C₂₀-Alkoxy substituiertes Benzyl, für C₂-C₈-Alkenyl oder für C₂-C₅-Albnyl steht,
   sowie die enantiomerenreinen Formen von Verbindungen der Formel (I).

Unter Einbeziehung der verschiedenen Bedeutungen (a), (b), (c) und (d) der Gruppe R der allgemeinen Formel (I) ergeben sich folgende hauptsächlichen Strukturen (la) bis (Id): 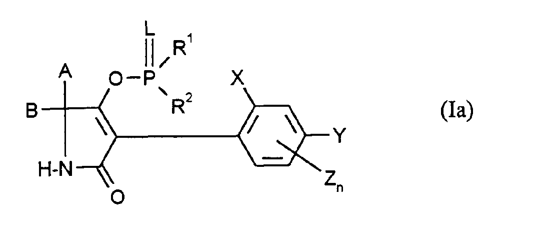 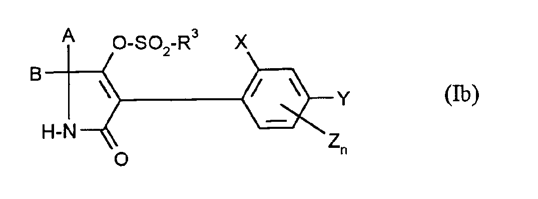 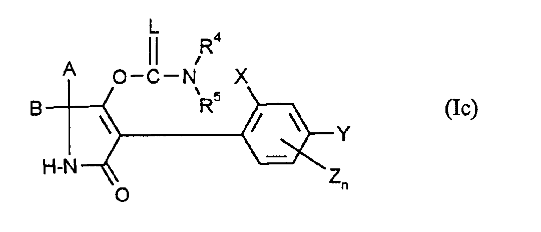 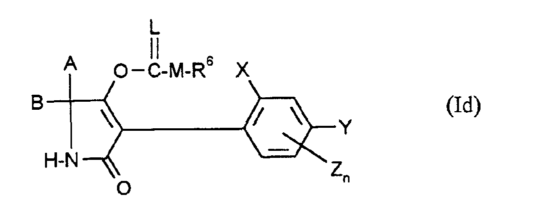 wobei
_{A}, _{B}, _{L}, M, X, Y, Zₙ, R¹, R², R³, R⁴, R⁵ und R⁶ die oben angegebenen Bedeutungen besitzen.

Weiterhin wurde gefunden, daß man 3-Aryl-pyrrolidon-2,4-dion-Derivate der Formel (la) 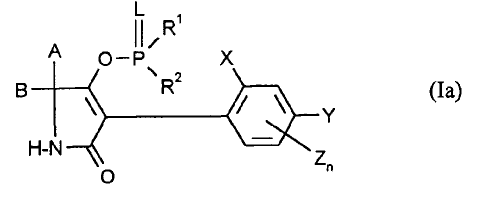 in welcher
A, B, L, X, Y, Z, R¹, R² und n die oben angegebene Bedeutung haben,

erhält, wenn man
A) 3-Aryl-pyrrolidin-2,4-dione der Formel (II) bzw. deren Enole 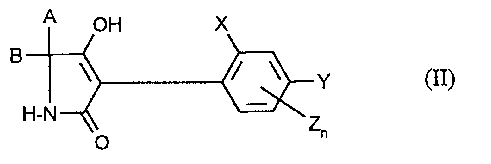 in welcher
   A, B, X, Y, Z und n die oben angegebene Bedeutung haben,
      mit Phosphorverbindungen der allgemeinen Formel (III) 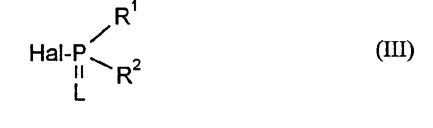 in welcher
   L, R¹ und R² die oben angegebene Bedeutung haben und
      Hal für Halogen, insbesondere Chlor und Brom steht,
      gegebenenfalls in Gegenwart eines Verdünnungsmittels, gegebenenfalls in Gegenwart eines Säurebindemittels und gegebenenfalls in Gegenwart eines Phasentransferkatalysators umsetzt.
B) Außerdem wurde gefunden, daß man Verbindungen der Formel (Ib) 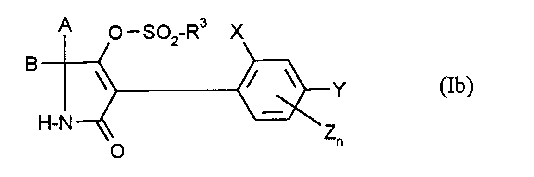 in welcher
   A, B, X, Y, Z, R³ und n die oben angegebene Bedeutung haben,
      erhält, wenn man Verbindungen der Formel (II) 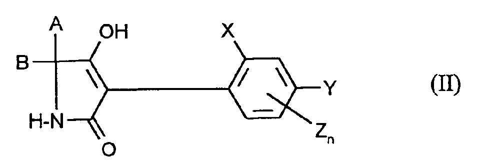 in welcher
   A, B, X, Y, Z und n die oben angegebene Bedeutung haben,
      mit Sulfonsäurechloriden der allgemeinen Formel (IV) 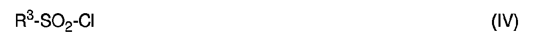 in welcher
   R³ die oben angegebene Bedeutung hat,

   gegebenenfalls in Gegenwart eines Verdünnungsmittels und gegebenenfalls in Gegenwart eines Säurebindemittels,
   umsetzt.
C) Ferner wurde gefunden, daß man Verbindungen der Formel (Ic) 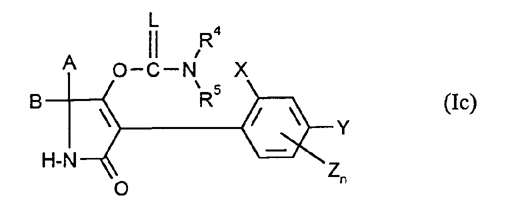 in welcher
   A, B, L, X, Y, Z, R⁴, R⁵ und n die oben angegebene Bedeutung haben,
      erhält, wenn man Verbindungen der Formel (II) 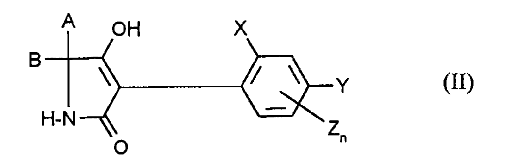 in welcher
   A, B, X, Y, Z und n die oben angegebene Bedeutung haben,
      wenn man
      a) mit Isocyanaten der allgemeinen Formel (V) 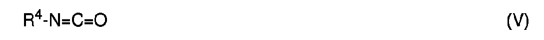 in welcher
         R⁴ die oben angegebene Bedeutung hat,

         gegebenenfalls in Gegenwart eines Verdünnungsmittels und gegebenenfalls in Gegenwart eines Katalysators oder
      β) mit Carbamidsäurechloriden oder Thiocarbamidsäurechloriden der allgemeinen Formel (VI) 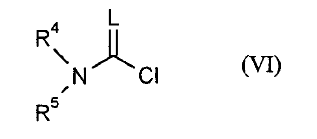 in welcher
         L, R⁴ und R⁵ die oben angegebene Bedeutung haben,

         gegebenenfalls in Gegenwart eines Verdünnungsmittels und gegebenenfalls in Gegenwart eines Säurebindemittels, umsetzt.
D) Ferner wurde gefunden, daß man Verbindungen der Formel (Id) 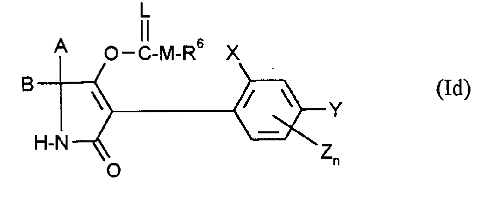 in welcher
   A, B, L, M, R⁶, X, Y, Z und n die oben angegebene Bedeutung haben,
      erhält, wenn man Verbindungen der Formel (II) 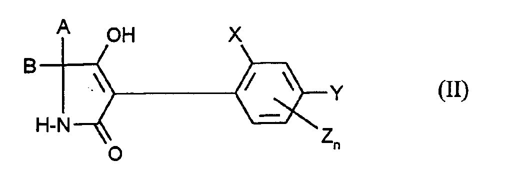 in welcher
   A, B, X, Y, Z und n die oben angegebene Bedeutung haben,
      a) mit Chlormonothioameisensäureestern, Chlorameisensäurethioestern oder Chlordithioameisensäureestern der allgemeinen Formel (VII) 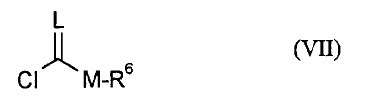 in welcher
         L, M, R⁶ die oben angegebene Bedeutung haben,

         gegebenenfalls in Gegenwart eines Verdünnungsmittels und gegebenenfalls in Gegenwart eines Säurebindemittels umsetzt,
         oder
      β) mit Schwefelkohlenstoff und anschließend mit Alkylhalogeniden der allgemeinen Formel (VIII) 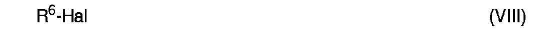 in welcher
         R⁶ die oben angegebene Bedeutung hat und
         Hal für Chlor, Brom oder lod steht,

         umsetzt.

Überraschenderweise wurde gefunden, daß die neuen substituierten 1-H-3-Aryl-pyrrolidin-2,4-dion-Derivate der Formel (I) sich durch hervorragende insektizide, akarizide und herbizide Wirkungen auszeichnen.

Bevorzugt sind substituierte 1-H-3-Aryl-pyrrolidin-2,4-dion-Derivate der Formel (I), in welcher
X für C₁-C₄-Alkyl, Halogen, C₁-C₄-Alkoxy steht,
Y für Wasserstoff, C₁-C₆-Alkyl, Halogen, C₁-C₄-Alkoxy, C₁-C₂-Halogenalkyl steht,
Z für C₁-C₄-Alkyl, Halogen, C₁-C₄-Alkoxy steht,
n für eine Zahl von 0 bis 3 steht,
A für Wasserstoff oder gegebenenfalls durch Halogen substituiertes geradkettiges oder verzweigtes C₁-C₁₀-Alkyl, C₃-C₆-Alkenyl, C₃-C₆-Alkinyl, C₁-C₈-Alkoxy-C₂-C₆-alkyl, C₁-C₆-Polyalkoxy-C₂-C₆-alkyl, C₁-C₈-Alkylthio-C₂-C₆- alkyl, Cycloalkyl mit 3 bis 7 Ringatomen, das durch 1 bis 2 Sauerstoff-und/oder Schwefelatome unterbrochen sein kann, steht,
B für Wasserstoff, geradkettiges oder verzweigtes C₁-C₁₀-Alkyl, C₁-C₆-Alkoxyalkyl steht,

oder worin
A und B gemeinsam mit dem Kohlenstoffatom, an das sie gebunden sind, einen 3-bis 7-gliedrigen gesättigten oder ungesättigten Ring bilden, der durch C₁-C₆-Alkyl, C₁-C₆-Alkoxy, C₁-C₃-Halogenalkyl, C₁-C₃-Halogenalk- oxy, Fluor, Chlor substituiert und der durch Sauerstoff und/oder Schwefel unterbrochen sein kann,
R für die Gruppen 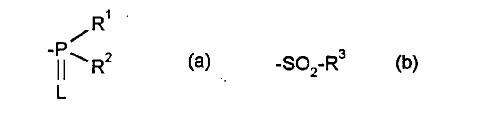 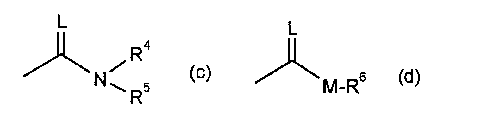 steht,
   in welchen
L und M jeweils für Sauerstoff oder Schwefel stehen und worin L und M nicht gleichzeitig für Sauerstoff stehen,
R¹, R² und R³ unabhängig voneinander für gegebenenfalls durch Halogen substituiertes C₁-C₆-Alkyl, C₁-C₆-Alkoxy, C₁-C₆-Alkylamino, Di-(C₁-C₆)-alkylamino, C₁-C₆-Alkylthio, C₃-C₄-Alkenylthio, C₂-C₄-Alkinylthio, C₃-C₆-Cycloalkylthio, für gegebenenfalls durch Fluor, Chlor, Brom, Nitro, Cyano, C₁-C₃-Alkoxy, Cᵢ-C₃-Halogenalkoxy, Ci-C3-Alkylthio, C₁-C₃-Halogenalkylthio, C₁-C₃-Alkyl, C₁-C₃-Halogenalkyl substituiertes Phenyl, Benzyl, Phenoxy oder Phenylthio stehen,
R⁴ und R⁵ unabhängig voneinander für gegebenenfalls durch Halogen substituiertes C₁-C₂₀-Alkyl, C₁-C₂ₒ-Alkoxy, C₂-C₈-Alkenyl, C₁-C₂₀-Alkoxy-C₁-C₂₀-alkyl, für gegebenenfalls durch Halogen, C₁-C₅-Halogenalkyl, C₁-C₅-Alkyl oder C₁-C₅-Alkoxy substituiertes Phenyl, für gegebenenfalls durch Halogen, C₁-C₅-Alkyl, C₁-C₅-Halogenalkyl oder C₁-C₅-Alkoxy substituiertes Benzyl stehen,
R⁶ für gegebenenfalls durch Halogen substituiertes C₁-C₂ₒ-Alkyl, das durch Sauerstoff unterbrochen sein kann, für gegebenenfalls durch Halogen, C₁-C₅-Halogenalkyl, C₁-C₅-Alkoxy substituiertes Phenyl, für gegebenenfalls durch Halogen, C₁-C₅-Halogenalkyl oder C₁-C₅-Alkoxy substituiertes Benzyl steht,
   sowie die enantiomerenreinen Formen von Verbindungen der Formel (I).

Besonders bevorzugt sind Verbindungen der Formel (I), in welchen
X für Methyl, Ethyl, Propyl, i-Propyl, Fluor, Chlor, Brom, Methoxy und Ethoxy steht,
Y für Wasserstoff, Methyl, Ethyl, Propyl, i-Propyl, Butyl, i-Butyl, tert.-Butyl, Fluor, Chlor, Brom, Methoxy, Ethoxy und Trifluormethyl steht,
Z für Methyl, Ethyl, i-Propyl, Butyl, i-Butyl, tert.-Butyl, Fluor, Chlor, Brom, Methoxy und Ethoxy steht,
n für eine Zahl von 0 bis 3 steht,
A für Wasserstoff, gegebenenfalls durch Halogen substituiertes geradkettiges oder verzweigtes C₁-C₈-Alkyl, C₃-C₄-Alkenyl, C₃-C₄-Albnyl, C₁-C₆-Alkoxy-C₂-C₄-alkyl, Cᵢ-C₄-Polyalkoxy-C₂-C₄-alkyl, C₁-C₆-Alkylthio-C₂-C₄-alkyl, Cycloalkyl mit 3 bis 6 Ringatomen, das durch 1 bis 2 Sauerstoff- und/oder Schwefelatomen unterbrochen sein kann, steht,
B für Wasserstoff, geradkettiges oder verzweigtes C₁-C₈-Alkyl, C₁-C₄-Alkoxyalkyl steht,

oder worin
A und B gemeinsam mit dem Kohlenstoffatom, an das sie gebunden sind, einen 3-bis 6-gliedrigen gesättigten oder ungesättigten Ring bilden, der durch C₁-C₄-Alkyl, C₁-C₄-Alkoxy, Trifluormethyl, Trifluormethoxy, Fluor, Chlor substituiert und der durch Sauerstoff und/oder Schwefel unterbrochen sein kann,
R für die Gruppen 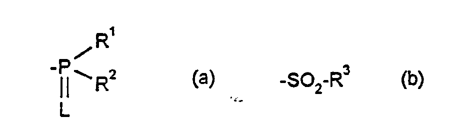 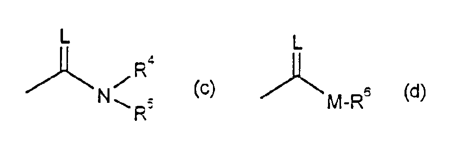 steht,
   in welchen
L und M jeweils für Sauerstoff oder Schwefel stehen und worin L und M nicht gleichzeitig für Sauerstoff stehen,
R¹, R² und R³ unabhängig voneinander für gegebenenfalls durch Fluor oder Chlor substituiertes C₁-C₄-Alkyl, C₁-C₄-Alkoxy, C₁-C₄-Alkylamino, Di-(C₁-C₄-alkyl)amino, C₁-C₄-Alkylthio, für gegebenenfalls durch Fluor, Chlor, Brom, Nitro, Cyano, C₁-C₂-Alkoxy, C₁-C₄-Fluoralkoxy, C₁-C₂-Chloralkoxy, C₁-C₂-Alkylthio, C₁-C₂-Fluoralkylthio, C₁-C₂-Chloralkylthio, C₁-C₃-Alkyl substituiertes Phenyl, Benzyl, Phenoxy oder Phenylthio stehen,
R⁴ und R⁵ unabhängig voneinander für gegebenenfalls durch Fluor, Chlor, Brom substituiertes C₁-C_{1 10}-Alkyl, C₁-C₁₀-Alkoxy, C₁-C₁₀-Alkoxy-(C₁-C₁₀)-alkyl, für gegebenenfalls durch Fluor, Chlor, Brom, C₁-C₂₀-Halogenalkyl, C₁-C₂₀-Alkyl oder C₁-C₄-Alkoxy substituiertes Phenyl, für gegebenenfalls durch Fluor, Chlor, Brom, C₁-C₄-Alkyl, C₁-C₄-Halogenalkyl oder C₁-C₄-Alkoxy substituiertes Benzyl stehen,
R⁶ für gegebenenfalls durch Fluor, Chlor oder Brom substituiertes C₁-C₁₀-Alkyl, das durch Sauerstoff unterbrochen sein kann, für gegebenenfalls durch Fluor, Chlor, Brom, C₁-C₄-Halogenalkyl, C₁-C₄-Alkoxy substituiertes Phenyl, für gegebenenfalls durch Fluor, Chlor, Brom, Ci-C4-Halogenalkyl oder C₁-C₄-Alkoxy substituiertes Benzyl steht,
   sowie die enantiomerenreinen Formen von Verbindungen der Formel (I).

Im einzelnen seien außer den bei den Herstellungsbeispielen genannten Verbindungen die folgenden substituierten 1-H-3-Aryl-pyrrolidin-2,4-dion-Derivate der allgemeinen Formel (I) genannt:

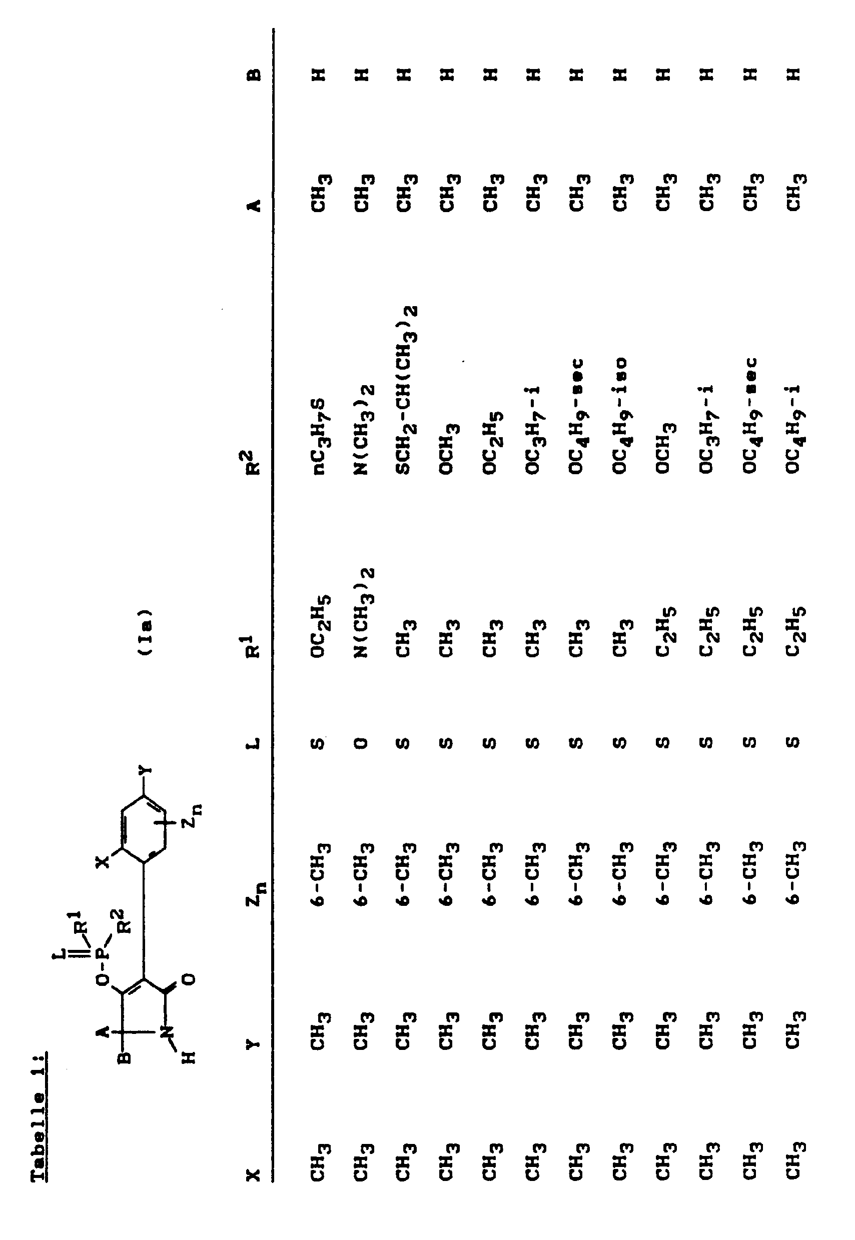

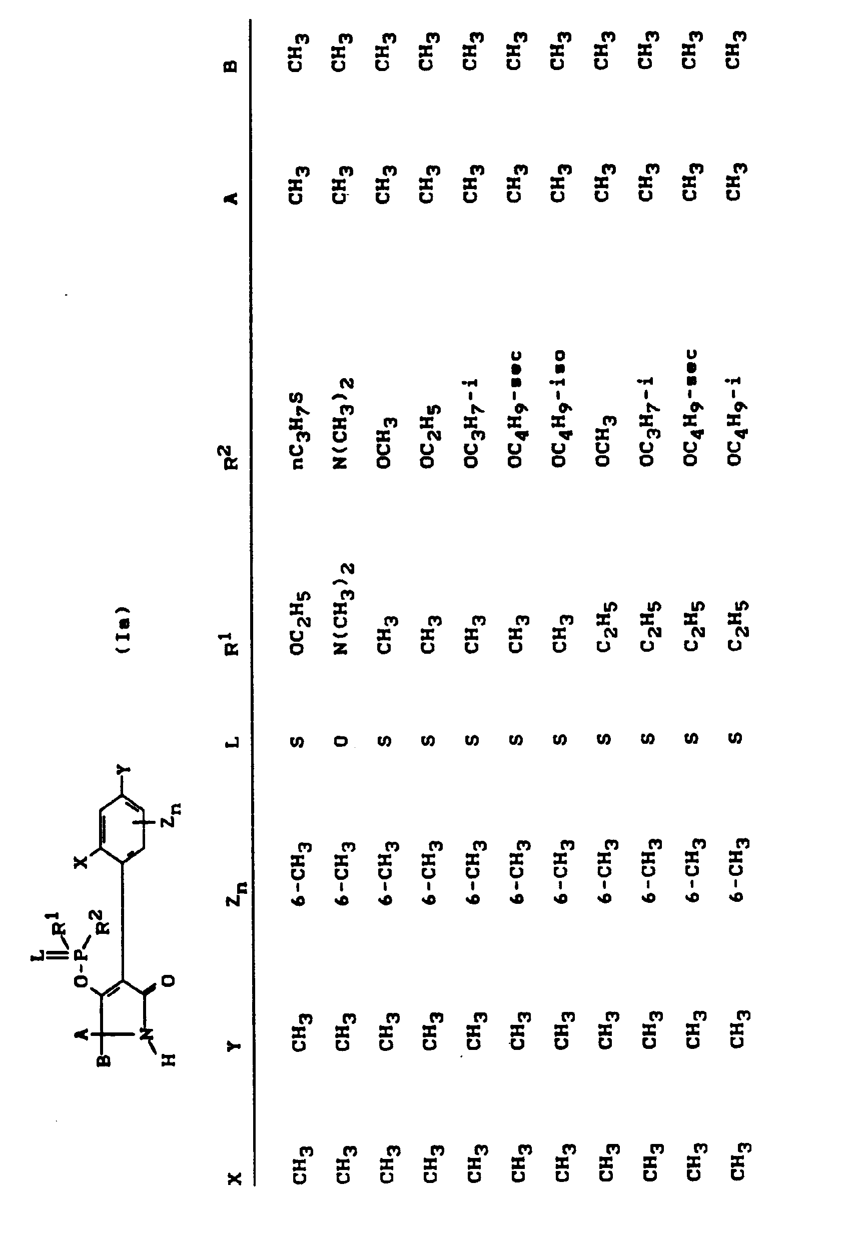

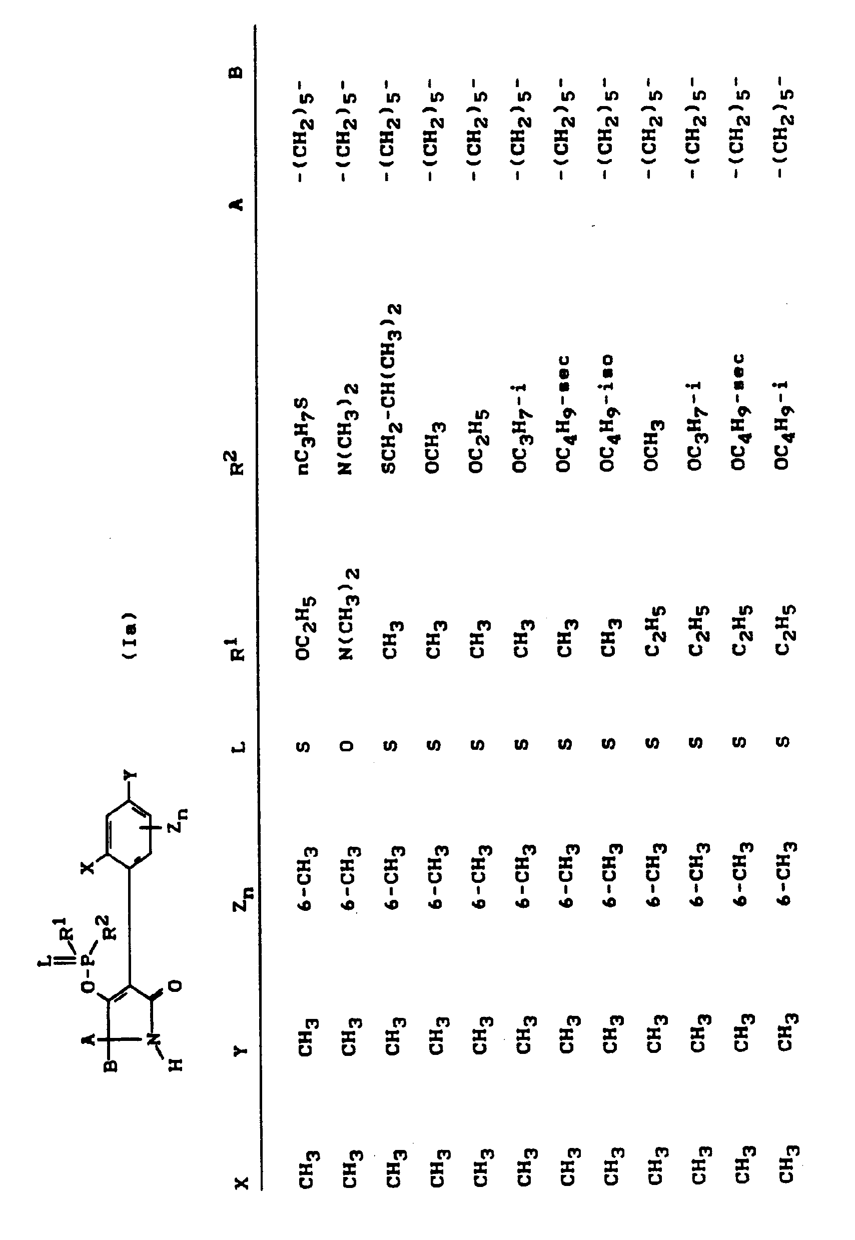

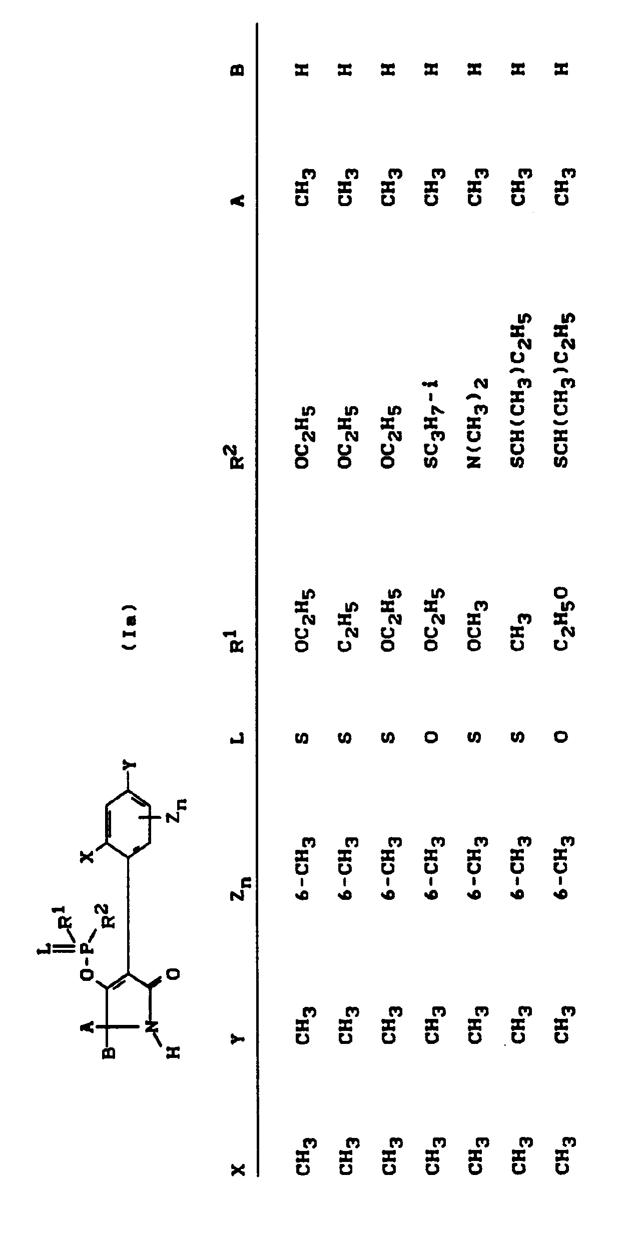

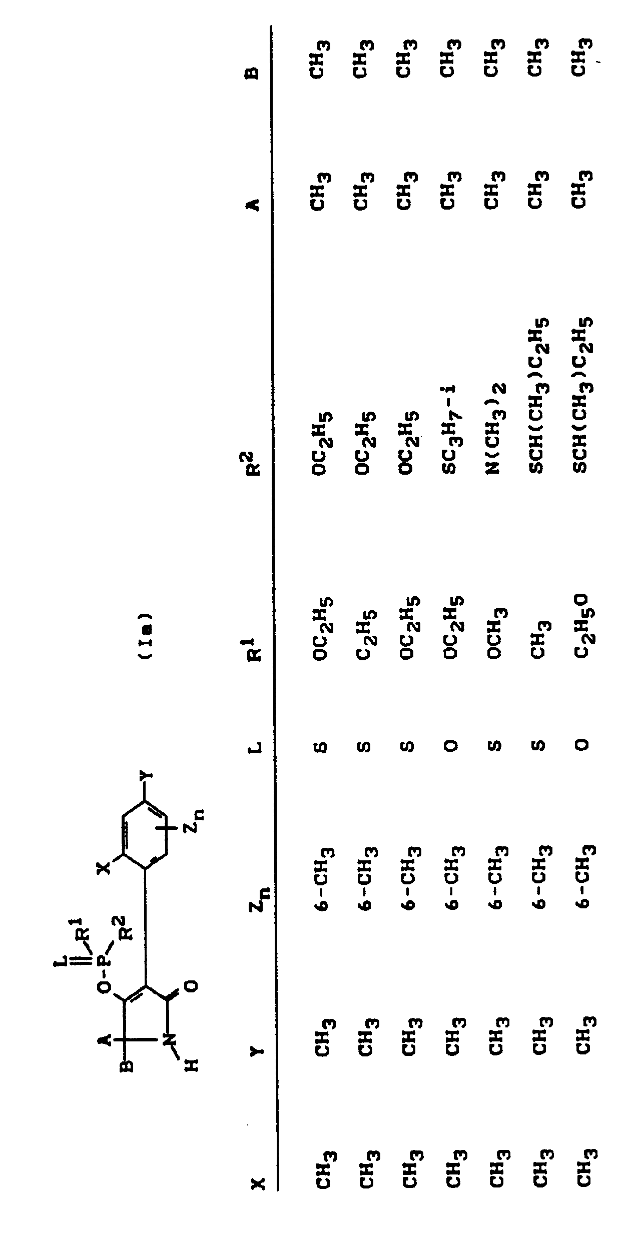

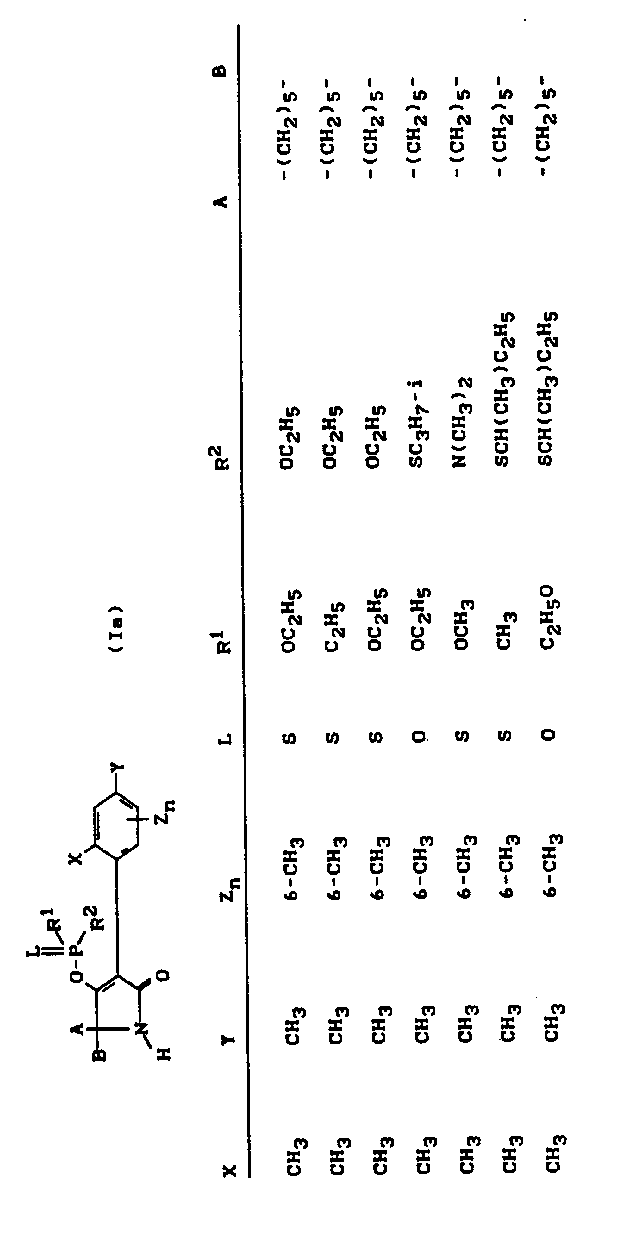

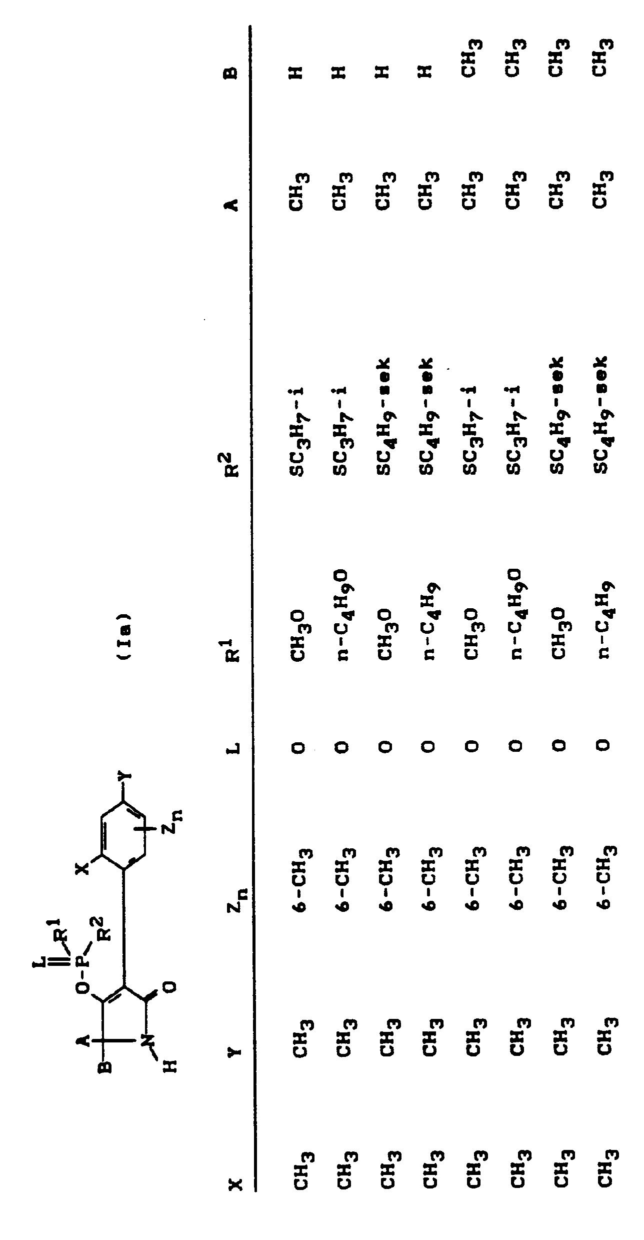

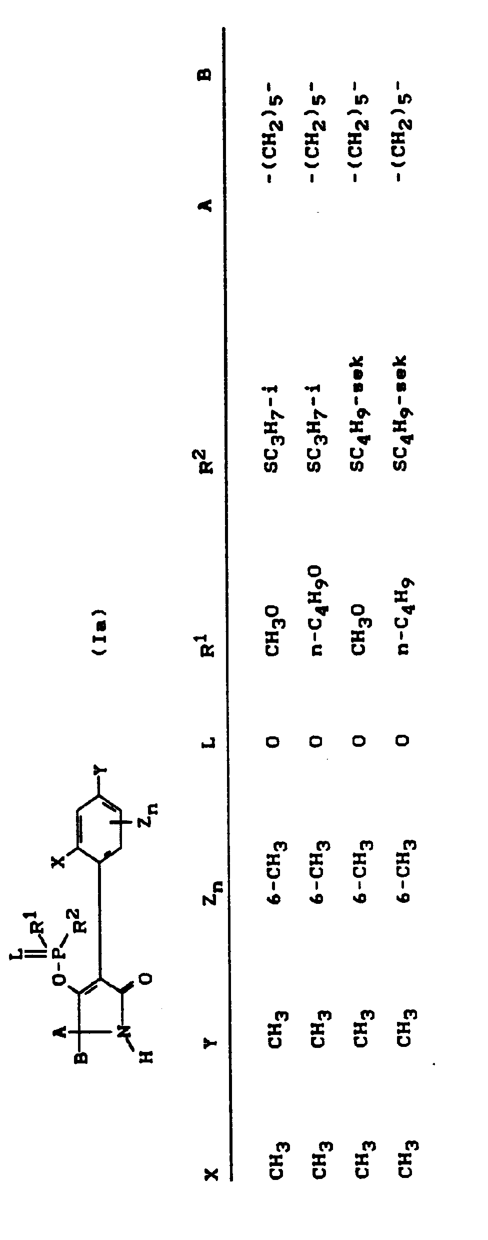

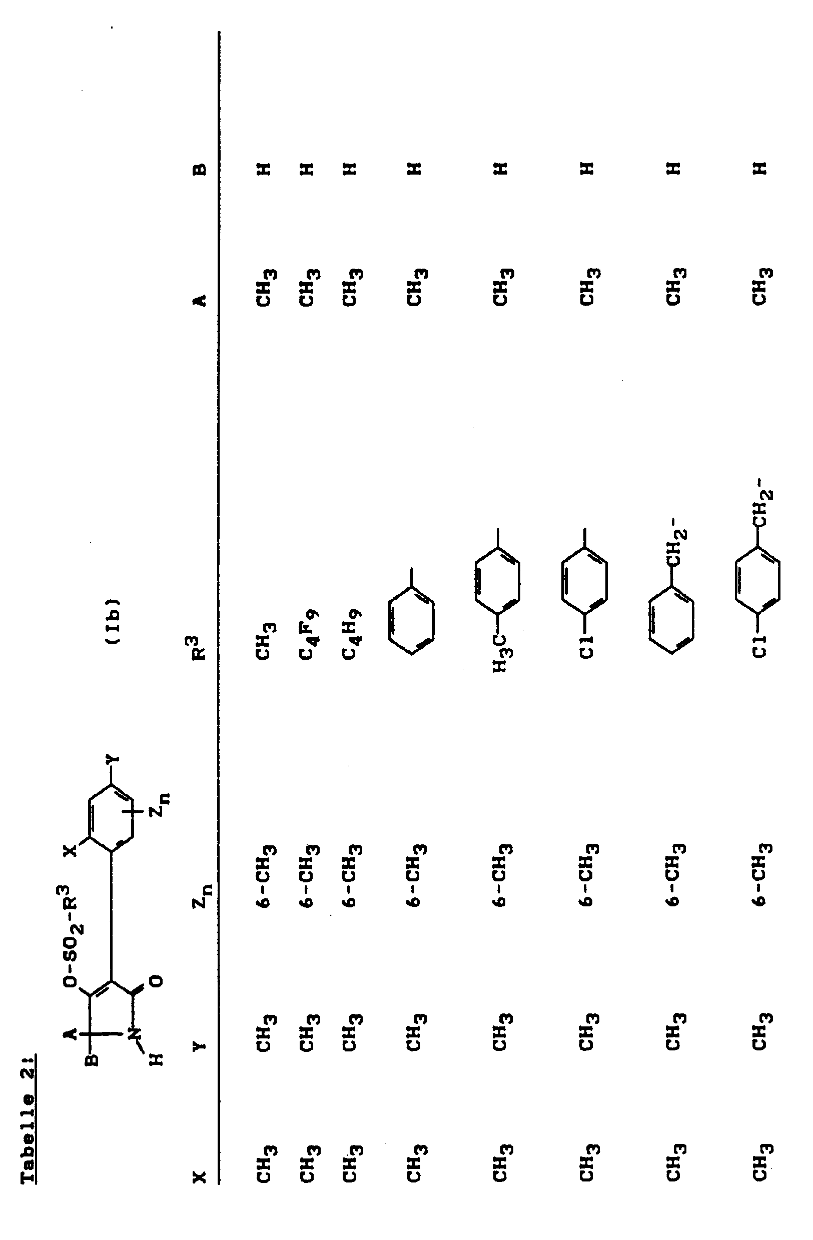

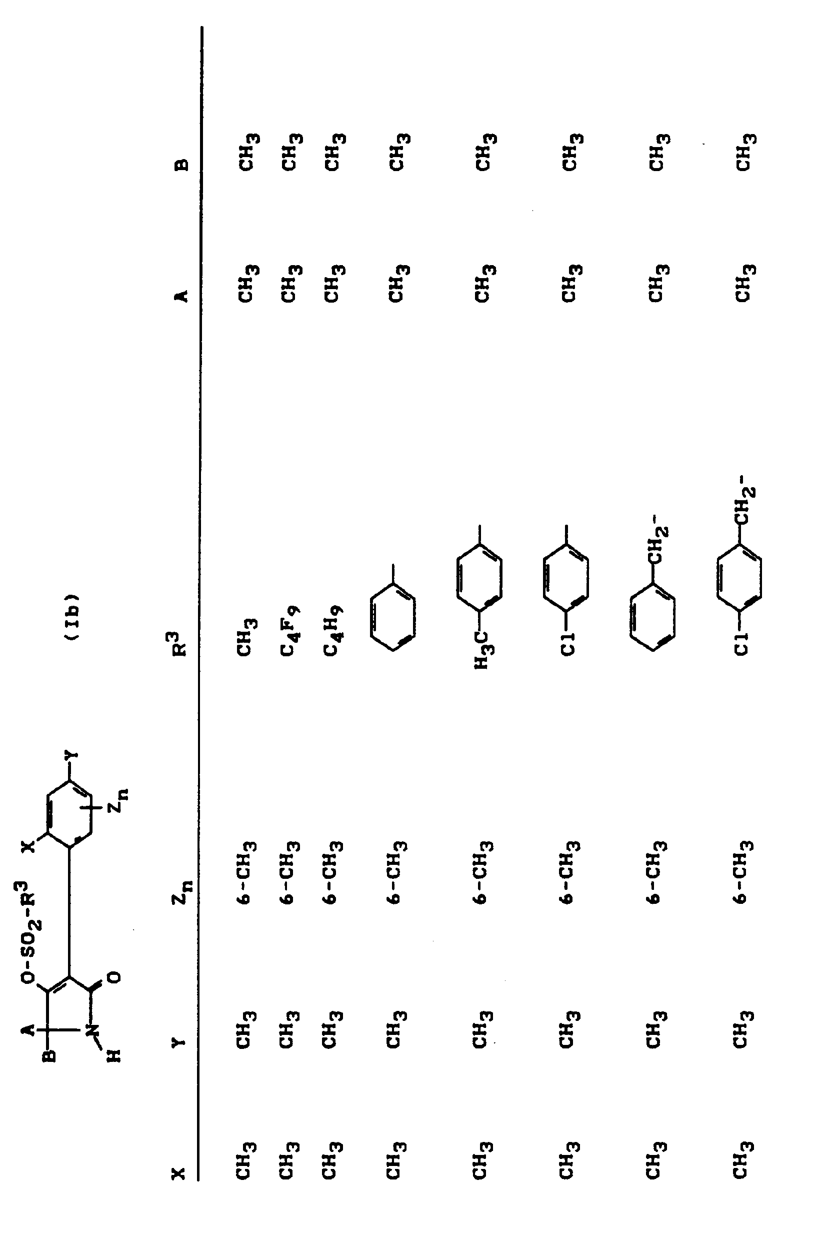

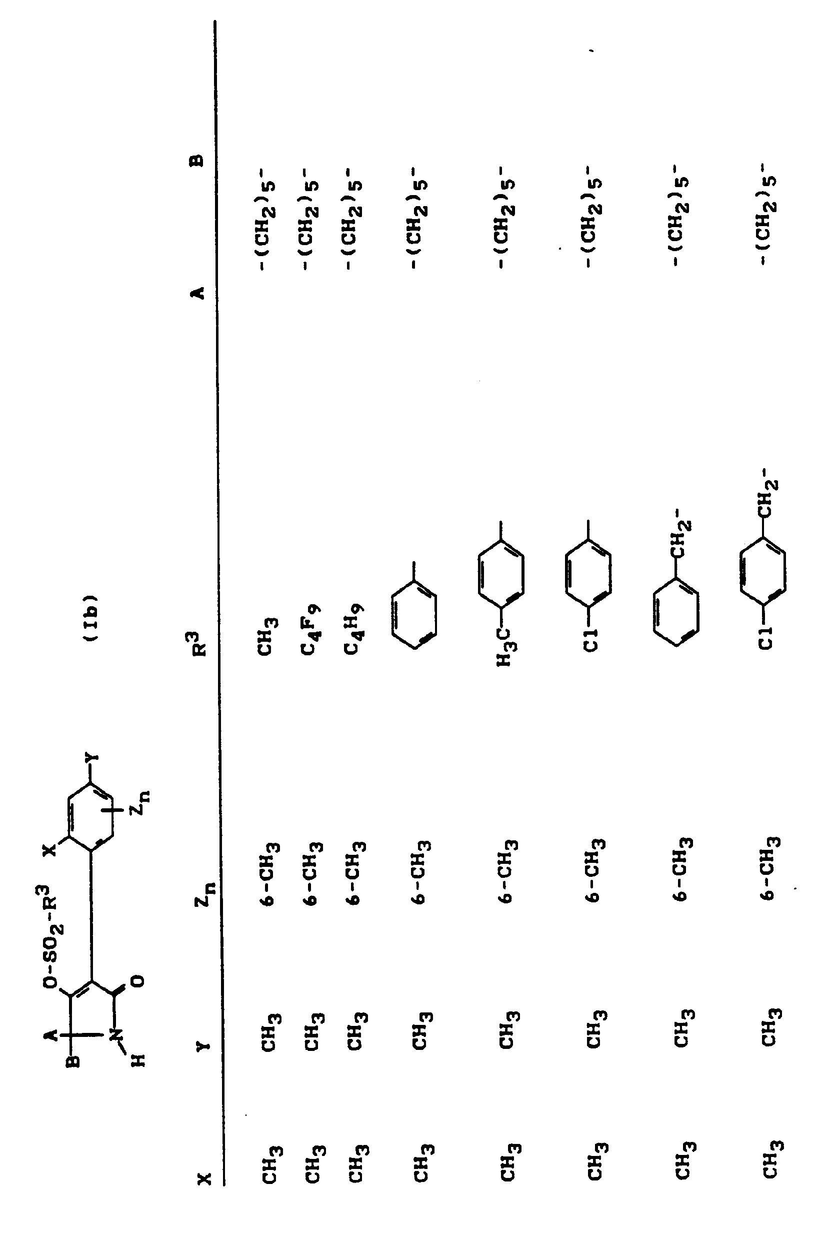

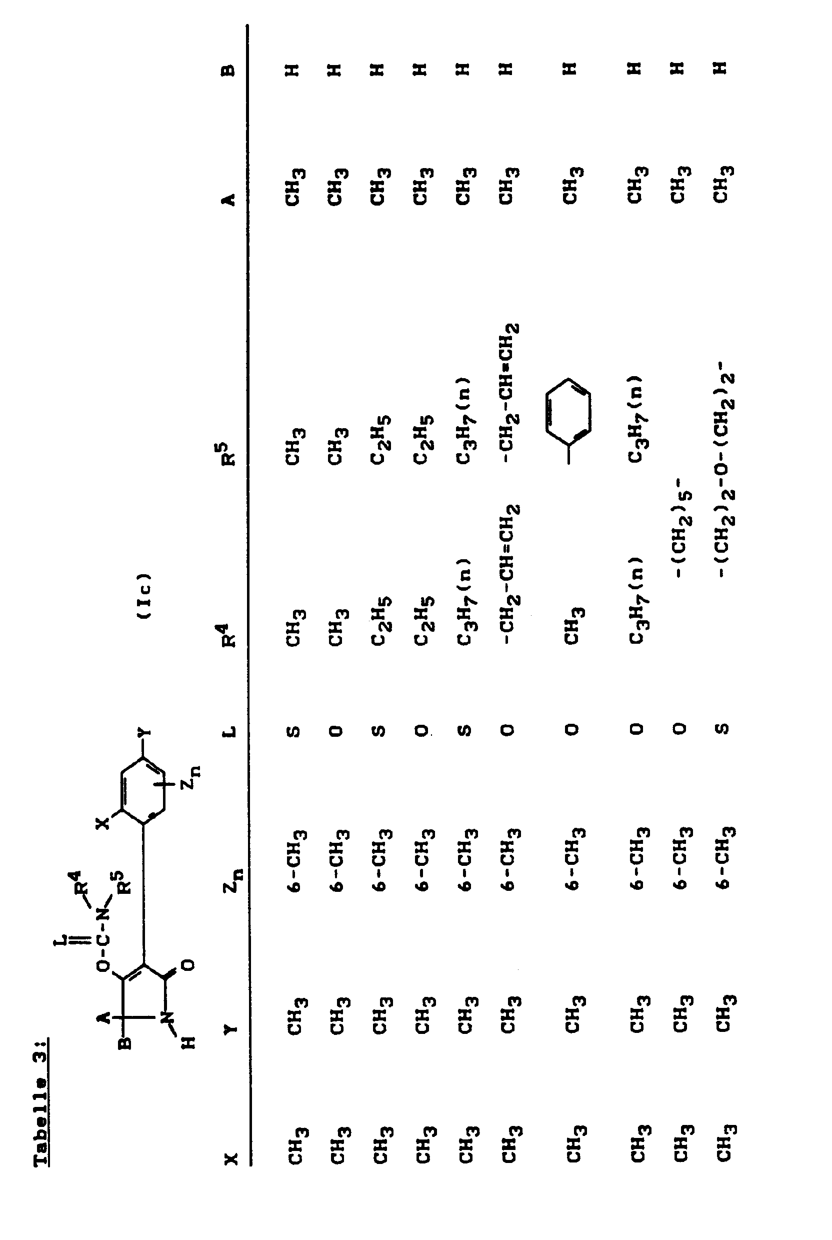

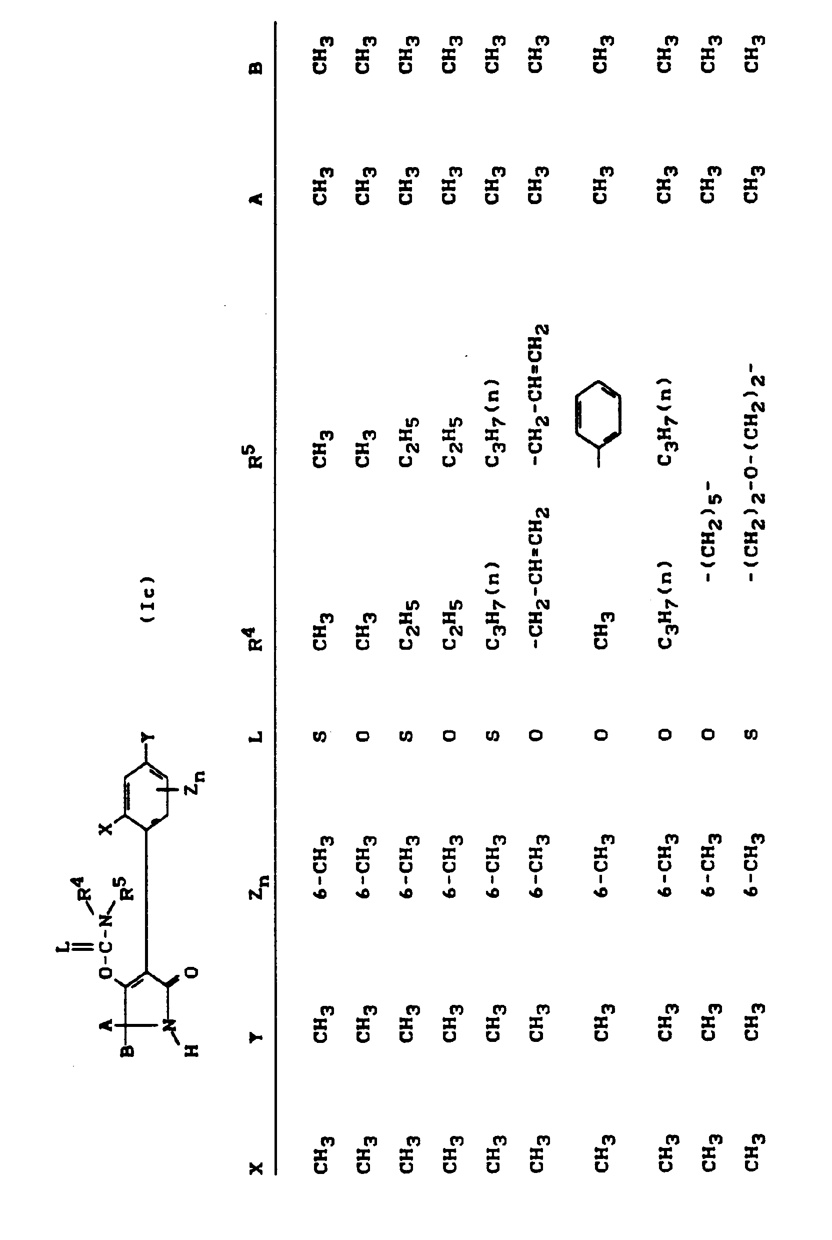

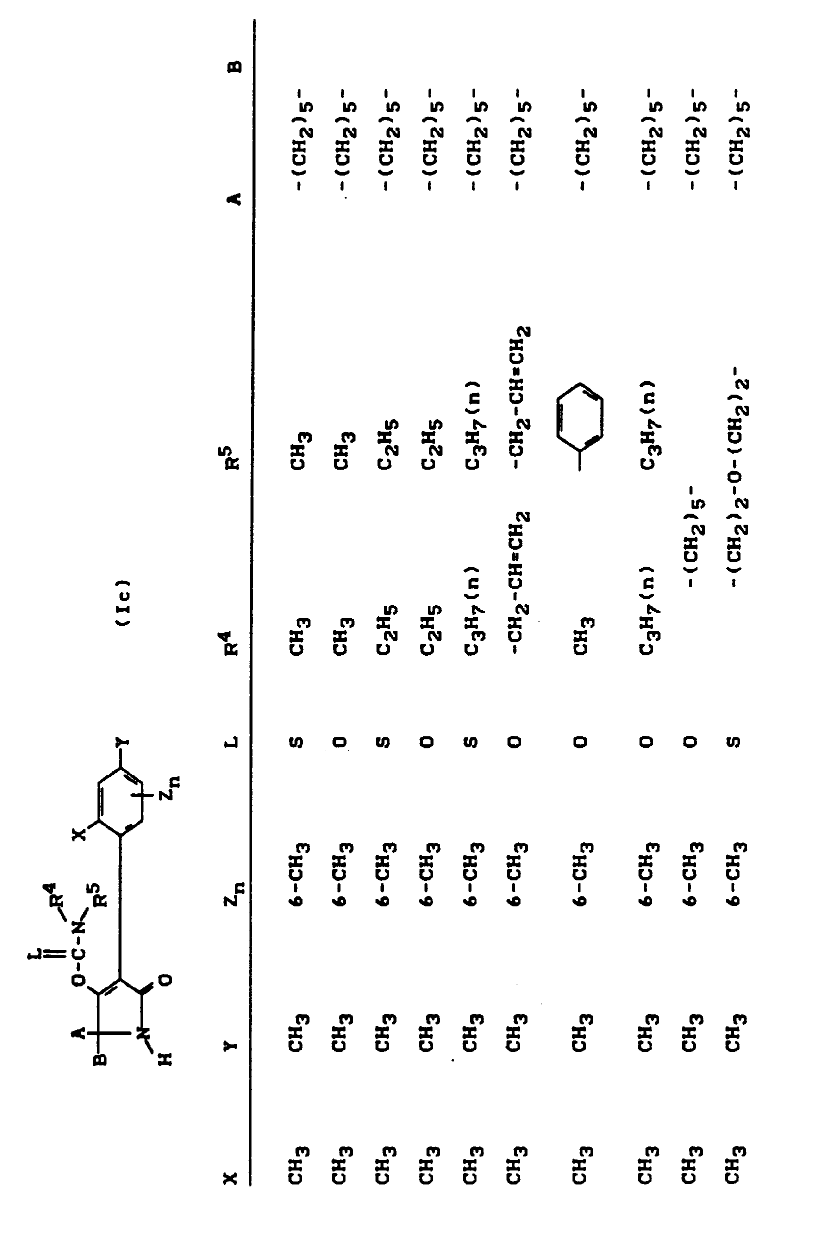

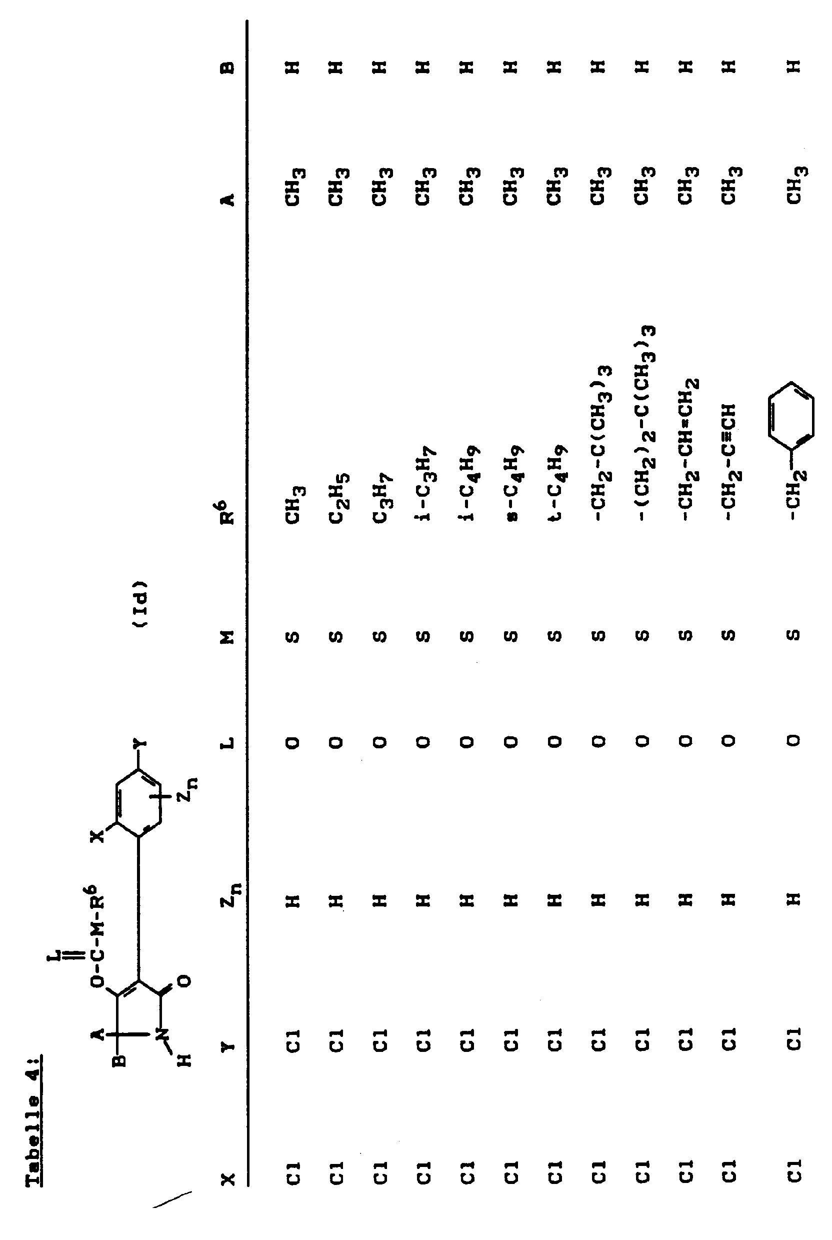

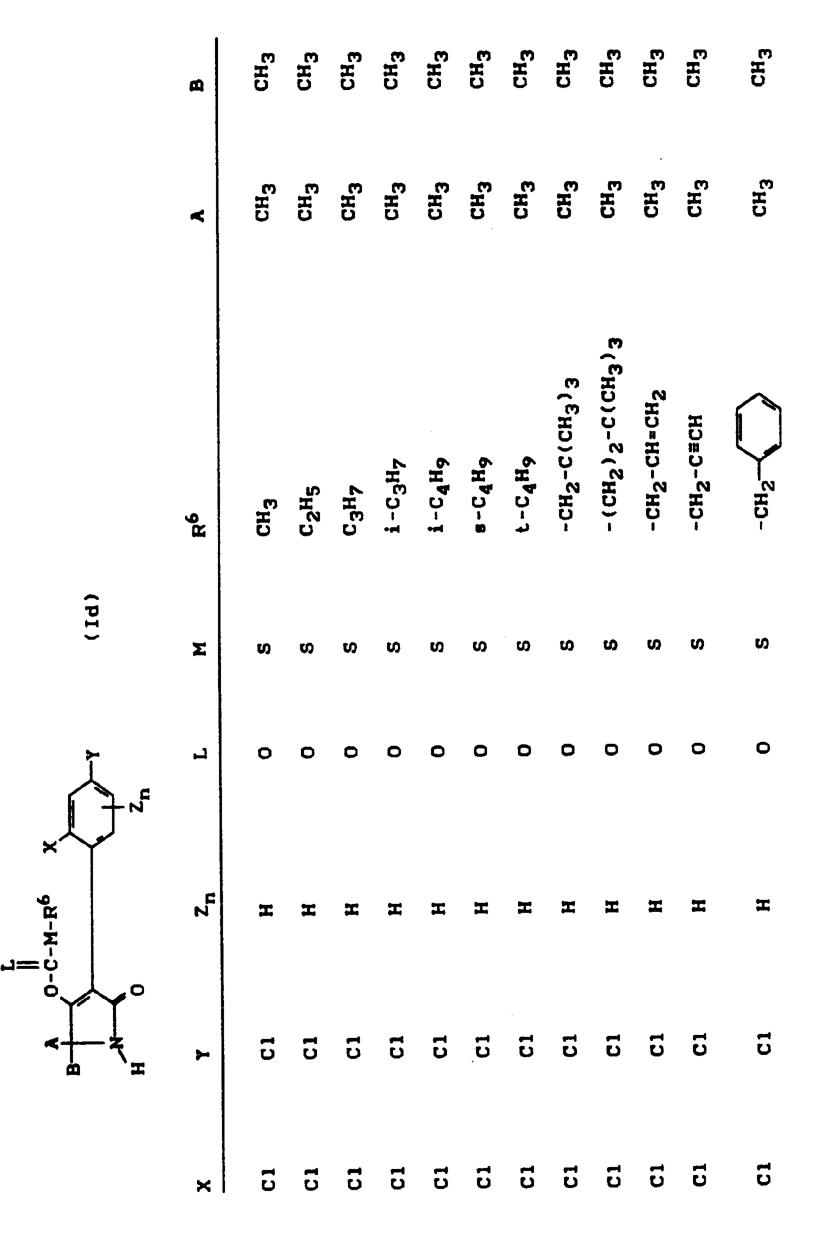

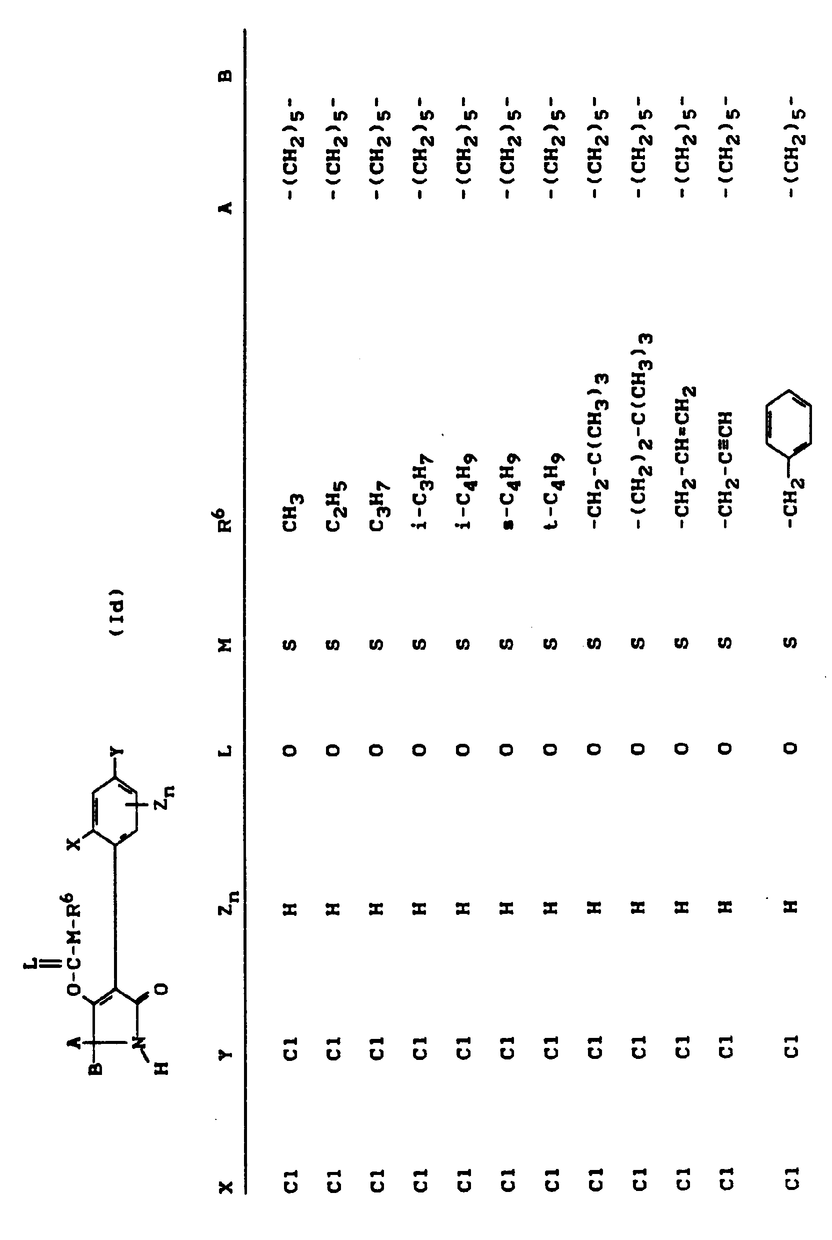

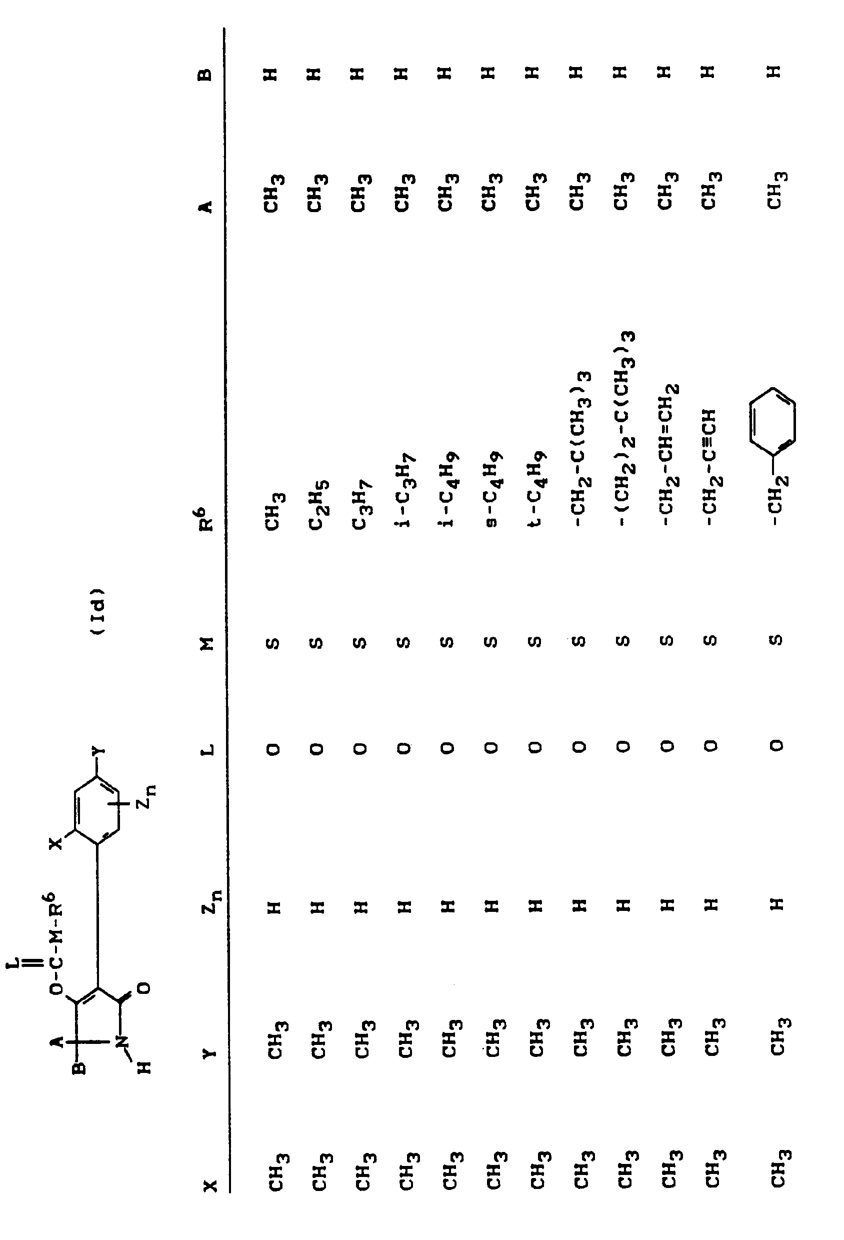

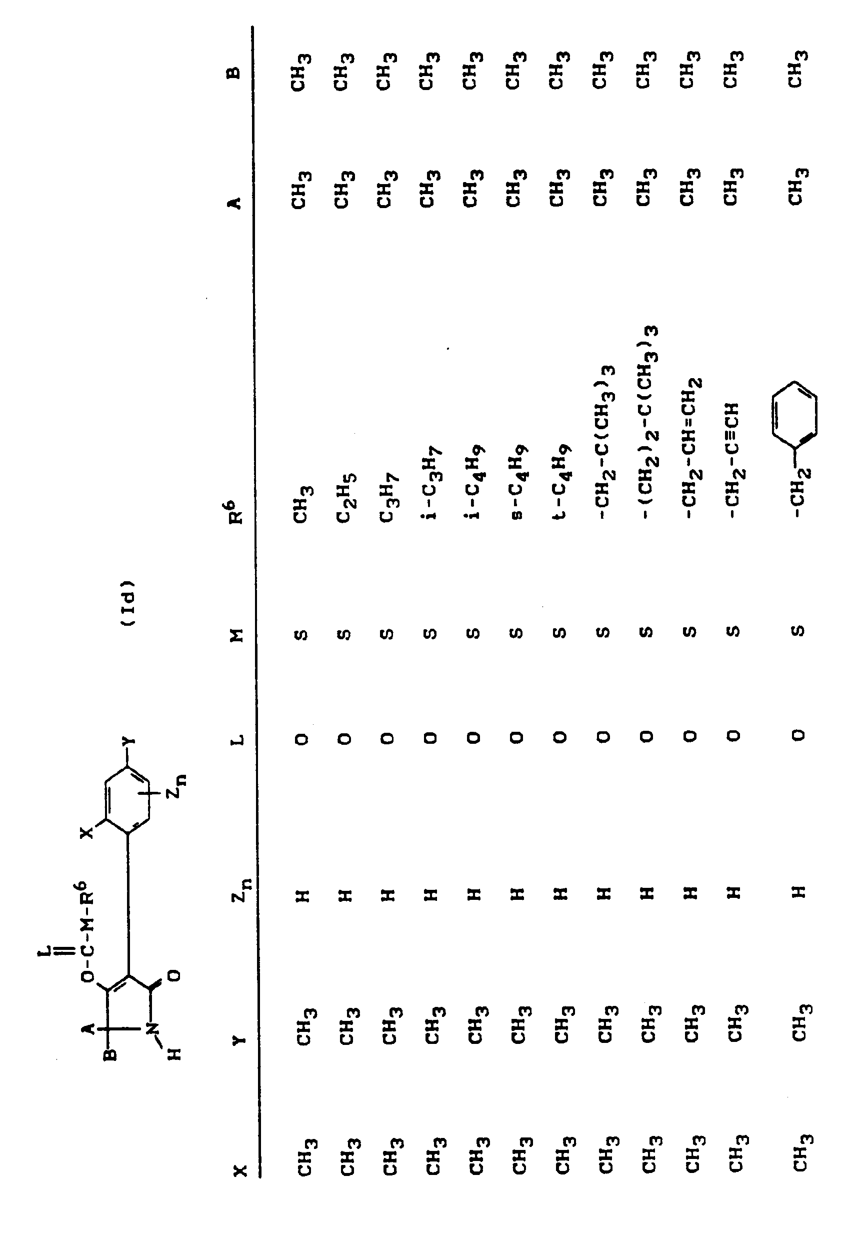

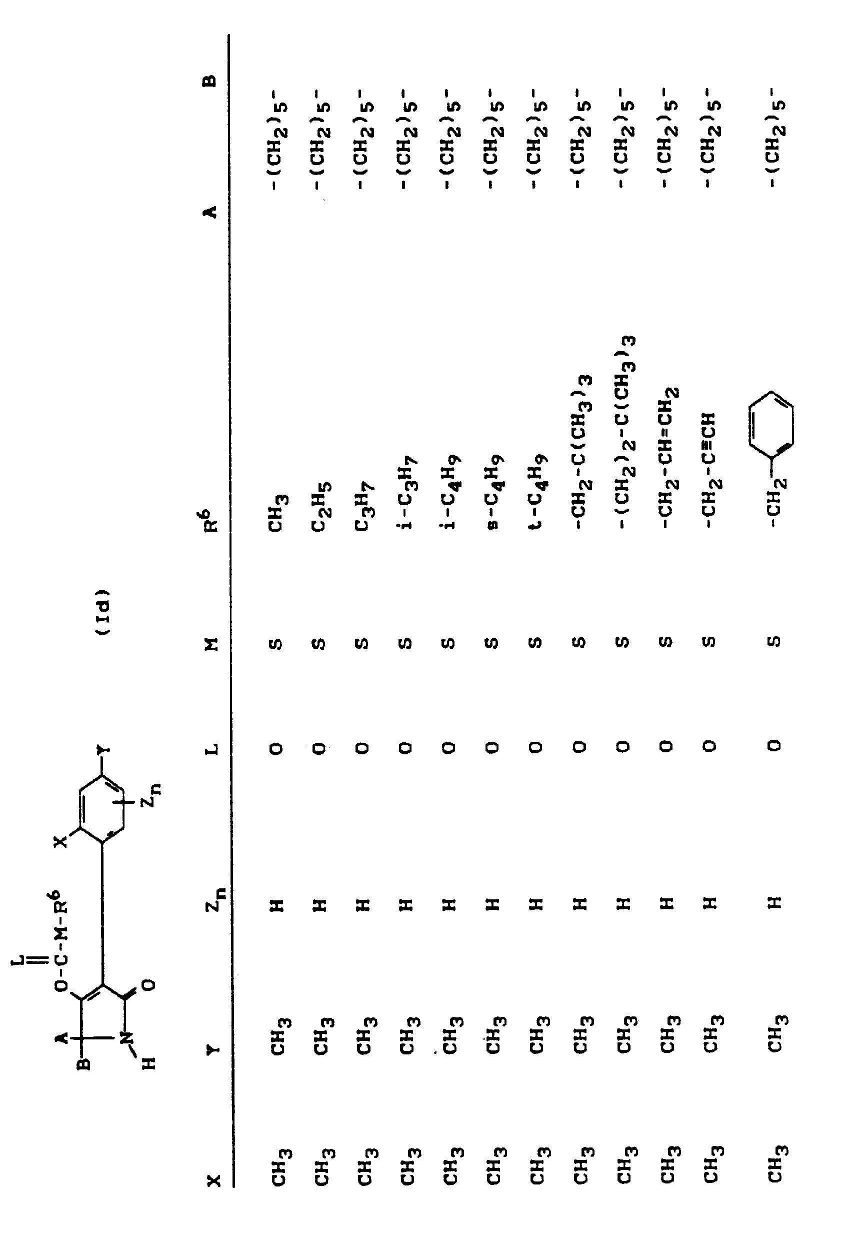

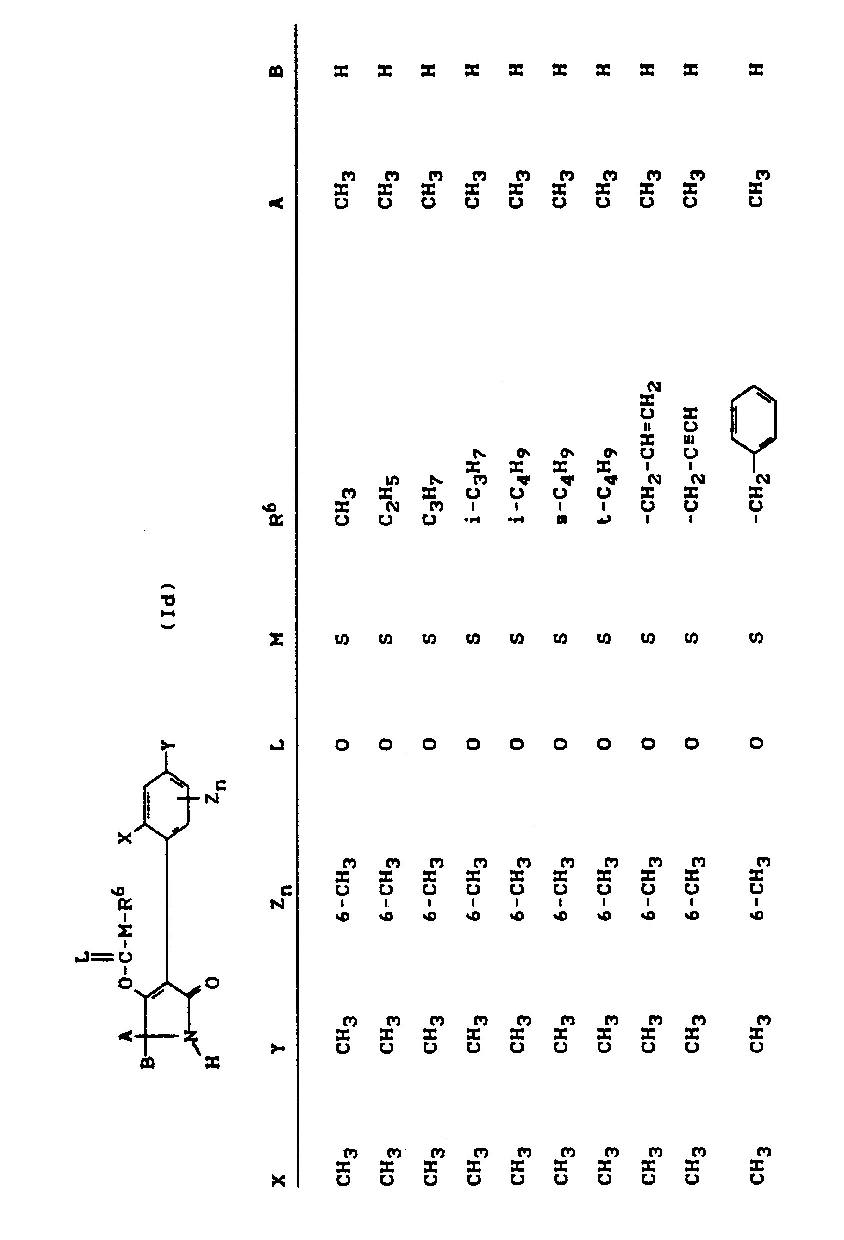

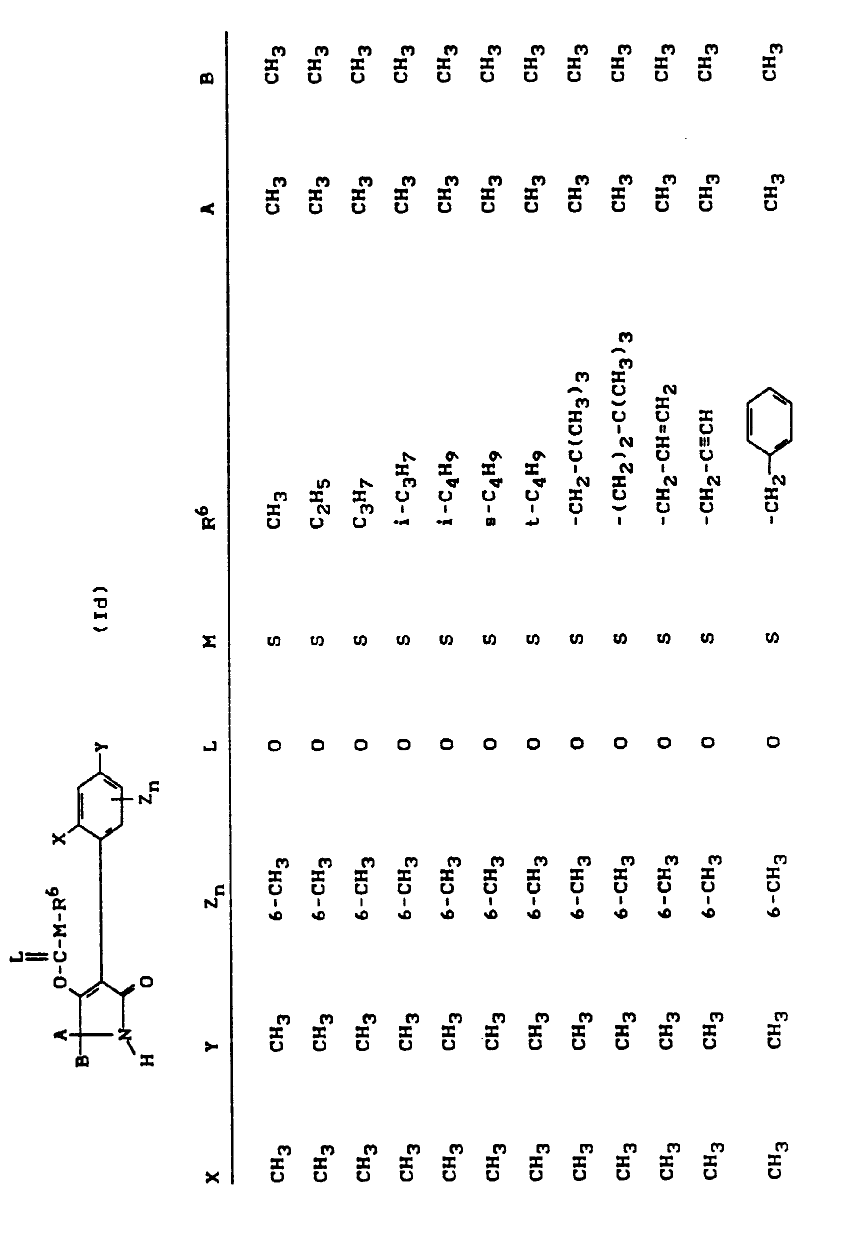

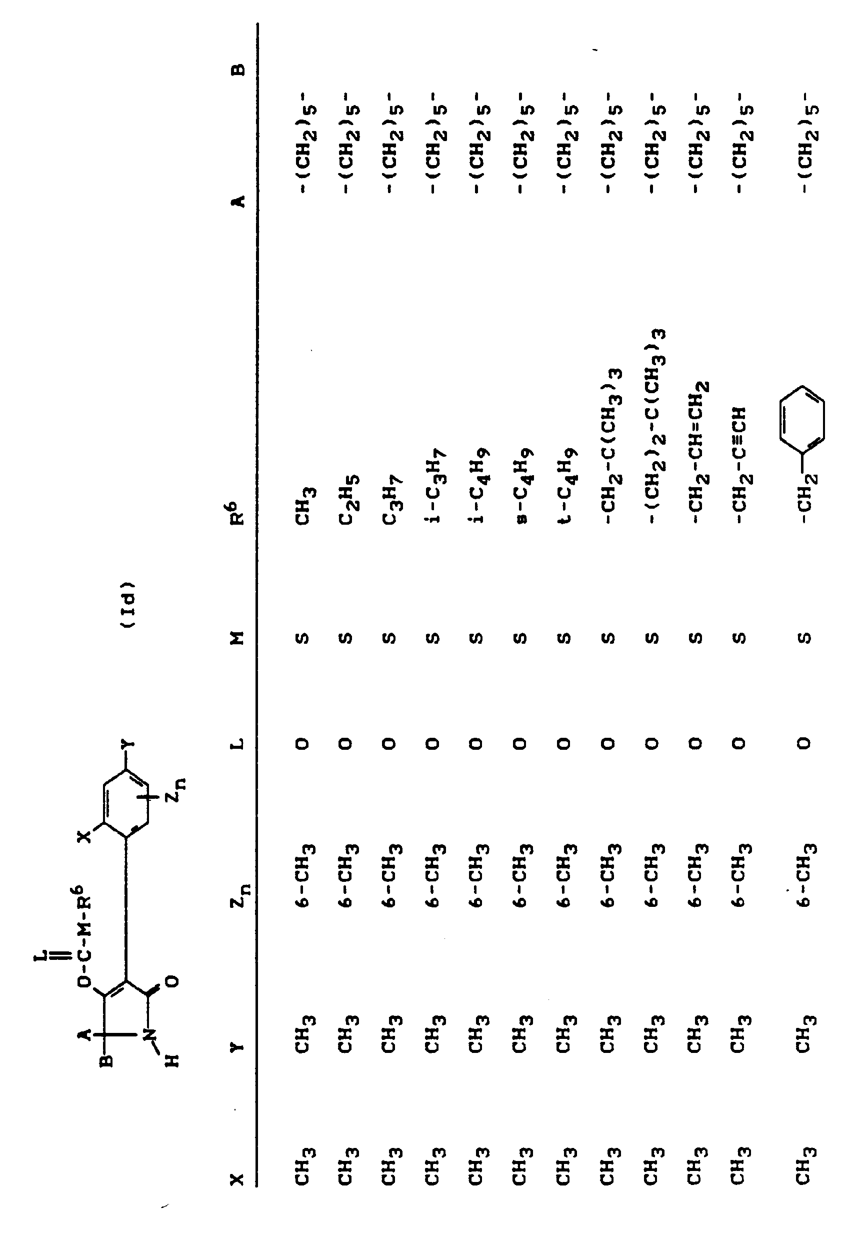

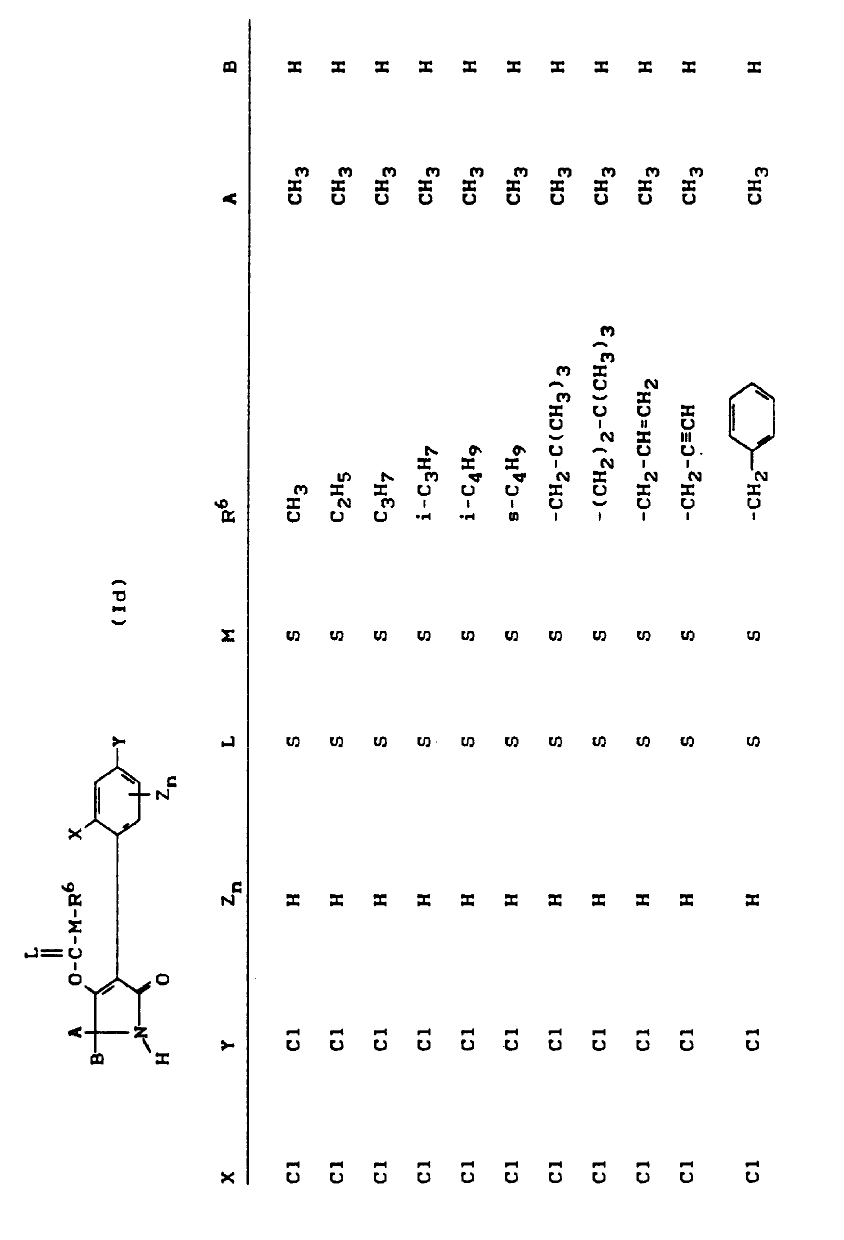

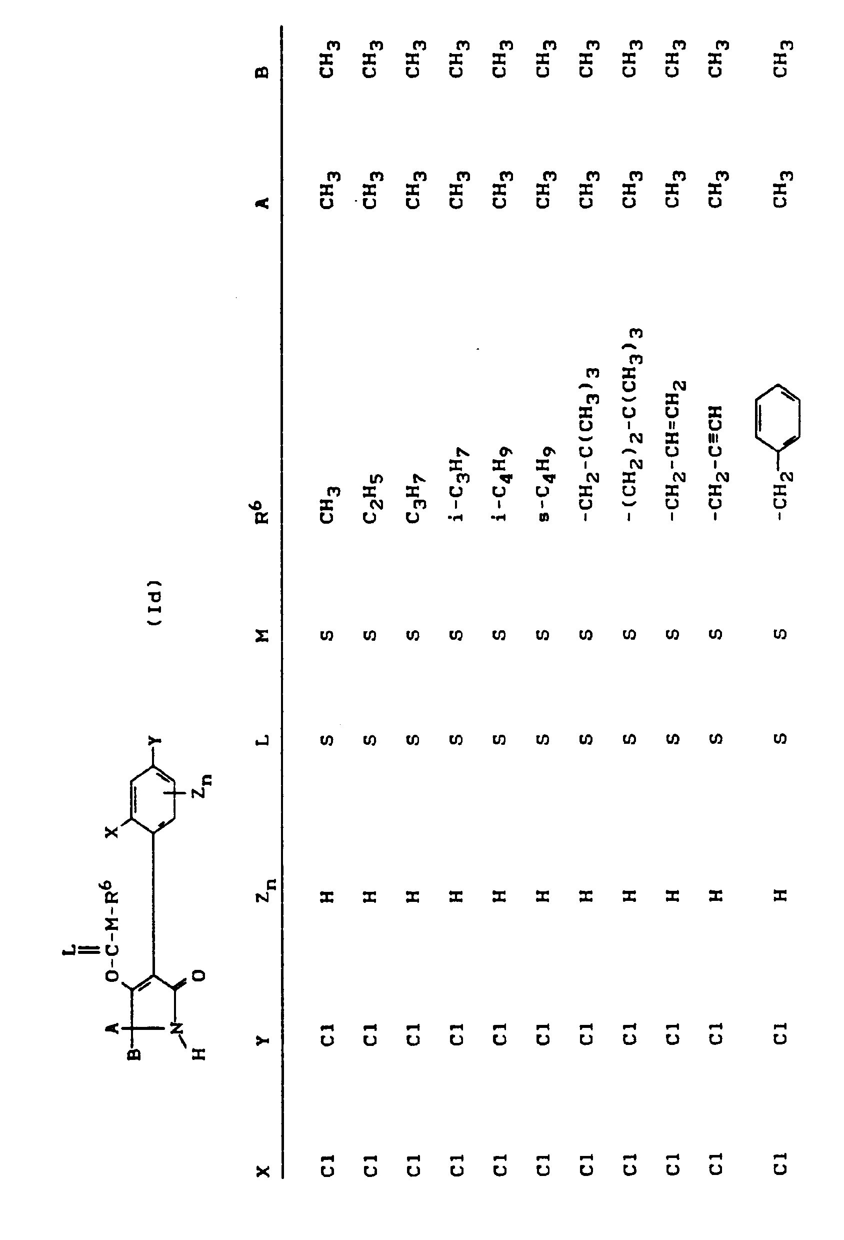

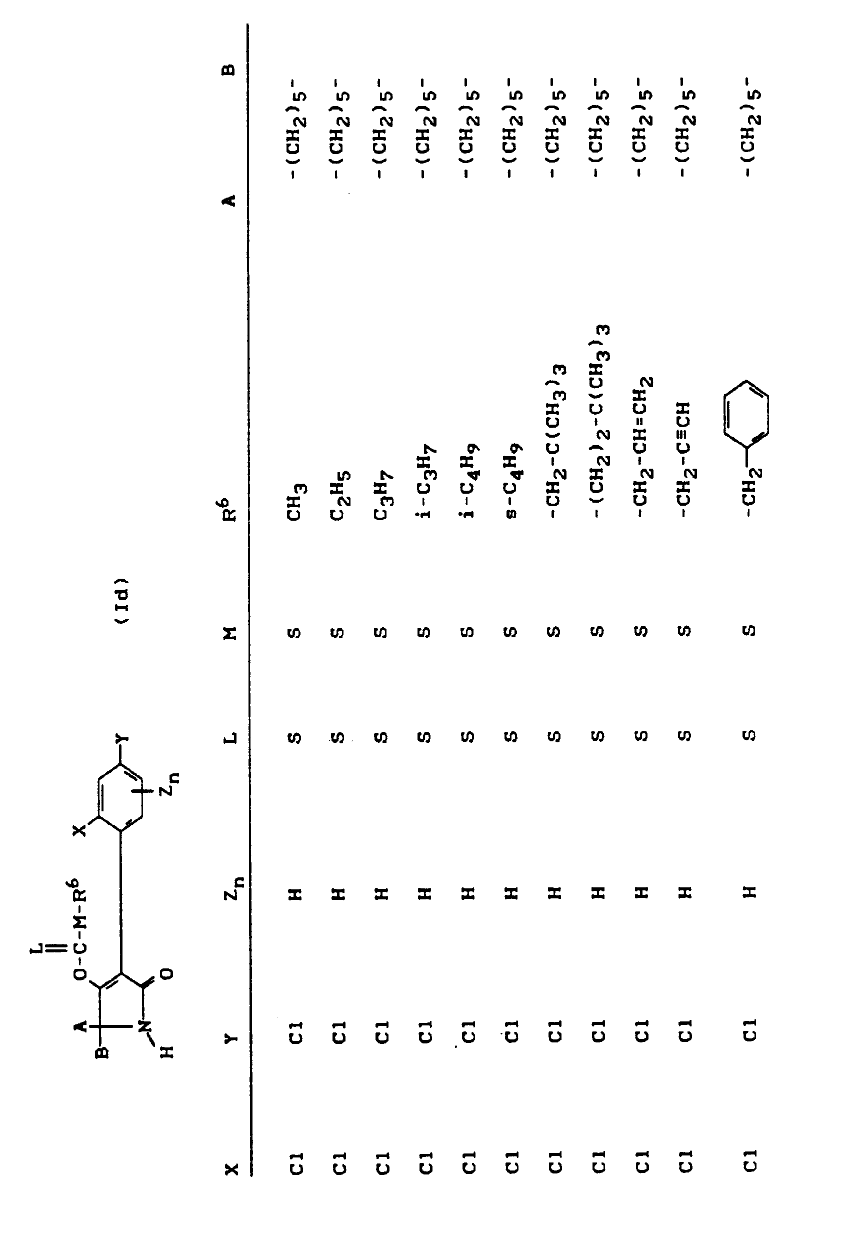

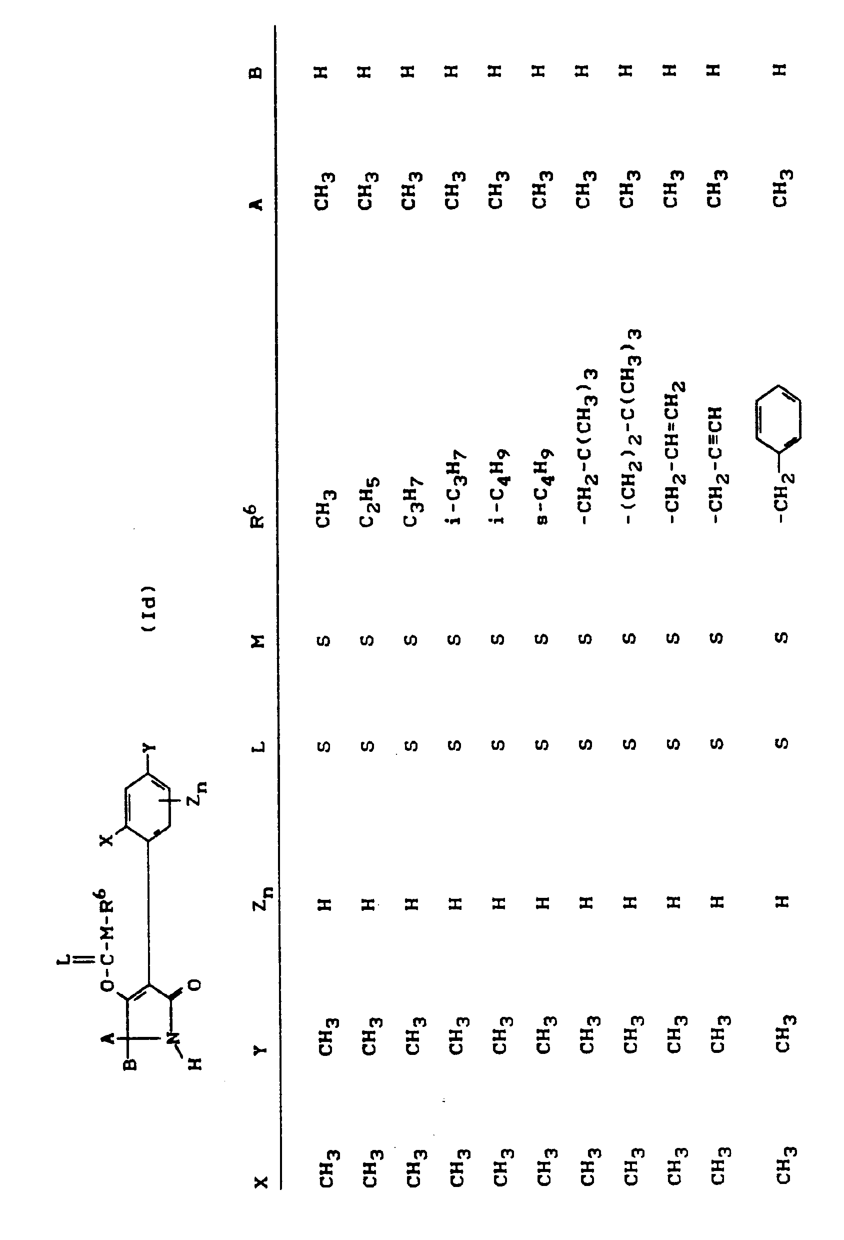

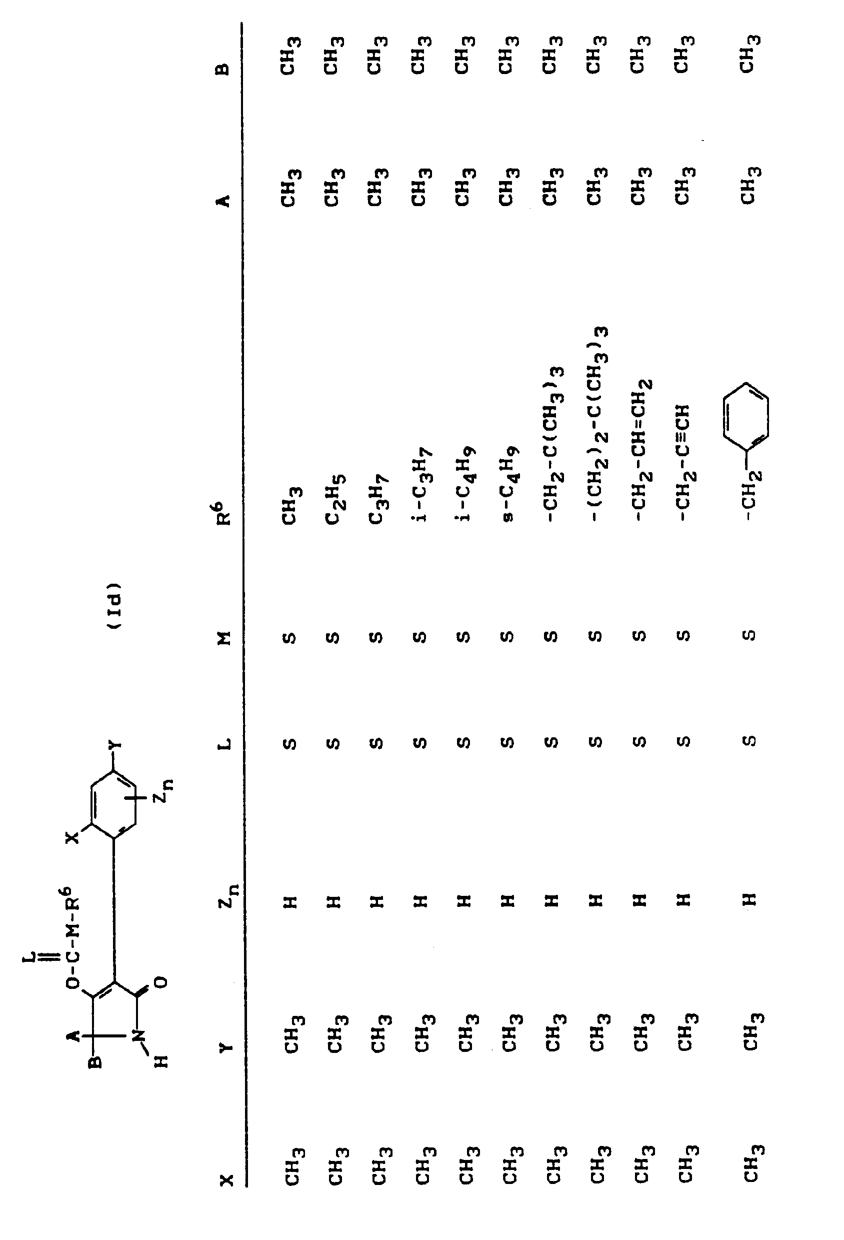

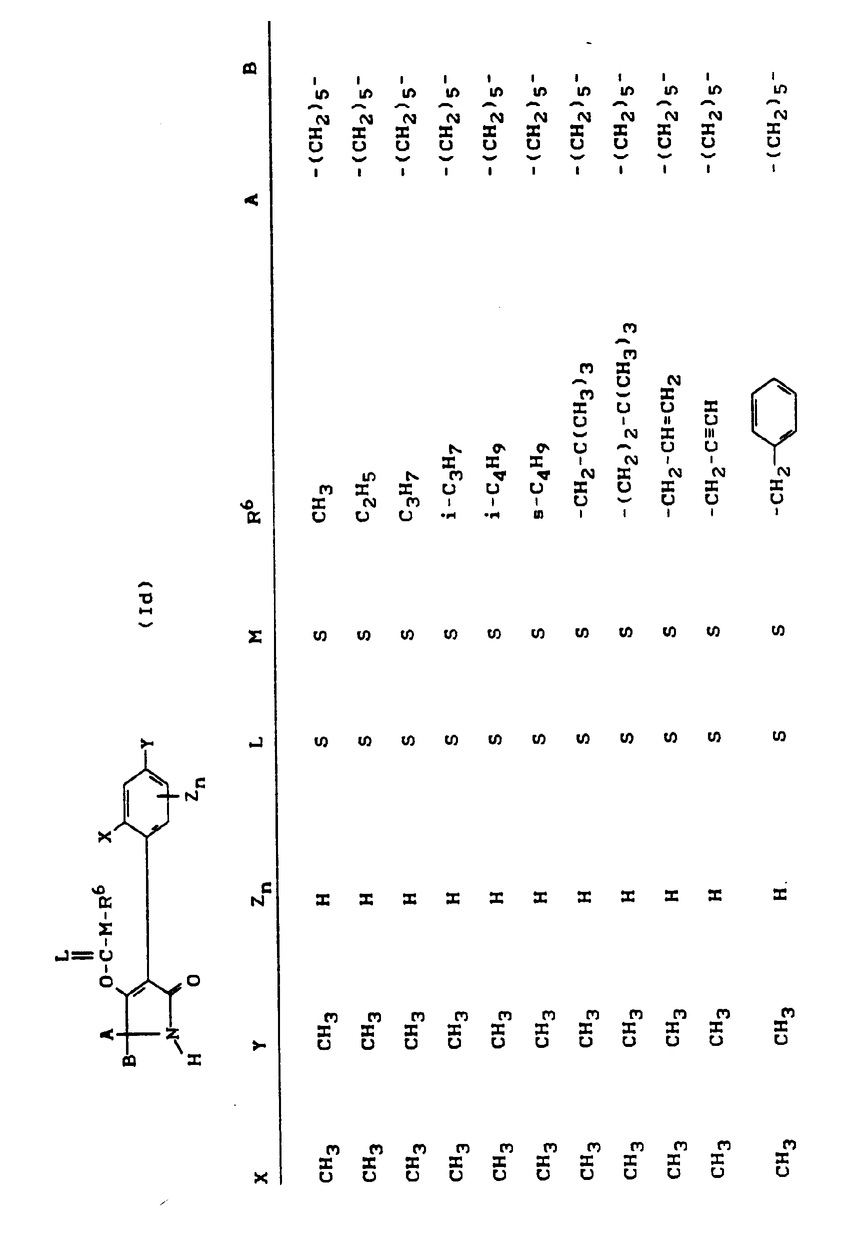

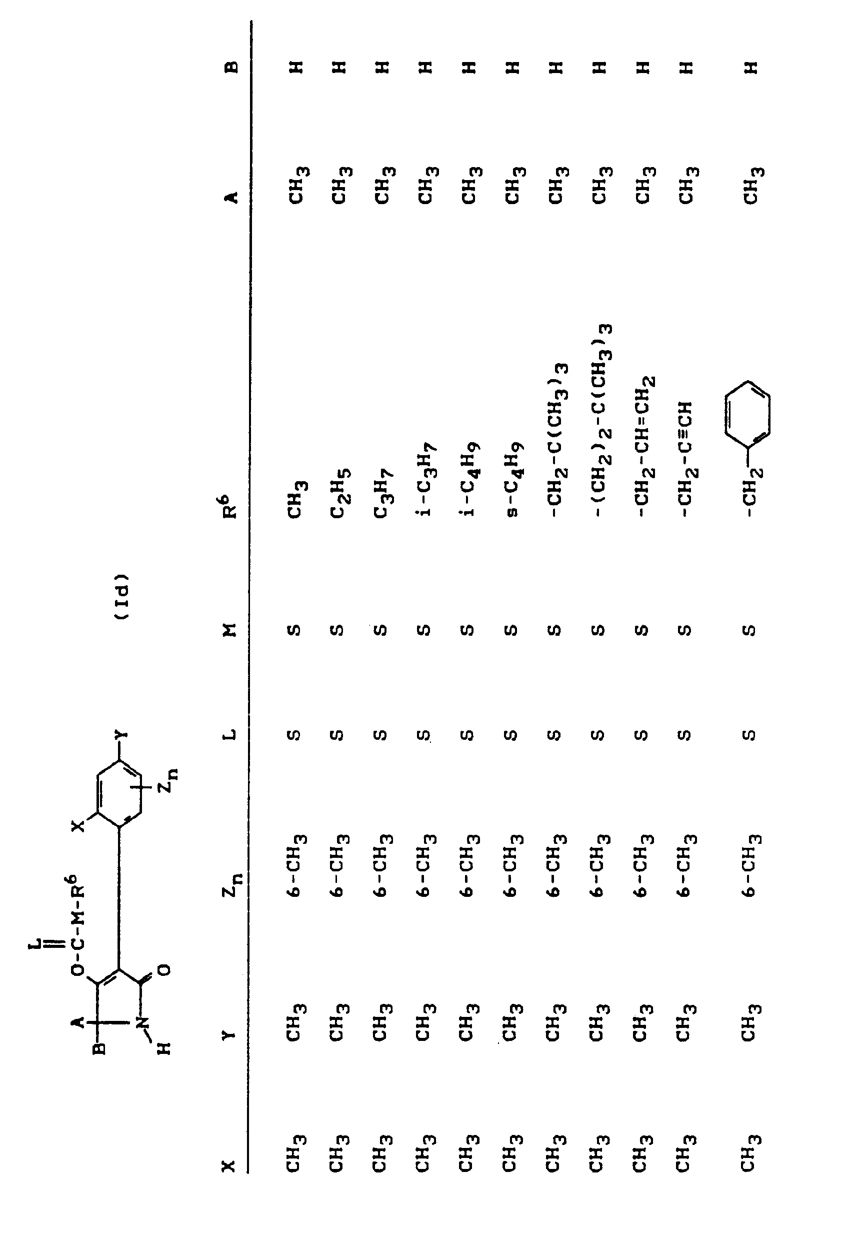

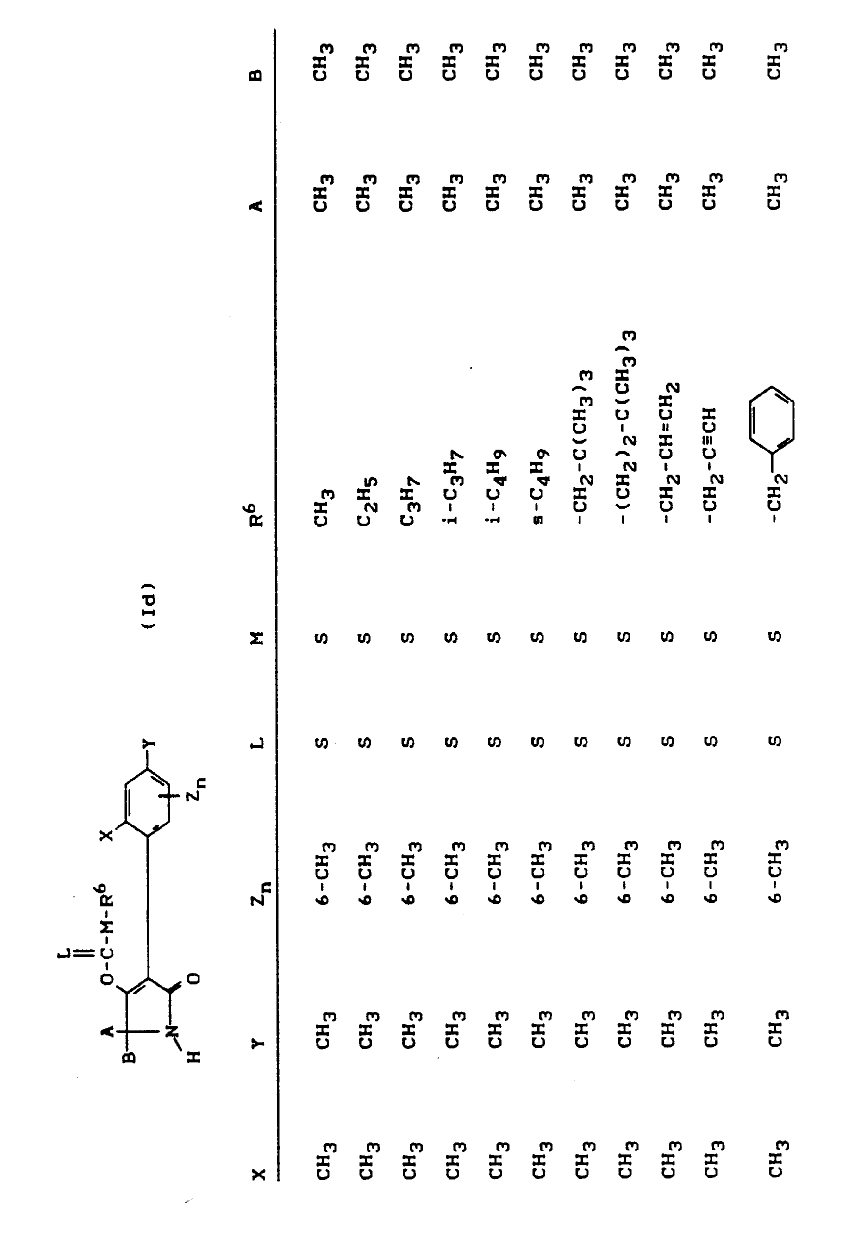

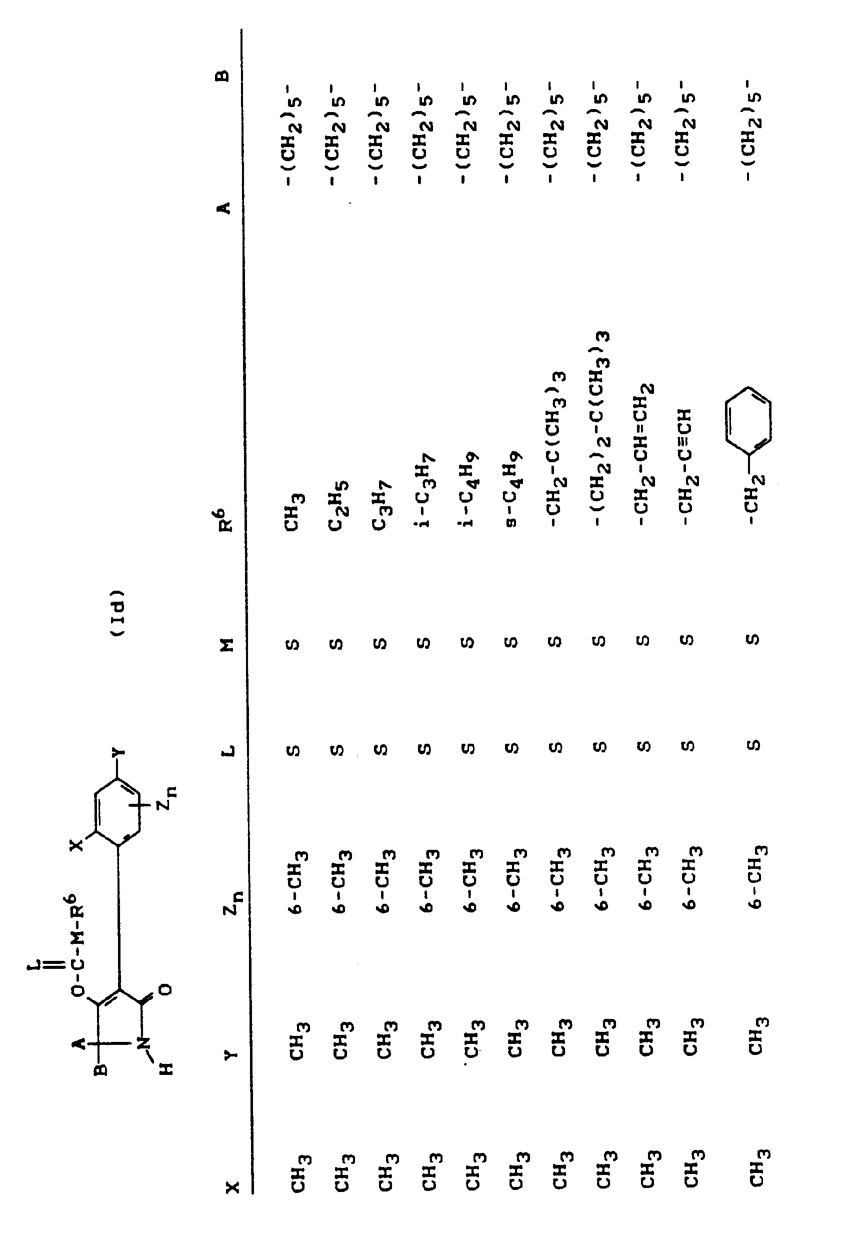

Verwendet man gemäß Verfahren (A) 3-(2,4-Dimethylphenyl)-5-isopropyl-2,4-pyrrolidin-dion und Methanthiophos- phonsäurechlorid-(2,2,2-trifluorethylester) als Ausgangsprodukte, so kann der Reaktionsverlauf durch folgendes Reaktionsschema wiedergegeben werden:

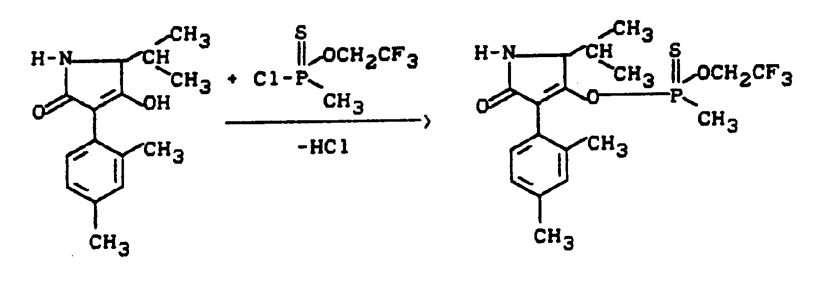

Verwendet man gemäß Verfahren (B) 3-(2,4-Dichlorphenyl)-5-methyl-2,4-pyrrolidin-dion und Methansulfonsäurechlorid als Ausgangsprodukte, so kann der Reaktionsverlauf durch folgendes Reaktionsschema wiedergegeben werden:

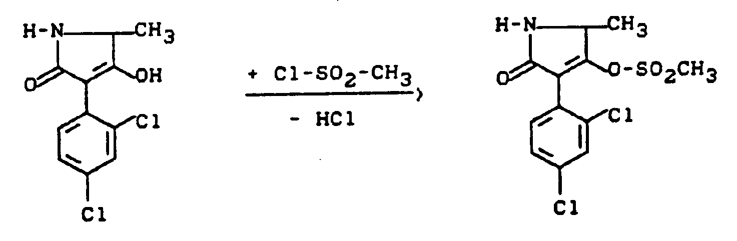

Verwendet man gemäß Verfahren (C_{α}) 3-(2,4,6-Trimethylphenyl)-5,5-pentamethylen-2,4-pyrrolidin-dion und Ethylisocyanat als Ausgangsprodukte, so kann der Reaktionsverlauf durch folgendes Schema wiedergegeben werden:

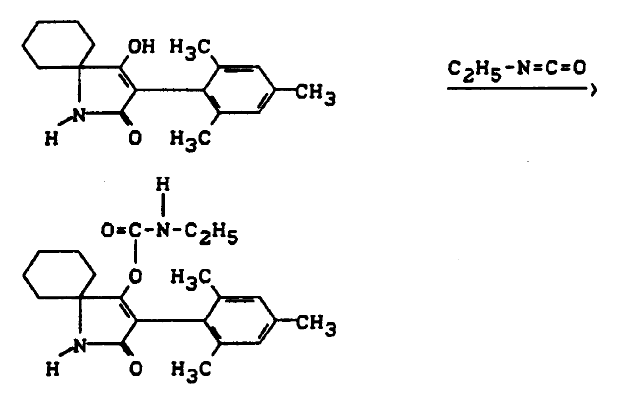

Verwendet man gemäß Verfahren (C_{ß}) 3-(2,4,6-Trimethylphenyl)-5-isopropyl-2,4-pyrrolidin-dion und Dimethylcarbamidsäurechlorid als Ausgangsprodukte, so kann der Reaktionsverlauf wie folgt wiedergegeben werden:

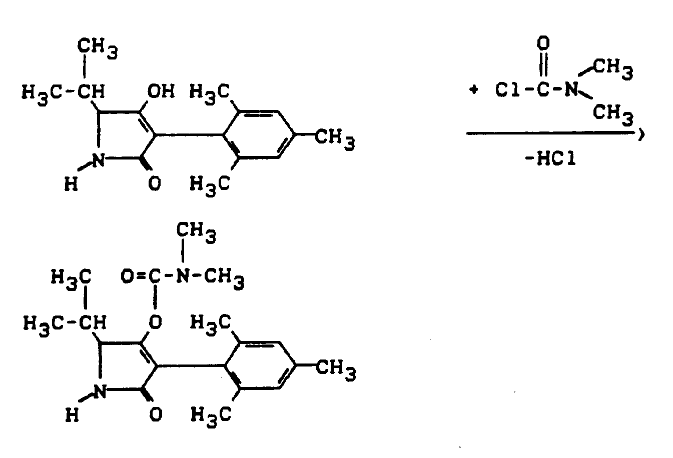

Verwendet man gemäß Verfahren (Dₐ) 3-(2,4,6-Trimethylphenyl)-5,5-dimethyl-2,4-pyrrolidin-dion und Chlormonothioameisensäuremethylester als Ausgangsprodukte, so kann der Reaktionsverlauf wie folgt wiedergegeben werden:

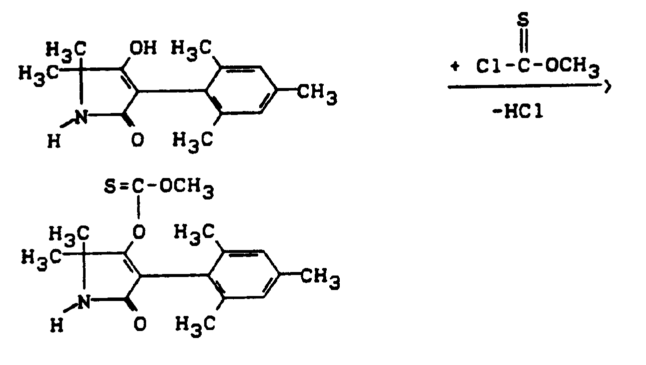

Verwendet man gemäß Verfahren (D_{ß}) 3-(2,4,6-Trimethylphenyl)-5,5-tetramethylen-2,4-pyrrolidin-dion, Schwefelkohlenstoff und Methyliodid als Ausgangskomponenten, so kann der Reaktionsverlauf wie folgt wiedergegeben werden:

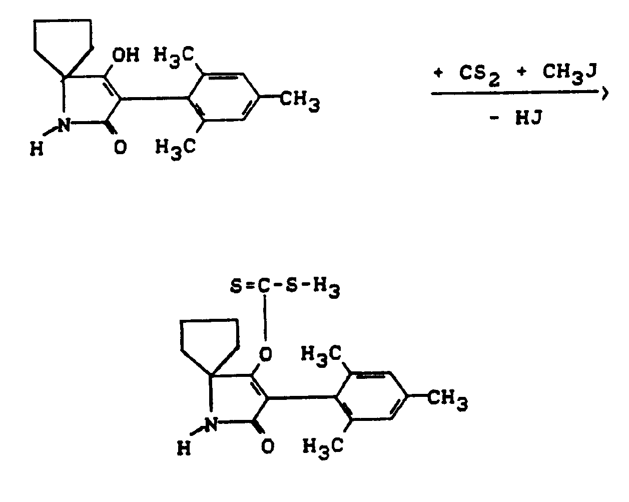

Die bei dem obigen Verfahren (A)-(D) als Ausgangsstoffe benötigten Verbindungen der Formel (11) 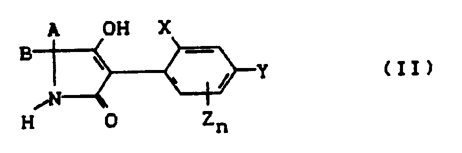 in welcher
A, B, X, Y, Z und n die oben angegebene Bedeutung haben, sind neu, aber Gegenstand früherer eigener Anmeldungen. So erhält man Verbindungen der Formel (II), wenn man
   N-Acylaminosäureester der Formel (IX) 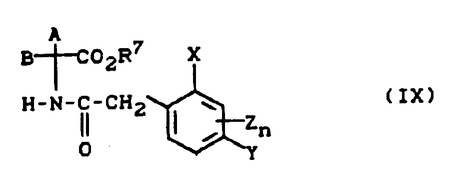 in welcher
A, B, X, Y, Z und n die oben angegebene Bedeutung haben und
R⁷ für Alkyl steht,

in Gegenwart eines Verdünnungsmittels und in Gegenwart einer Base intramolekular kondensiert.

Die bei dem obigen Verfahren als Ausgangsstoffe benötigten Verbindungen der Formel (IX) 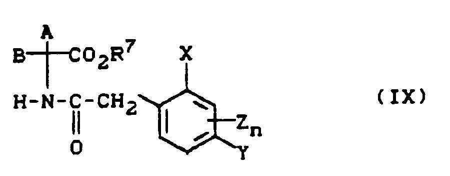 in welcher
A, B, X, Y, Z, n und R⁷ die oben angegebene Bedeutung haben sind teilweise bekannt oder lassen sich nach im Prinzip bekannten Methoden in einfacher Weise herstellen. So erhält man z.B. Acyl-aminosäureester der Formel (IX), wenn man
   a) Aminosäureester der Formel (X), 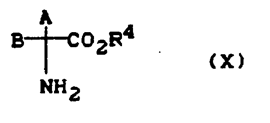 in welcher
      R⁴ für Wasserstoff (Xa) und Alkyl (Xb) steht und
      A und B die oben angegebene Bedeutung haben,

      mit Phenylessigsäurehalogeniden der Formel (XI) 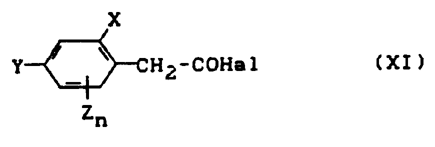 in welcher
      X, Y, Z und n die oben angegebene Bedeutung haben und
      Hal für Chlor oder Brom steht,

      acyliert (Chem. Reviews 52 237-416 (1953));
      oder wenn man Acylaminosäuren der Formel (Ila), 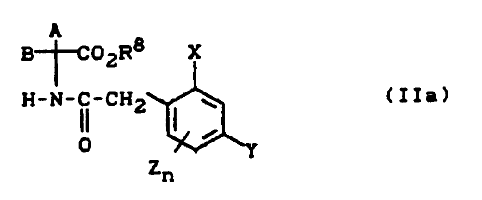 in welcher
      A, B, X, Y, Z und n die oben angegebene Bedeutung haben, und
      _{R}⁸ für Wasserstoff steht,

      verestert (Chem. Ind. (London) 1568 (1968).

Beim Herstellungsverfahren (A) setzt man zum Erhalt von Verbindungen der Struktur (la) auf 1 Mol der Verbindung (11), 1 bis 2, vorzugsweise 1 bis 1,3 Mol der Phosphorverbindung der Formel (III) bei Temperaturen zwischen -40 und 150°C, vorzugsweise zwischen -10 und 110°C um.

Als gegebenenfalls zugesetzte Verdünnungsmittel kommen alle inerten, polaren organischen Lösungsmittel infrage wie halogenierte Kohlenwasserstoffe, Ether, Amide, Nitrile, Alkohole, Sulfide, Sulfone, Sulfoxide etc.

Vorzugsweise werden Acetonitril, Dimethylsulfoxid, Tetrahydrofuran, Dimethylformamid, Dimethylsulfid eingesetzt.

Als gegebenenfalls zugesetzte Säurebindemittel kommen übliche anorganische oder organische Basen infrage wie Hydroxide, Carbonate. Beispielhaft seien Natriumhydroxid, Natriumcarbonat, Kaliumcarbonat, Pyridin aufgeführt..

Die Umsetzung kann bei Normaldruck oder unter erhöhtem Druck durchgeführt werden, vorzugsweise wird bei Normaldruck gearbeitet. Die Aufarbeitung geschieht nach üblichen Methoden der organischen Chemie. Die Reinigung der anfallenden Endprodukte geschieht vorzugsweise durch Kristallisation, chromatographische Reinigung oder durch sogenanntes "Andestillieren", d.h. Entfernung der flüchtigen Bestandteile im Vakuum.

Beim Herstellungsverfahren (B) setzt man pro Mol Ausgangsverbindung der Formel (11) ca. 1 Mol Sulfonsäurechlorid (IV) bei 0 bis 150°C, vorzugsweise bei 20 bis 70°C um.

Als gegebenenfalls zugesetzte Verdünnungsmittel kommen alle inerten, polaren organischen Lösungsmittel infrage wie halogenierte Kohlenwasserstoffe, Ether, Amide, Nitrile, Alkohole, Sulfone, Sulfoxide.

Vorzugsweise werden Methylenchlorid, Dimethylsulfoxid, Tetrahydrofuran, Dimethylformamid, Dimethylsulfid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung II dar, kann auf den weiteren Zusatz von Säurebindemitteln verzichtet werden.

Werden Säurebindemittel eingesetzt, so kommen übliche anorganische oder organische Basen infrage, beispielhaft seien Natriumhydroxid, Natriumcarbonat, Kaliumcarbonat, Pyridin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Beim Herstellungsverfahren (B) kann gegebenenfalls unter Phasen-Transfer-Bedingungen gearbeitet werden (W.J. Spillane et al., J. Chem. Soc. Perkin Trans I, (3) 677-9 (1982)). In diesem Fall setzt man pro Mol Ausgangsverbindung der Formel (11) 0,3 bis 1,5 Mol Sulfonsäurechlorid (IV), bevorzugt 0,5 Mol bei 0 bis 150°C, vorzugsweise bei 20 bis 70°C um. Als Phasen-Transfer-Katalysatoren können z.B. alle quartären Ammoniumsalze verwendet werden, vorzugsweise Tetraoctylammoniumbromid und Benzyltriethylammoniumchlorid. Als organische Lösungsmittel können in diesem Fall alle unpolaren inerten Lösungsmittel dienen, bevorzugt werden Benzol und Toluol eingesetzt.

Beim Herstellungsverfahren (Cₐ) setzt man pro Mol Ausgangsverbindung der Formel (11) ca. 1 Mol Isocyanat der Formel (V) bei 0 bis 100°C, vorzugsweise bei 20 bis 50°C um.

Als gegebenenfalls zugesetzte Verdünnungsmittel kommen alle inerten organischen Lösungsmittel infrage, wie Ether, Amide, Nitrile, Sulfone, Sulfoxide.

Gegebenenfalls können Katalysatoren zur Beschleunigung der Reaktion zugesetzt werden. Als Katalysatoren können sehr vorteilhaft zinnorganische Verbindungen, wie z.B. Dibutylzinndilaurat eingesetzt werden. Es wird vorzugsweise bei Normaldruck gearbeitet.

Beim Herstellungsverfahren (C_{ß}) setzt man pro Mol Ausgangsverbindung der Formel (11) ca. 1 Mol Carbamidsäurechlorid bzw. Thiocarbamidsäurechlorid der Formel (VI) bei 0 bis 150°C, vorzugsweise bei 20 bis 70°C um.

Als gegebenenfalls zugesetzte Verdünnungsmittel kommen alle inerten polaren organischen Lösungsmittel infrage wie Ether, Amide, Alkohole, Sulfone, Sulfoxide.

Vorzugsweise werden Dimethylsulfoxid, Tetrahydrofuran, Dimethylformamid, Dimethylsulfid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung 11 dar, kann auf den weiteren Zusatz von Säurebindemitteln verzichtet werden.

Werden Säurebindemittel eingesetzt, so kommen übliche anorganische oder organische Basen infrage, beispielhaft seien Natriumhydroxid, Natriumcarbonat, Kaliumcarbonat, Pyridin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Beim Herstellungsverfahren (DJ setzt man pro Mol Ausgangsverbindung der Formel (11) ca. 1 Mol Chlormonothioameisensäureester bzw. Chlordithioameisensäureester der Formel (VII) bei 0 bis 120°C, vorzugsweise bei 20 bis 60°C um.

Als gegebenenfalls zugesetzte Verdünnungsmittel kommen alle inerten organischen Lösungsmittel infrage, wie halogenierte Kohlenwasserstoffe, Ether, Amide, Alkohole, Sulfone, Sulfoxide.

Vorzugsweise werden Methylenchlorid, Dimethylsulfoxid, Tetrahydrofuran, Dimethylformamid, Dimethylsulfid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung I dar, kann auf den weiteren Zusatz von Säurebindemitteln verzichtet werden.

Werden Säurebindemittel eingesetzt, so kommen übliche anorganische oder organische Basen infrage, beispielhaft seien Natriumhydroxid, Natriumcarbonat, Kaliumcarbonat, Pyridin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Beim Herstellungsverfahren (D_{ß}) setzt man pro Mol Ausgangsverbindung der Formel (11) die äquimolare Menge bzw. einen Überschuß Schwefelkohlenstoff zu. Man arbeitet hierbei vorzugsweise bei Temperaturen von 0 bis 50°C und insbesondere bei 20 bis 30°C.

Oft ist es zweckmäßig zunächst aus der Verbindung der Formel (11) durch Zusatz eines Deprotonierungsmittels (wie z.B. Kaliumtertiärbutylat oder Natriumhydrid) das entsprechende Salz herzustellen. Man setzt die Verbindung (11) solange mit Schwefelkohlenstoff um bis die Bildung der Zwischenverbindung abgeschlossen ist, z.B. mehrstündiges Rühren bei Raumtemperatur.

Die weitere Umsetzung mit dem Alkylhalogenid der Formel (VIII) erfolgt vorzugsweise bei 0 bis 70°C und insbesondere bei 20 bis 50°C. Hierbei wird mindestens die äquimolare Menge Alkylhalogenid eingesetzt.

Man arbeitet bei Normaldruck oder unter erhöhtem Druck, vorzugsweise bei Normaldruck.

Die Aufarbeitung erfolgt wiederum nach üblichen Methoden.

Die Wirkstoffe eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.
   Aus der Ordnung der Diplopoda z.B. Blaniulus guttulatus.
   Aus der Ordnung der Chilopoda z.B. Geophilus carpophagus, Scutigera spec.
   Aus der Ordnung der Symphyla z.B. Scutigerella immaculata.
   Aus der Ordnung der Thysanura z.B. Lepisma saccharina.
   Aus der Ordnung der Collembola z.B. Onychiurus armatus.
   Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
   Aus der Ordnung der Dermaptera z.B. Forficula auricularia.
   Aus der Ordnung der Isoptera z.B. Reticulitermes spp..
   Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
   Aus der Ordnung der Thysanoptera z. B. Hercinothrips femoralis, Thrips tabaci.
   Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
   Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Acanthoscelides obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Cono derus spp., Melolontha melolontha, Amphimallon solsti tialis, Costelytra zealandica.
   Aus der Ordnung der Hymenoptera z.B. Diprion spp., Hoplocampa spp., Lasius spp., Monomorium pharaonis, Vespa spp.
   Aus der Ordnung der Diptera z. B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z.B. Xenopsylla cheopis, Ceratophyllus spp..
   Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen Wirkstoffe zeichnen sich durch eine hohe insektizide und akarizide Wirksamkeit aus.

Sie lassen sich mit besonders gutem Erfolg zur Bekämpfung von pflanzenschädigenden Insekten, wie beispielsweise gegen die Larven des Meerrettichblattkäfers (Phaedon cochleariae) oder gegen die Larven der grünen Reiszikade (Nephotettix cincticeps) oder gegen pflanzenschädigende Milben, wie beispielsweise gegen die gemeine Spinnmilbe oder die Bohnenspinnmilbe (Tetranychus urticae) einsetzen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten und Endoparasiten) wie Schildzecken, Lederzecken, Räudemilben, Laufmilben, Fliegen (stechend und leckend), parasitierende Fliegenlarven, Läuse, Haarlinge, Federlinge, Flöhe und endparasitisch lebende Würmer.

Sie sind gegen normalsensible und resistente Arten und Stämme sowie gegen alle parasitierenden und nicht parasitierenden Entwicklungsstadien der Ekto- und Endoparasiten wirksam.

Die erfindungsgemäßen Wirkstoffe können weiterhin als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.
Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.
Dikotyle Kulturen der Gattungen: Gossypium, Glycine, Beta, Daucus, Phaseolus, Pisum, Solanum, Linum, lpomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.
Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee- , Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und Verwendung der erfindungsgemäßen Wirkstoffe geht aus den nachfolgenden Beispielen hervor.

### Herstellungsbeispiele:

### Beispiel 1

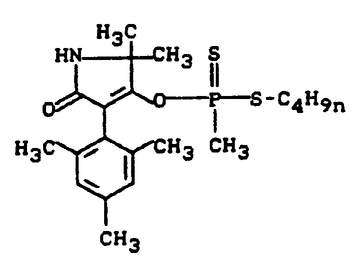

4 g (16,3 mmol) 3-(2,4,6-Trimethylphenyl)-5,5-dimethylpyrrolidin-2,4-dion werden in 10 ml Tetrahydrofuran gelöst. Dazu werden 2,5 ml (18 mmol) Triethylamin und danach bei Raumtemperatur 3,6 g (17,8 mmol) Methan-dithiophos- phonsäure-s-(n-butylester)-chlorid gegeben. Der Ansatz wird ca. zwei Stunden bei 50°C gerührt und das Reaktionsende chromatographisch überprüft. Nach dem Abdestillieren des Lösungsmittels wird der verbleibende Rückstand über eine Kieselgelfritte (Laufmittel Toluol: Essigester 8:2) gereinigt.

Man erhält 1,6 g (29,2% der Theorie) Methan-dithiophosphonsäure-0-[3-(2,4,6-trimethylphenyl)-5,5-dimethylpyrro- lidin-2-on-]-S-(n-butylester) vom Schmelzpunkt 98°C.

### Beispiel 2

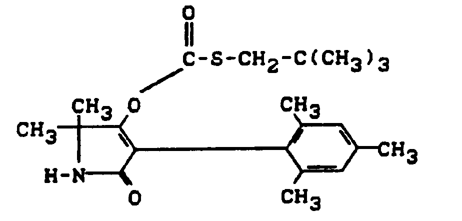

3,68 g (15 mmol) 3-(2,4,6-Trimethylphenyl)-5,5-dimethylpyrrolidin-2,4-dion werden in 60 ml absolutem Methylenchlorid vorgelegt. Dazu werden 2,3 ml (16,5 mmol) Triethylamin und danach bei 0 bis 10°C 2,75 g (16,5 mmol) Chlorthiokohlensäure-S-(2,2-dimethylpropyl)-ester gelöst in 15 ml absolutem Methylenchlorid zugetropft. Der Ansatz wird ca. 2 Stunden bei Raumtemperatur gerührt und das Reaktionsende chromatographisch überprüft. Das Reaktionsgemisch wird nacheinander mit 10 %iger Citronensäure, Natriumhydrogencarbonat- und Kochsalzlösung gewaschen, die organische Phase getrocknet und das Lösungsmittel abdestilliert. Nach dem Umkristallisieren aus Essigester/Hexan 1:4 erhält man 2,74 g (49 % der Theorie)Thiolkohlensäure-S-(2,2-dimethylpropyl)-ester-O-[3-(2,4,6-trimethylphenyl)-5,5-dimethylpyrrolidin-2-on] vom Schmelzpunkt 197-200°C.

### Beispiel 3

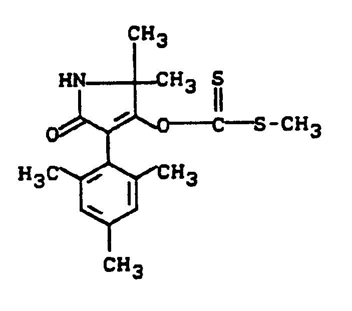

4,91 g (20 mmol) 3-(2,4,6-Trimethylphenyl)-S,S-dimethylpyrrolidin-2,4₋dion werden in 40 ml wasserfreiem Dimethylformamid gelöst. Dazu werden 1,08 g Natriummethanolat gegeben und der Ansatz ca. 10 Minuten nachgerührt. Nach der Zugabe von 1,17 ml Schwefelkohlenstoff wird 3 Stunden bei Raumtemperatur gerührt und anschließend 1,24 ml Methyljodid zugetropft. Der Reaktionsansatz wird weitere 3 Stunden bei Raumtemperatur gerührt und das Reaktionsende chromatographisch überprüft. Das Reaktionsgemisch wird in 120 ml Wasser eingerührt, der Niederschlag abgesaugt, das Filtrat in Dichlormethan aufgenommen und mit 200 ml 0,5 N Natronlauge gewaschen. Die organische Phase wird über Magnesiumsulfat getrocknet und eingeengt. Das erhaltene Rohprodukt wird in 10 ml Essigester heiß aufgeschlämmt und abgesaugt. Man erhält 2,1 g (31,3 % der Theorie) Thiolthionkohlensäure-S-methyl-O-[3-(2,4,6-trimethylphenyl)-5,5-dimethylpyrrolidin-2-on] vom Schmelzpunkt 214-215°C.

In analoger Weise zu den Beispielen 1, 2 und 3 und unter Berücksichtigung der Angaben in der Beschreibung zu den erfindungsgemäßen Verfahren, werden die nachfolgend in Tabelle 5 aufgeführten Endprodukte der Formel (I) erhalten.

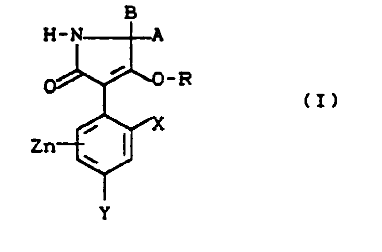

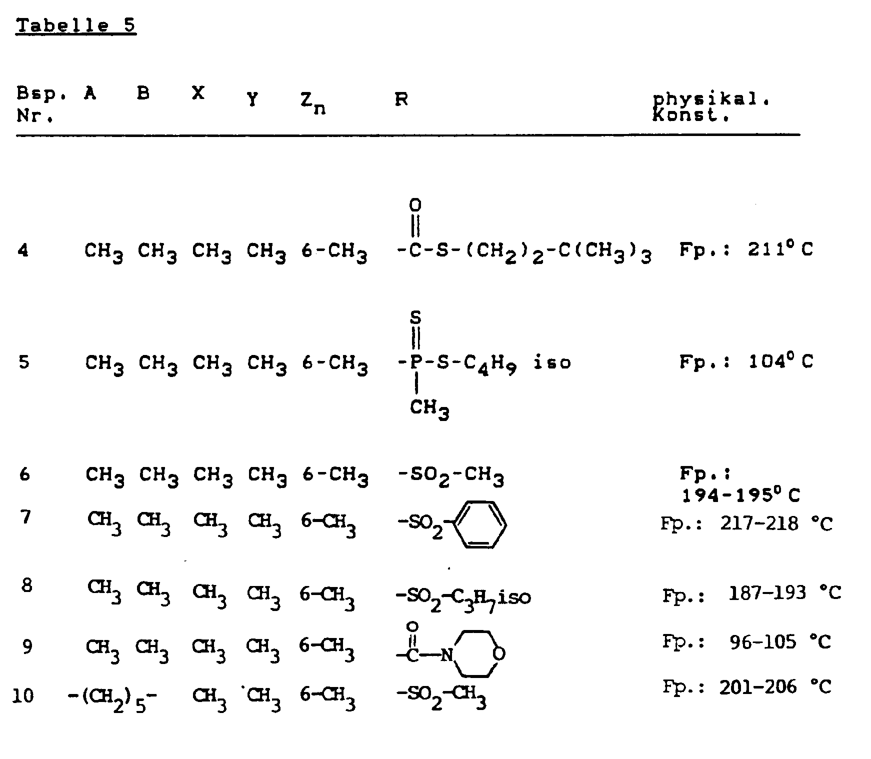

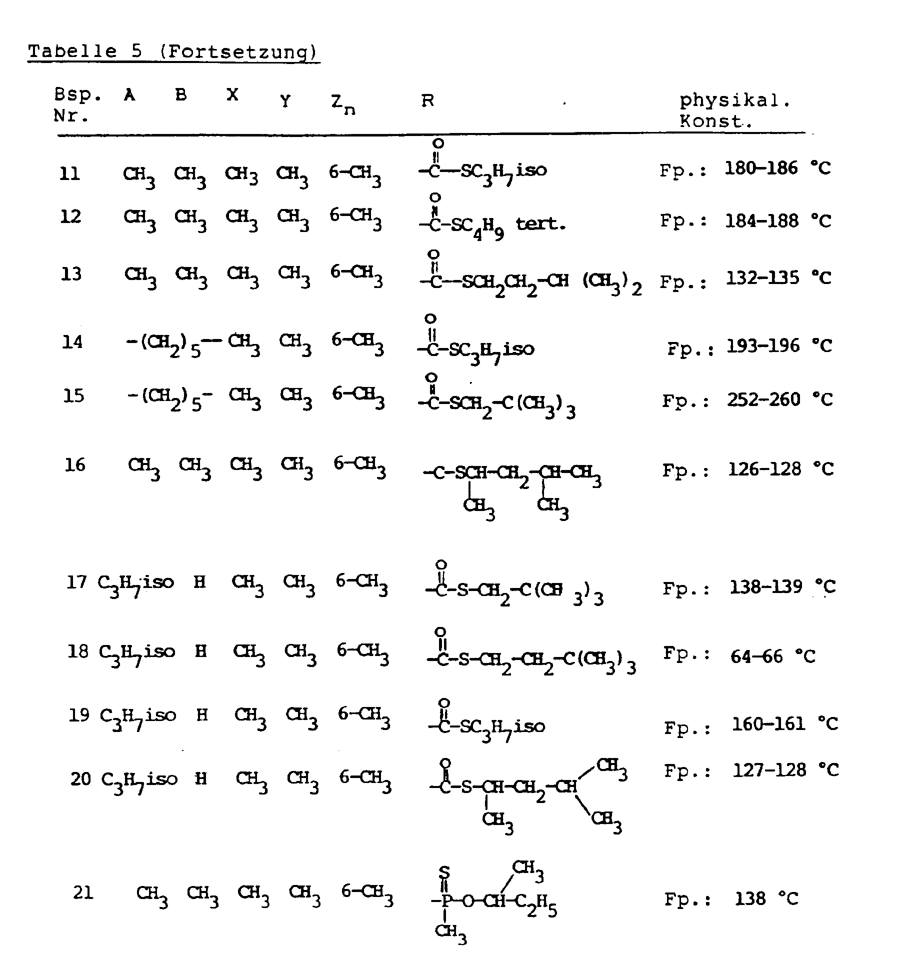

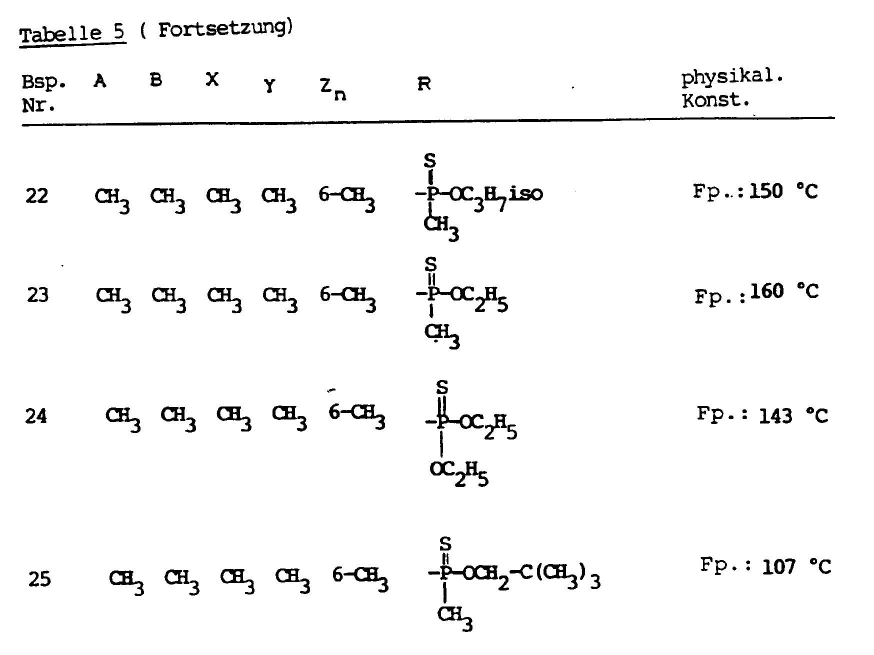

### Anwendungsbeispiele:

In den folgenden Anwendungsbeispielen wurden die nachstehend aufgeführten Verbindungen als Vorgleichssubstanzen eingesetzt:

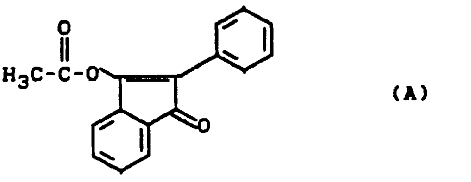

### 3-(Acetyloxy)-2-phenyl-1H-inden-1-on

### (bekannt aus US 4 104 043)

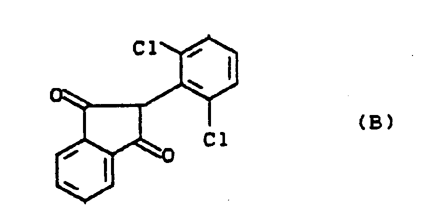

### 2-(2,6-Dichlorphenyl)-1 H-inden-1,3(2H)-dion

### (bekannt aus US 3 954 998)

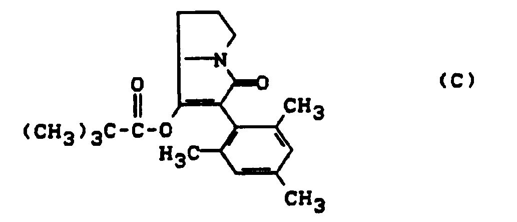

### 2,2-Dimethyl-2,3,5, 7a-tetrahydro-5-oxo-6-(2,4,6-trimethylphenyl)-1H-pyrrolizin-7-yl-propionsäureester

### (bekannt aus EP-A 355 599).

### Beispiel A

### Tetranychus-Test (OP-resistent)

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

Bei diesem Test zeigen z.B. die folgenden Verbindungen der Herstellungsbeispiele überlegene Wirksamkeit gegenüber dem Stand der Technik: 1, 2 und 5.

### Beispiel B

### Nephotettix-Test

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichstteil Wirkstoff mit der angegebenen Menge Lösungsmittel und der angegebenen Menge Emulgator und verdünnt das Konzentrat mit Wasser auf die gewünschte Konzentration.

Reiskeimlinge (Oryza sativa) werden durch Tauchen in die Wirkstoffzubereitung der gewünschten Konzentration behandelt und mit der Grünen Reiszikade (Nephotettix cincticeps) besetzt, solange die Keimlinge noch feucht sind.

Nach der gewünschten Zeit wird die Abtötung in % bestimmt. Dabei bedeutet 100 %, daß alle Zikaden abgetötet wurden; 0 % bedeutet, daß keine Zikaden abgetötet wurden.

Bei diesem Test zeigen z.B. die folgenden Verbindungen der Herstellungsbeispiele überlegene Wirksamkeit gegenüber dem Stand der Technik: 1, 2 und 5.

### Beispiel C

### Phaedon-Larven-Test

Lösungsmittel: 3 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käferlarven abgetötet wurden; 0 % bedeutet, daß keine Käfer-Larven abgetötet wurden.

Bei diesem Test zeigen z.B. die folgenden Verbindungen der Herstellungsbeispiele überlegene Wirksamkeit gegenüber dem Stand der Technik: 1, 2, 4 und 5.

### Beispiel D

### Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100 % = totale Vernichtung

In diesem Test zeigt beispielsweise die Verbindung gemäß Herstellungsbeispiel (2) starke Wirkung gegen Unkräuter.

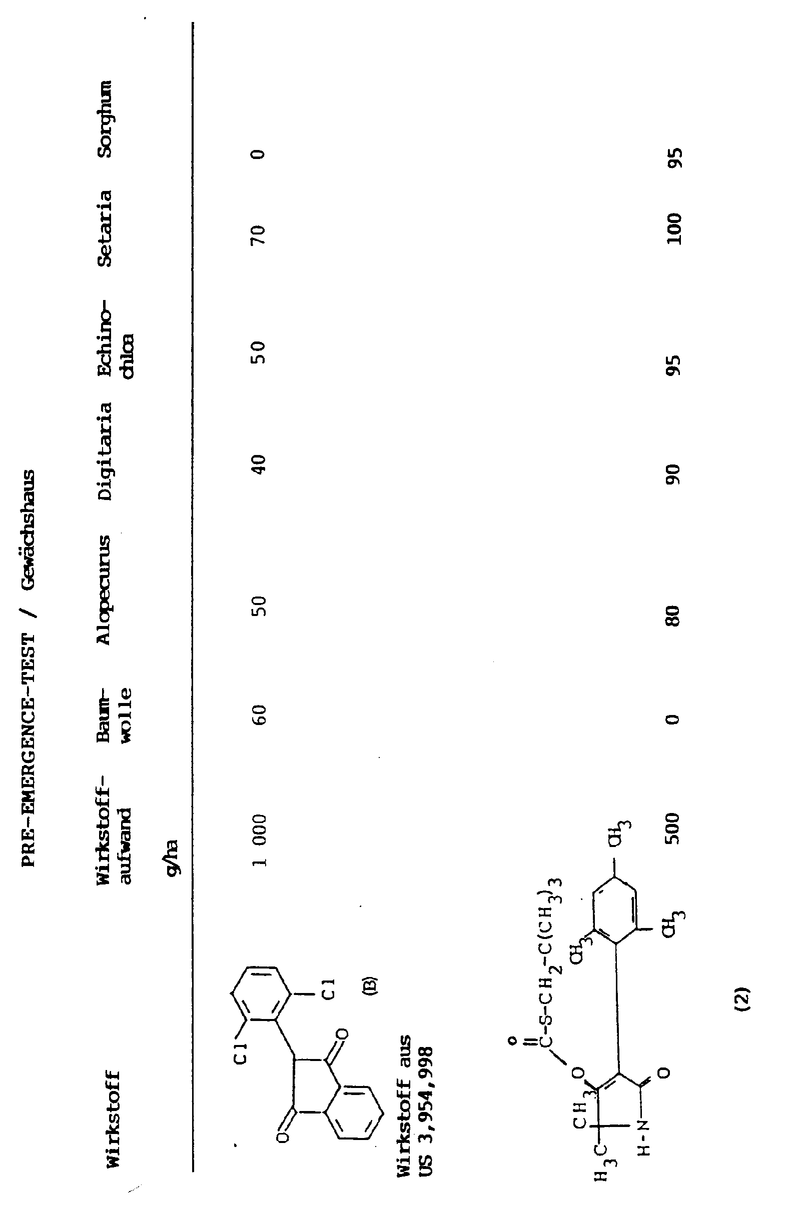

## Patentansprüche (Patentansprüche für folgende(n) Vertragsstaat(en) : BE, CH, DE, FR, GB, GR, IT, LI, NL)

1. Substituierte 3-Aryl-pyrrolidin-2,4-dion-Derivate der allgemeinen Formel (I) 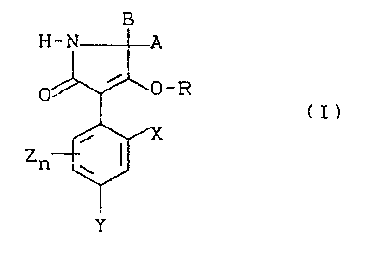 in welcher
X für C₁-C₆-Alkyl, Halogen, C₁-C₆-Alkoxy steht.
Y für Wasserstoff. C₁-C₆-Alkyl, Halogen, C₁-C₆-Alkoxy, C₁-C₃-Halogenalkyl steht,
Z für C₁-C₆-Alkyl, Halogen, C₁-C₆-Alkoxy steht,
n für eine Zahl von 0-3 steht,
A für Wasserstoff oder gegebenenfalls durch Halogen substituiertes geradkettiges oder verzweigtes C₁-C₁₂-Alkyl, C₃-C₈-Alkenyl, C₃-C₈-Alkinyl, C₁-C₁₀-Alkoxy-C₂-C₈-alkyl,C₁-C₈-Polyalkoxy-C₂-C₈-alkyl, C₁-C₁₀-Alkylthio-C₂-C₈-alkyl, Cycloalkyl mit 3-8 Ringatomen das durch Sauerstoff und/oder Schwefel unterbrochen sein kann
B für Wasserstoff, geradkettiges oder verzweigtes C₁-C₁₂-Alkyl, C₁-C₈-Alkoxyalkyl steht.
oder worin
A und B gemeinsam mit dem Kohlenstoffatom. an das sie gebunden sind, einen 3- bis 8-gliedrigen gesättigten oder ungesättigten Ring bilden, der durch Sauerstoff und/oder Schwefel unterbrochen und durch gegebenenfalls halogeniertes Alkyl. Alkoxy, Phenyl und Halogen substituiert sein kann,
R für die Gruppen 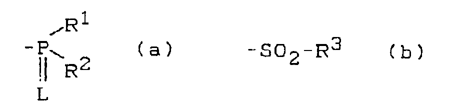 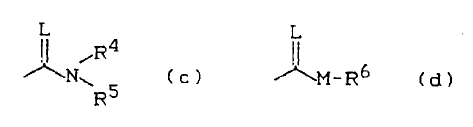 steht,
in welchen
L und M jeweils für Sauerstoff oder Schwefel steht und wobei L und M nicht gleichzeitig für Sauerstoff stehen,
R¹, R² und R³ unabhängig voneinander für gegebenenfalls durch Halogen substituiertes C₁-C₈-Alkyl, C₁-C₈-Alkoxy, C₁-C₈-Alkylamino, Di-(C₁-C₈)-Alkyl-amino C₁-C₈-Alkylthio C₂-C₅-Alkenylthio, C₂-C₅-Alki- nylthio, C₃-C₇-Cycloalkylthio für gegebenenfalls durch Halogen, Nitro, Cyano, C₁-C₄-Alkoxy, C₁-C₄-Halogenalkoxy, C₁-C₄-Alkylthio, C₁-C₄-Halogenalkylthio C₁-C₄-Alkyl, C₁-C₄-Halogenalkyl substituiertes Phenyl, Benzyl, Phenoxy oder Phenylthio stehen.
R⁴ und R⁵ unabhängig voneinander für gegebenenfalls durch Halogen substituiertes C₁-C₂₀-Alkyl, C₁-C₂₀-Alkoxy, C₂-C₈-Alkenyl, C₁-C₂₀-Alkoxy-C₁-C₂₀-alkyl, für gegebenenfalls durch Halogen, C₁-C₂₀-Halogen-alkyl, C₁-C₂₀-Alkyl oder C₁-C₂₀-Alkoxy substituiertes Phenyl, für gegebenenfalls durch Halogen, C-₁C₂₀-Alkyl, C₁-C₂₀-Halogenalkyl oder C₁-C₂₀-Alkoxy substituiertes Benzyl steht oder zusammen für einen gegebenenfalls durch Sauerstoff unterbrochenen C₂-C₆-Alkylenring stehen,
R⁶ für gegebenenfalls durch Halogen substituiertes C₁-C₂₀-Alkyl, das durch Sauerstoff unterbrochen sein kann, für gegebenenfalls durch Halogen, C₁-C₂ₒ-Halogenalkyl, C₁-C₂₀-Alkoxy substituiertes Phenyl, für gegebenenfalls durch Halogen, C₁-C₂₀-Halogenalkyl oder C₁-C₂₀-Alkoxy substituiertes Benzyl, für C₂-C₈-Alkenyl oder für C₂-C₅-Alkinyl steht,
sowie die enantiomerenreinen Formen von Verbindungen der Formel (I).

2. Substituierte 3-Aryl-pyrrolidin-2,4-dion-Derivate der Formeln (la) bis (Id) 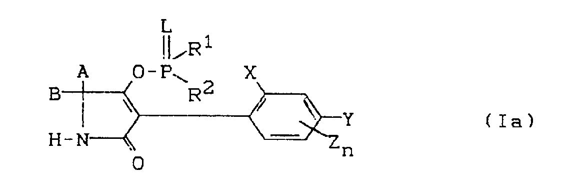 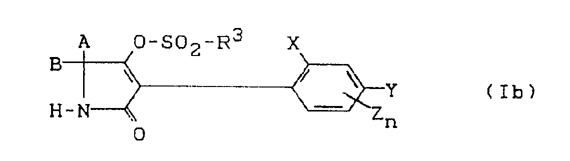 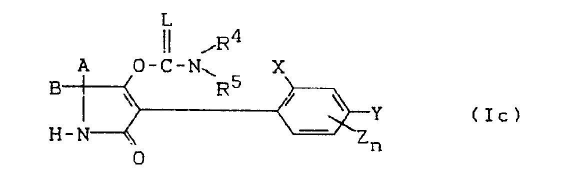 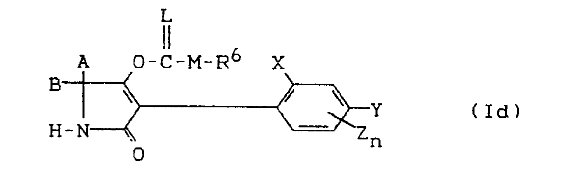 worin
_{A}, _{B}, L, M, X, Y, Zₙ, R¹, R², R³, R⁴, R⁵ und R⁶ die im Anspruch 1 angegebene Bedeutung haben.

3. Substituierte 3-Aryl-pyrrolidin-2,4-dion-Derivate der allgemeinen Formel (I) gemäß Anspruch 1, in welcher
X für C₁-C₄-Alkyl, Halogen, C₁-C₄-Alkoxy steht,
Y für Wasserstoff, C₁-C₆-Alkyl, Halogen, C₁-C₄-Alkoxy, C₁-C₂-Halogenalkyl steht,
Z für C₁-C₄-Alkyl, Halogen, C₁-C₄-Alkoxy steht,
n für eine Zahl von 0-3 steht,
A für Wasserstoff oder gegebenenfalls durch Halogen substituiertes geradkettiges oder verzweigtes C₁-C₁₀-Alkyl, C₃-C₆-Alkenyl, C₃-C₆-Alkinyl, C₁-C₈-Alkoxy-C₂-C₆-alkyl, C₁-C₆-Poly-alkoxy-C₂-C₆-alkyl, C₁-C₈-Alkylthio-C₂-C₆-alkyl, Cycloalkyl mit 3-7 Ringatomen, das durch 1-2 Sauerstoff- und/oder Schwefelatomen unterbrochen sein kann, steht,
B für Wasserstoff, geradkettiges oder verzweigtes C₁-C₁₀-Alkyl, C₁-C₆-Alkoxyalkyl steht,
oder worin
A und B gemeinsam mit dem Kohlenstoffatom, an das sie gebunden sind, einen 3- bis 7-gliedrigen gesättigten oder ungesättigten Ring bilden, der durch C₁-C₆-Alkyl, C₁-C₆-Alkoxy, C₁-C₃-Haloalkyl, C₁-C₃-Haloalk- oxy, Fluor, Chlor substituiert und der durch Sauerstoff und/oder Schwefel unterbrochen sein kann.
R für die Gruppen 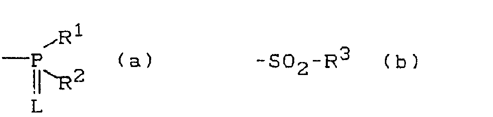 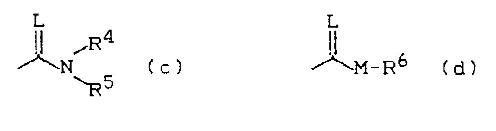 in welchen
L und M jeweils für Sauerstoff oder Schwefel stehen und worin L und M nicht gleichzeitig für Sauerstoff stehen.
R¹, R² und R³ unabhängig voneinander für gegebenenfalls durch Halogen substituiertes Cᵢ-C₆-Alkyl, C₁-C₆-Alkoxy, C₁-C₆-Alkylamino, Di-(C₁-C₆)-Alkylamino, C₁-C₆-Alkylthio, C₃-C₄-Alkenylthio, C₂-C₄-Alki- nylthio, C₃-C₆-Cycloalkylthio, für gegebenenfalls durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl, C₁-C₃-Halogenalkyl substituiertes Phenyl, Benzyl, Phenoxy oder Phenylthio stehen,
R⁴ und R⁵ unabhängig voneinander für gegebenenfalls durch Halogen substituiertes C₁-C₂₀-Alkyl, C₁-C₂₀-Alkoxy, C₂-C₈-Alkenyl, C₁-C₂₀-Alkoxy-C₁-C₂₀-alkyl, für gegebenenfalls durch Halogen, C₁-C_{S}-Halogenalkyl, C₁-C₅-Alkyl oder C₁-C₅-Alkoxy substituiertes Phenyl, für gegebenenfalls durch Halogen, C₁-C₅-Alkyl, C₁-C₅-Halogenalkyl oder C₁-C₅-Alkoxy substituiertes Benzyl steht,
R⁶ für gegebenenfalls durch Halogen substituiertes C₁-C₂₀-Alkyl, das durch Sauerstoff unterbrochen sein kann, für gegebenenfalls durch Halogen C₁-C₅-Halogenalkyl, C₁-C₅-Alkoxy substituiertes Phenyl, für gegebenenfalls durch Halogen, C₁-C₅-Halogenalkyl oder C₁-C₅-Alkoxy substituiertes Benzyl steht,
sowie die enantiomerenreinen Formen von Verbindungen der Formel (I).

4. Substituierte 3-Aryl-pyrrolidin-2,4-dion-Derivate der allgemeinen Formel (I) gemäß Anspruch 1, in welcher
X für Methyl, Ethyl, Propyl, i-Propyl, Fluor, Chlor, Brom, Methoxy und Ethoxy steht,
Y für Wasserstoff, Methyl, Ethyl, Propyl, i-Propyl, Butyl, i-Butyl, tert.-Butyl, Fluor, Chlor, Brom, Methoxy, Ethoxy und Trifluormethyl steht,
Z für Methyl, Ethyl, i-Propyl, Butyl, i-Butyl, tert.-Butyl, Fluor, Chlor, Brom, Methoxy und Ethoxy steht,
n für eine Zahl von 0-3 steht,
A für Wasserstoff, gegebenenfalls durch Halogen substituiertes geradkettiges oder verzweigtes C₁-C₈-Alkyl, C₃-C₄-Alkenyl, C₃-C₄-Alkinyl, C₁-C₆-Alkoxy-C₂-C₄-alkyl, C₁-C₄-Polyalkoxy-C₂-C₄-alkyl, C₁-C₆-Alkylthio-C₂-C₄-alkyl, Cycloalkyl mit 3-6 Ringatomen, das durch 1-2 Sauerstoff-und/oder Schwefelatomen unterbrochen sein kann, steht,
B für Wasserstoff, geradkettiges oder verzweigtes C₁-C₈-Alkyl, C₁-C₄-Alkoxyalkyl steht,
oder worin
A und B gemeinsam mit dem Kohlenstoffatom, an das sie gebunden sind, einen 3- bis 6-gliedrigen gesättigten oder ungesättigten Ring bilden, der durch C₁-C₄-Alkyl, C₁-C₄-Alkoxy, Trifluormethyl, Trifluormethoxy, Fluor, Chlor substituiert und der durch Sauerstoff und/oder Schwefel unterbrochen sein kann,
R für die Gruppen 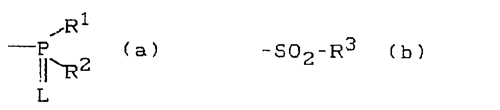 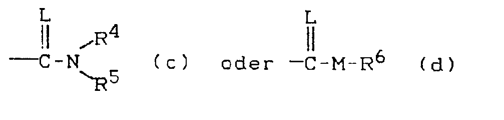 steht,
in welchen
L und M jeweils für Sauerstoff oder Schwefel stehen und worin L und M nicht gleichzeitig für Sauerstoff stehen.
R¹, R² und R³ unabhängig voneinander für gegebenenfalls durch Fluor oder Chlor substituiertes C₁-C₄-Alkyl, C₁-C₄-Alkoxy, C₁-C₄-Alkylamino, Di-(C₁-C₄-Alkyl)amino, C₁-C₄-Alkylthio für gegebenenfalls durch Fluor, Chlor, Brom, Nitro, Cyano, C₁-C₂-Alkoxy, C₁-C₄-Fluoralkoxy, C₁-C₂-Chloralkoxy, C₁-C₂-Alkylthio, C₁-C₂-Fluoralkylthio C₁-C₂-Chloralkylthio, C₁-C₃-Alkyl substituiertes Phenyl, Benzyl, Phenoxy oder Phenylthio stehen.
R⁴ und R⁵ unabhängig voneinander für gegebenenfalls durch Fluor, Chlor, Brom substituiertes C₁-C₁₀-Alkyl, C₁-C₁₀-Alkoxy, C₁-C₁₀-Alkoxy-(C₁-C₁₀)alkyl, für gegebenenfalls durch Fluor, Chlor, Brom, C₁-C₂₀-Halogenalkyl, C₁-C₂₀-Alkyl oder C₁-C₄-Alkoxy substituiertes Phenyl, für gegebenenfalls durch Fluor, Chlor, Brom, C₁-C₄-Alkyl, C₁-C₄-Halogenalkyl oder C₁-C₄-Alkoxy substituiertes Benzyl steht,
R⁶ für gegebenenfalls durch Fluor, Chlor, Brom substituiertes C₁-C₁₀-Alkyl, das durch Sauerstoff unterbrochen sein kann, für gegebenenfalls durch Fluor, Chlor, Brom, Cᵢ-C₄-Halogenalkyl, C₁-C₄-Alkoxy substituiertes Phenyl, für gegebenenfalls durch Fluor, Chlor, Brom, C₁-C₄-Halogenalkyl oder C₁-C₄-Alkoxy substituiertes Benzyl steht,
sowie die enantiomerenreiner Formen von Verbindungen der Formel (I).

5. Verfahren zur Herstellung von substituierten 3-Aryl-pyrrolidin-2,4-dion-Derivaten der allgemeinen Formel (I) gemäß Anspruch 1, dadurch gekennzeichnet, daß man zum Erhalt von substituierten 3-Aryl-pyrrolidin-2,4-dionen der Formel (la) 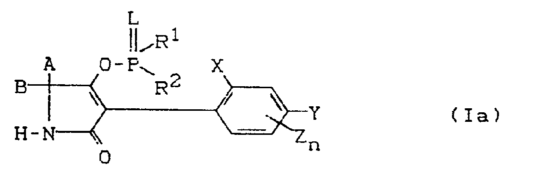 in welcher
A B, L, X, Y, Z, R¹, R² und n die in Anspruch angegebene Bedeutung haben,
A) 3-Aryl-pyrrolidin-2,4-dione der Formel (II) bzw. deren Enole 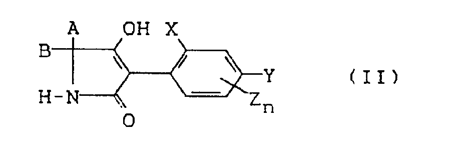 in welcher
A, B, X, Y, Z und n die oben angegebene Bedeutung haben
mit Phosphorverbindungen der allgemeinen Formel (III) 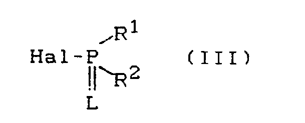 in welcher
L, R¹ und R² die oben angegebene Bedeutung haben und
Hal für Halogen, insbesondere Chlor und Brom steht,
gegebenenfalls in Gegenwart eines Verdünnungsmittels, gegebenenfalls in Gegenwart eines Säurebindemitteis und gegebenenfalls in Gegenwart eines Phasentransferkatalysators umsetzt,
B) oder daß man zum Erhalt von Verbindungen der Formel (Ib) 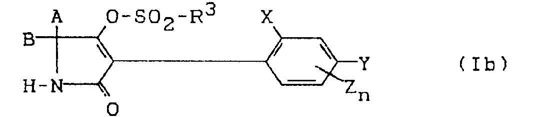 in welcher
A, B, X, Y, Z, R³ und n die Anspruch 1 angegebene Bedeutung haben.
Verbindungen der Formel (II) 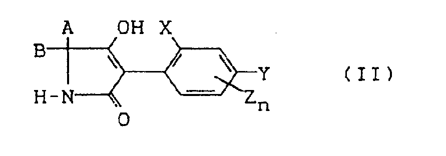 in welcher
A, B, X, Y, Z und n die oben angegebene Bedeutung haben
mit Sulfonsäurechloriden der allgemeinen Formel (IV) 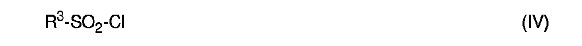 in welcher
R³ die oben angegebene Bedeutung hat
gegebenenfalls in Gegenwart eines Verdünnungsmittels und gegebenenfalls in Gegenwart eines Säurebindemittels,
C) oder daß man zum Erhalt von Verbindungen der Formel (Ic) 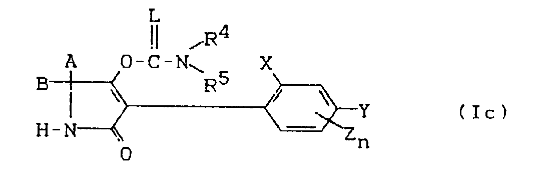 in welcher
A, B, L, X, Y, Z, R⁴, R⁵ und n die in Anspruch 1 angegebene Bedeutung haben,
Verbindungen der Formel (II), 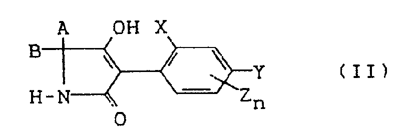 in welcher
A, B, X, Y, Z und n die oben angegebene Bedeutung haben
entweder
a) mit Isocyanaten der allgemeinen Formel (V) 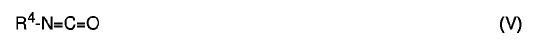 in welcher
R⁴ die oben angegebene Bedeutung hat gegebenenfalls in Gegenwart eines Verdünnungsmittels und gegebenenfalls in Gegenwart eines Katalysators
oder
oder
β) mit Carbamidsäurechloriden oder Thiocarbamidsäurechloriden der allgemeinen Formel (VI) 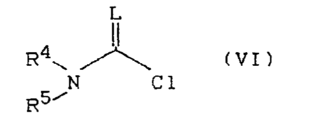 in welcher
L, R⁴ und R⁵ die oben angegebene Bedeutung haben
gegebenenfalls in Gegenwart eines Verdünnungsmittels und gegebenenfalls in Gegenwart eines Säurebindemittels,
umsetzt,
D) oder daß man zum Erhalt von Verbindungen der Formel (Id) 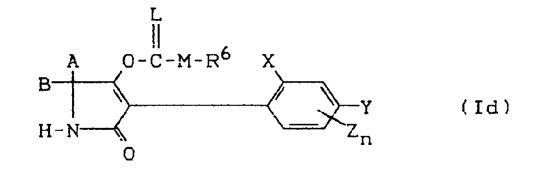 in welcher
A, B, L, M, R⁶, X, Y, Z und n die in Anspruch 1 angegebene Bedeutung haben. Verbindungen der Formel (II) 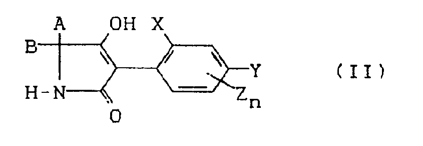 in welcher
A, B, X, Y, Z und n die oben angegebene Bedeutung haben. entweder
a) mit Chlormonothioameisensäureestern. Chlorameisensäurethioestern oder Chlordithioameisensäureestern der allgemeinen Formel VII 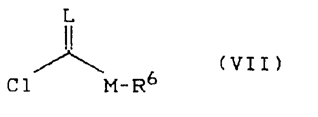 in welcher
L, M, R⁶ die oben angegebene Bedeutung haben
gegebenenfalls in Gegenwart eines Verdünnungsmittels und gegebenenfalls in Gegenwart eines Säurebindemittels umsetzt, oder
β) mit Schwefelkohlenstoff und anschließend mit Alkylhalogeniden der allgemeinen Formel VIII 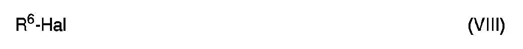 in welcher
R⁶ die oben angegebene Bedeutung hat und
Hal für Chlor, Brom, Jod
steht,
umsetzt.

6. Insektizide, akarizide und herbizide Mittel, gekennzeichnet durch einen Gehalt an mindestens einem 3-Aryl-pyrrolidin-2,4-dion-Derivat der Formel (I).

7. Verfahren zur Bekämpfung von Insekten und/oder Spinnentieren und/oder Unkräutern, dadurch gekennzeichnet, daß man 3-Aryl-pyrrolidin-2,4-dion-Derivate der Formel (I) auf Insekten und/oder Spinnentiere und/oder Unkräuter und/oder deren Lebensraum einwirken läßt.

8. Verwendung von 3-Aryl-pyrrolidin-2,4-dion-Derivaten der Formel (I) zur Bekämpfung von Insekten und/oder Spinnentieren und/oder Unkräutern.

9. Verfahren zur Herstellung von insektiziden und/oder akariziden und/oder herbiziden Mitteln, dadurch gekennzeichnet, daß man 3-Aryl-pyrrolidin-2,4-dion-Derivate der Formel (I) mit Streckmitteln und/oder oberflächenaktiven Mitteln vermischt.

## Patentansprüche (Patentansprüche für folgende(n) Vertragsstaat(en) : ES)

1. Verfahren zur Herstellung von substituierten 3-Aryl-pyrrolidin-2,4-dion-Derivaten der allgemeinen Formel (I) 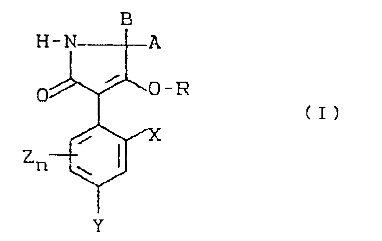 in welcher
X für C₁-C₆-Alkyl, Halogen, C₁-C₆-Alkoxy steht.
Y für Wasserstoff, C₁-C₆-Alkyl, Halogen, C₁-C₆-Alkoxy, C₁-C₃-Halogenalkyl steht,
Z für C₁-C₆-Alkyl, Halogen, C₁-C₆-Alkoxy steht,
n für eine Zahl von 0-3 steht,
A für Wasserstoff oder gegebenenfalls durch Halogen substituiertes geradkettiges oder verzweigtes Cᵢ-C₁₂-Alkyl, C₃-C₈-Alkenyl, C₃-C₈-Alkinyl, C₁-C₁₀-Alkoxy-C₂-C₈-alkyl, C₁-C₈-Polyalkoxy-C₂-C₈-alkyl, C₁-C₁₀-Alkylthio-C₂-C₈-alkyl, Cycloalkyl mit 3-8 Ringatomen, das durch Sauerstoff und/oder Schwefel unterbrochen sein kann,
B für Wasserstoff, geradkettiges oder verzweigtes C₁-C₁₂-Alkyl, C₁-C₈-Alkoxyalkyl steht,
oder worin
A und B gemeinsam mit dem Kohlenstoffatom, an das sie gebunden sind, einen 3- bis 8-gliedrigen gesättigten oder ungesättigten Ring bilden, der durch Sauerstoff und/oder Schwefel unterbrochen und durch gegebenenfalls halogeniertes Alkyl, Alkoxy, Phenyl und Halogen substituiert sein kann,
R für die Gruppen 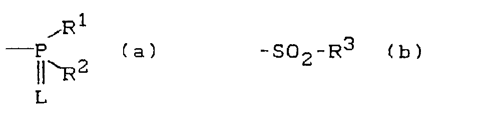 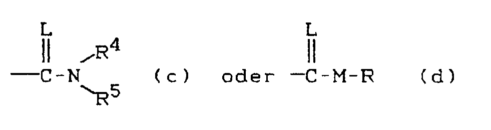 steht,
in welchen
L und M jeweils für Sauerstoff oder Schwefel steht und wobei L und M nicht gleichzeitig für Sauerstoff stehen,
R¹, R² und R³ unabhängig voneinander für gegebenenfalls durch Halogen substituiertes C₁-C₈-Alkyl, C₁-C₈-Alkoxy, C₁-C₈-Alkylamino, Di-(C₁-C₈)-Alkylamino, C₁-C₈-Alkylthio, C₂-C₅-Alkenylthio, C₂-C₅-Alki- nylthio, C₃-C₇-Cycloalkylthio, für gegebenenfalls durch Halogen, Nitro, Cyano, C₁-C₄-Alkoxy, C₁-C₄-Halogenalkoxy, C₁-C₄-Alkylthio, C₁-C₄-Halogenalkylthio, C₁-C₄-Alkyl, C₁-C₄-Halogenalkyl substituiertes Phenyl, Benzyl, Phenoxy oder Phenylthio stehen.
R⁴ und R⁵ unabhängig voneinander für gegebenenfalls durch Halogen substituiertes C₁-C₂₀-Alkyl, C₁-C₂₀-Alkoxy. C₂-C₈-Alkenyl, C₁-C₂₀-Alkoxy-C₁-C₂₀-alkyl, für gegebenenfalls durch Halogen, C₁-C₂₀-Halogenalkyl, C₁-C₂₀-Alkyl oder C₁-C₂₀-Alkoxy substituiertes Phenyl, für gegebenenfalls durch Halogen, C₁-C₂₀-Alkyl, C₁-C₂₀-Halogenalkyl oder C₁-C₂₀-Alkoxy substituiertes Benzyl steht oder zusammen für einen gegebenenfalls durch Sauerstoff unterbrochenen C₂-C₆-Alkylenring stehen,
R⁶ für gegebenenfalls durch Halogen substituiertes C₁-C₂₀-Alkyl, das durch Sauerstoff unterbrochen sein kann, für gegebenenfalls durch Halogen. C₁-C₂₀-Halogenalkyl, C₁-C₂₀-Alkoxy substituiertes Phenyl, für gegebenenfalls durch Halogen, C₁-C₂₀-Halogenalkyl oder C₁-C₂₀-Alkoxy substituiertes Benzyl, für C₂-C₈-Alkenyl oder für C₂-C₅-Alkinyl steht.
dadurch gekennzeichnet, daß man zum Erhalt von substituierten 3-Aryl-pyrrolidin-2,4-dionen der Formel (la) 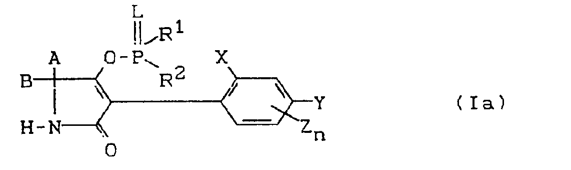 in welcher
A, B, L, X, Y, Z, R¹, R² und n die in Anspruch 1 angegebene Bedeutung haben,
A) 3-Aryl-pyrrolidin-2,4-dione der Formel (II) bzw. deren Enole 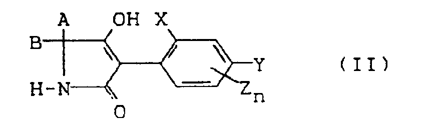 in welcher
A, B, X, Y, Z und n die oben angegebene Bedeutung haben
mit Phosphorverbindungen der allgemeinen Formel (III) 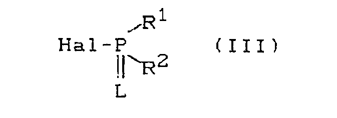 in welcher
L, R¹ und R² die oben angegebene Bedeutung haben und
Hal für Halogen, insbesondere Chlor und Brom steht,
gegebenenfalls in Gegenwart eines Verdünnungsmittels, gegebenenfalls in Gegenwart eines Säurebindemittels und gegebenenfalls in Gegenwart eines Phasentransferkatalysators umsetzt,
B) oder daß man zum Erhalt von Verbindungen der Formel (Ib) 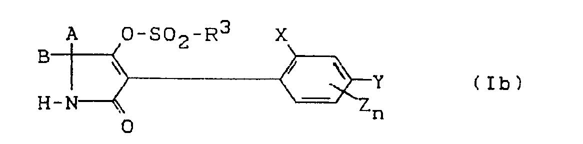 in welcher
A B, X, Y, Z, R³ und n die in Anspruch 1 angegebene Bedeutung haben,
Verbindungen der Formel (II) 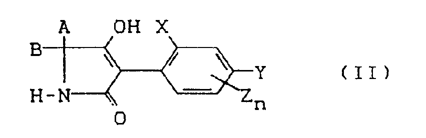 in welcher
A, B, X, Y, Z und n die oben angegebene Bedeutung haben
mit Sulfonsäurechloriden der allgemeinen Formel (IV) 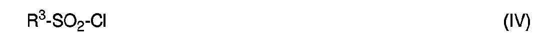 in welcher
R³ die oben angegebene Bedeutung hat
gegebenenfalls in Gegenwart eines Verdünnungsmittels und gegebenenfalls in Gegenwart eines Säurebindemittels.
C) oder daß man zum Erhalt von Verbindungen der Formel (Ic) 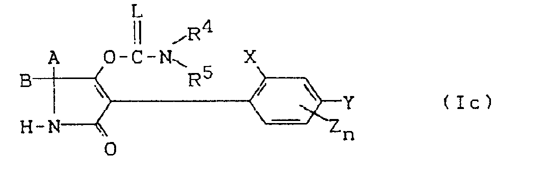 in welcher
A, B, L, X, Y, Z, R⁴, R⁵ und n die in Anspruch 1 angegebene Bedeutung haben,
Verbindungen der Formel (II), 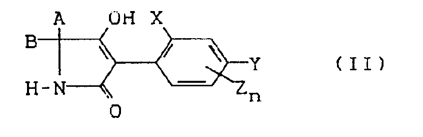 in welcher
A, B, X, Y, Z und n die oben angegebene Bedeutung haben
entweder
a) mit Isocyanaten der allgemeinen Formel (V) 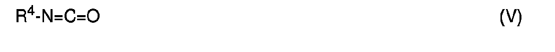 in welcher
R⁴ die oben angegebene Bedeutung hat
gegebenenfalls in Gegenwart eines Verdünnungsmittels und gegebenenfalls in Gegenwart eines Katalysators oder
β) mit Carbamidsäurechloriden oder Thiocarbamidsäurechloriden der allgemeinen Formel (VI) 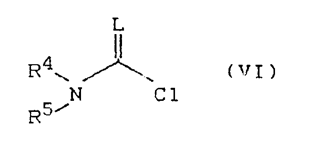 in welcher
L, R⁴ und R⁵ die oben angegebene Bedeutung haben
gegebenenfalls in Gegenwart eines Verdünnungsmittels und gegebenenfalls in Gegenwart eines Säurebindemittels.
umsetzt,
D) oder daß man zum Erhalt von Verbindungen der Formel (Id) 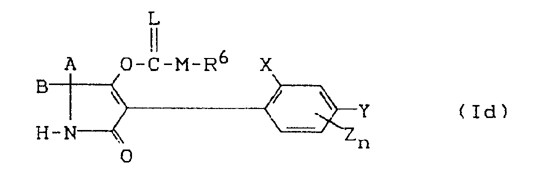 in welcher
A, B, L, M, R⁶, X, Y, Z und n die in Anspruch 1 angegebene Bedeutung haben,
Verbindungen der Formel (II) 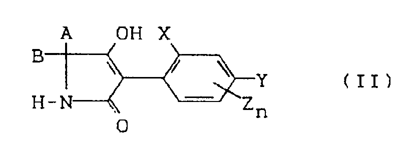 in welcher
A, B, X, Y, Z und n die oben angegebene Bedeutung haben,
entweder
a) mit Chlormonothioameisensäureestern, Chlorameisensäurethioestern oder Chlordithioameisensäureestern der allgemeinen Formel VII 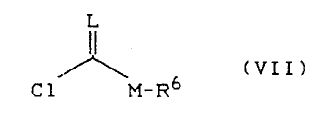 in welcher
L, M, R⁶ die oben angegebene Bedeutung haben
gegebenenfalls in Gegenwart eines Verdünnungsmittels und gegebenenfalls in Gegenwart eines Säurebindemittels umsetzt,
oder
β) mit Schwefelkohlenstoff und anschließend mit Alkylhalogeniden der allgemeinen Formel VIII 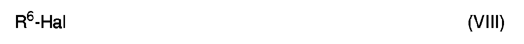 in welcher
R⁶ die oben angegebene Bedeutung hat und
Hal für Chlor, Brom, Jod
steht,
umsetzt.

2. Verfahren zur Herstellung von substituierten 3-Aryl-pyrrolidin-2,4-dion-Derivaten gemäß Anspruch 1 zum Erhalt von Verbindungen der Formeln (la) bis (Id) 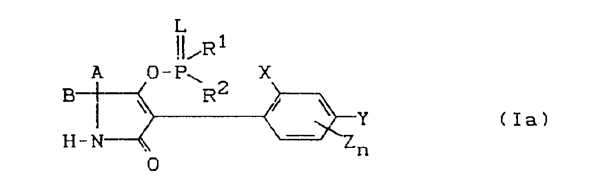 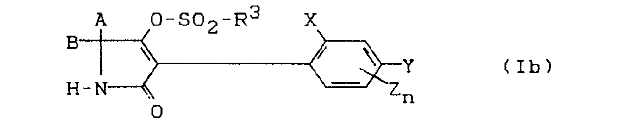 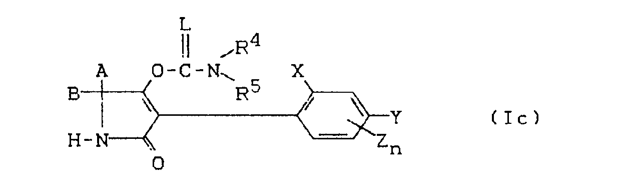 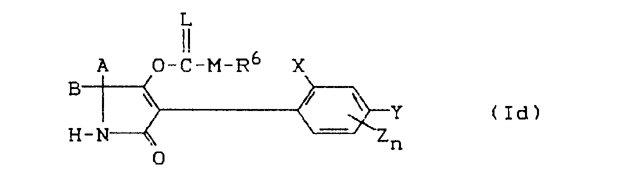 worin
_{A}, _{B}, _{L}, _{M}, _{X}, Y, Zₙ, R¹, R², R³, R⁴, R⁵ und R⁶ die im Anspruch 1 angegebene Bedeutung haben.

3. Verfahren zur Herstellung von substituierten 3-Aryl-pyrrolidin-2,4-dion-Derivaten gemäß Anspruch 1 zum Erhalt von Verbindungen (I) gemäß Anspruch 1, wobei
X für C₁-C₄-Alkyl, Halogen, C₁-C₄-Alkoxy steht.
Y für Wasserstoff, C₁-C₆-Alkyl, Halogen, C₁-C₄-Alkoxy, C₁-C₂-Halogenalkyl steht.
Z für C₁-C₄-Alkyl, Halogen, Ci-C4-Alkoxy steht.
n für eine Zahl von 0-3 steht.
A für Wasserstoff oder gegebenenfalls durch Halogen substituiertes geradkettiges oder verzweigtes C₁-C₁₀-Alkyl, C₃-C₆-Alkenyl, C₃-C₆-Alkinyl, C₁-C₈-Alkoxy-C₂-C₆-alkyl, C₁-C₆-Polyalkoxy-C₂-C₆-alkyl, C₁-C₈-Alkylthio-C₂-C₆-alkyl, Cycloalkyl mit 3-7 Ringatomen, das durch 1-2 Sauerstoff- und/oder Schwefelatomen unterbrochen sein kann, steht
B für Wasserstoff, geradkettiges oder verzweigtes C₁-C₁₀-Alkyl, C₁-C₆-Alkoxyalkyl steht,
oder worin
A und B gemeinsam mit dem Kohlenstoffatom, an das sie gebunden sind einen 3-bis 7-gliedrigen gesättigten oder ungesättigten Ring bilden, der durch C₁-C₆-Alkyl, C₁-C₆-Alkoxy, C₁-C₃-Haloalkyl, C₁-C₃-Haloalk- oxy, Fluor, Chlor substituiert und der durch Sauerstoff und/oder Schwefel unterbrochen sein kann.
R für die Gruppen 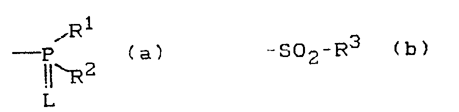 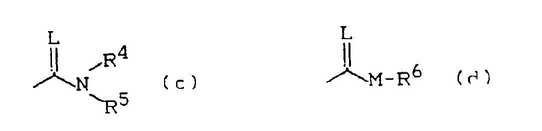 in welchen
L und M jeweils für Sauerstoff oder Schwefel stehen und worin L und M nicht gleichzeitig für Sauerstoff stehen
R¹, R² und R³ unabhängig voneinander für gegebenenfalls durch Halogen substituiertes C₁-C₆-Alkyl, C₁-C₆-Alkoxy, C₁-C₆-Alkylamino, Di-(C₁-C₆)-Alkylamino, C₁-C₆-Alkylthio, C₃-C₄-Alkenylthio, C₂-C₄-Alki- nylthio, C₃-C₆-Cycloalkylthio, für gegebenenfalls durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl, C₁-C₃-Halogenalkyl substituiertes Phenyl, Benzyl, Phenoxy oder Phenylthio stehen,
R⁴ und R⁵ unabhängig voneinander für gegebenenfalls durch Halogen substituiertes C₁-C₂₀-Alkyl, C₁-C₂₀-Alkoxy, C₂-C₈-Alkenyl, C₁-C₂₀-Alkoxy-C₁-C₂₀-alkyl, für gegebenenfalls durch Halogen, C₁-C₅-Halogenalkyl, C₁-C₅-Alkyl oder C₁-C₅-Alkoxy substituiertes Phenyl, für gegebenenfalls durch Halogen, C₁-C₅-Alkyl, C₁-C₅-Halogenalkyl oder C₁-C₅-Alkoxy substituiertes Benzyl steht,
R⁶ für gegebenenfalls durch Halogen substituiertes C₁-C₂₀-Alkyl, das durch Sauerstoff unterbrochen sein kann. für gegebenenfalls durch Halogen, C₁-C₅-Halogenalkyl, C₁-C₅-Alkoxy substituiertes Phenyl, für gegebenenfalls durch Halogen, C₁-C₅-Halogenalkyl oder C₁-C₅-Alkoxy substituiertes Benzyl steht,
sowie die enantiomerenreinen Formen von Verbindungen der Formel (I).

4. Insektizide, akarizide und herbizide Mittel, gekennzeichnet, durch einen Gehalt an mindestens einem 3-Aryl-pyrrolidin-2,4-dion-Derivat der Formel (I).

5. Verfahren zur Bekämpfung von Insekten und/oder Spinnentieren und/oder Unkräutern, dadurch gekennzeichnet, daß man 3-Aryl-pyrrolidin-2,4-dion-Derivate der Formel (I) auf Insekten und/oder Spinnentiere und/oder Unkräuter und/oder deren Lebensraum einwirken läßt.

6. Verwendung von 3-Aryl-pyrrolidin-2,4-dion-Derivaten der Formel (I) zur Bekämpfung von Insekten und/oder Spinnentieren und/oder Unkräutern.

7. Verfahren zur Herstellung von insektiziden und/oder akariziden und/oder herbiziden Mitteln, dadurch gekennzeichnet, daß man 3-Aryl-pyrrolidin-2,4-dion-Derivate der Formel (I) mit Streckmitteln und/oder oberflächenaktiven Mitteln vermischt.

## Claims (Claims for the following Contracting State(s) : BE, CH, DE, FR, GB, GR, IT, LI, NL)

1. Substituted 3-aryl-pyrrolidine-2,4-dione derivatives of the general formula (I) 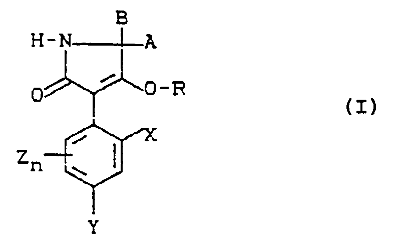 in which
X represents C₁-C₆-alkyl, halogen or C₁-C₆-alkoxy,
Y represents hydrogen, C₁-C₆-alkyl, halogen, C₁-C₆-alkoxy or C₁-C₃-halogenoalkyl,
Z represents C₁-C₆-alkyl, halogen or C₁-C₆-alkoxy,
n represents a number from 0-3,
A represents hydrogen or in each case straight-chain or branched C₁-C₁₂-alkyl, C₃-C₈-alkenyl, C₃-C₈-alkinyl, C₁-C₁₀-alkoxy-C₂-C₈-alkyl, C₁-C₈-polyalkoxy-C₂-C₈-alkyl and C₁-C₁₀-alkylthio-C₂-C₈-alkyl, each of which is optionally substituted by halogen, or represents cycloalkyl which has 3-8 ring atoms and which can be interrupted by oxygen and/or sulphur,
B represents hydrogen or in each case straight-chain or branched C₁-C₁₂-alkyl or C₁-C₈-alkoxy-alkyl,
or in which
A and B together with the carbon atom to which they are bonded form a 3- to 8-membered saturated or unsaturated ring which can be interrupted by oxygen and/or sulphur and which can be substituted by in each case optionally halogenated alkyl, alkoxy or phenyl and halogen,
R represents the groups 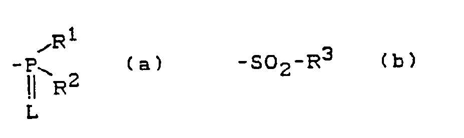 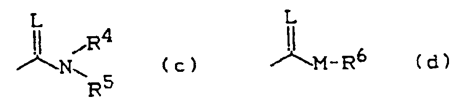 in which
Land M in each case represent oxygen or sulphur and where L and M do not simultaneously represent oxygen,
R¹, R² and R³ independently of each other represent C₁-C₈-alkyl, C₁-C₈-alkoxy, C₁-C₈-alkylamino, di-(C₁-C₈)-alkylamino, C₁-C₈-alkylthio, C₂-C₅-alkenylthio, C₂-C₅-alkinylthio and C₃-C₇-cycloalkylthio, each of which is optionally substituted by halogen, or represent phenyl, benzyl, phenoxy or phenylthio, each of which is optionally substituted by halogen, nitro, cyano, C₁-C₄-alkoxy, C₁-C₄-halog- enoalkoxy, C₁-C₄-alkylthio, C₁-C₄-halogenoalkylthio, C₁-C₄-alkyl or C₁-C₄-halogenoalkyl,
R⁴ and R⁵ independently of each other represents C₁-C₂₀-alkyl, C₁-C₂₀-alkoxy, C₂-C₈-alkenyl or C₁-C₂₀- alkoxy-C₁-C₂₀-alkyl, each of which is optionally substituted by halogen, or represents phenyl which is optionally substituted by halogen, C₁-C₂₀-halogenoalkyl, C₁-C₂₀-alkyl or C₁-C₂₀-alkoxy, or represents benzyl which is optionally substituted by halogen, C₁-C₂₀-alkyl, C₁-C₂₀-halogenoalkyl or C₁-C₂₀-alkoxy, or together represent a C₂-C₆-alkylene ring which is optionally interrupted by oxygen,
R⁶ represents C₁-C₂₀-alkyl which is optionally substituted by halogen and which can be interrupted by oxygen or represents phenyl which is optionally substituted by halogen, C₁-C₂₀-halogenoalkyl or C₁-C₂₀-alkoxy, or represents benzyl which is optionally substituted by halogen, C₁-C₂₀-halogenoalkyl or C₁-C₂₀-alkoxy, or represents C₂-C₈-alkenyl or C₂-C₅-alkinyl,
and the pure enantiomeric forms of compounds of the formula (I).

2. Substituted 3-aryl-pyrrolidine-2,4-dione derivatives of the formulae (la) to (Id) 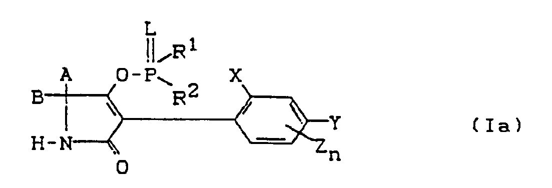 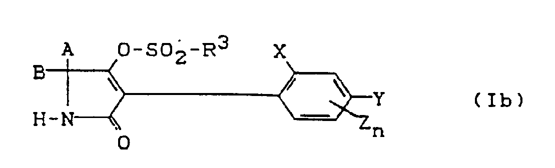 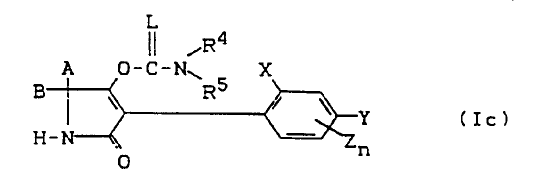 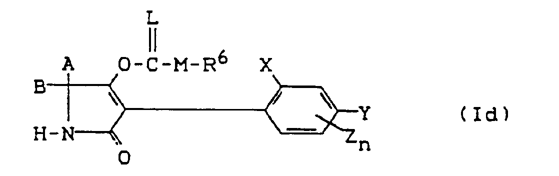 in which
A, B, L, M, X, Y, Zₙ, R¹, R², R³, R⁴, R⁵ and R⁶ have the meaning given in Claim 1.

3. Substituted 3-aryl-pyrrolidine-2,4-dione derivatives of the general formula (I) according to Claim 1, in which
X represents C₁-C₄-alkyl, halogen or Cᵢ-C₄-alkoxy,
Y represents hydrogen, C₁-C₆-alkyl, halogen, C₁-C₄-alkoxy or C₁-C₂-halogenoalkyl,
Z represents C₁-C₄-alkyl, halogen or C₁-C₄-alkoxy,
n represents a number from 0-3,
A represents hydrogen or in each case straight-chain or branched C₁-C₁₀-alkyl, C₃-C₆-alkenyl, C₃-C₆-alkinyl, C₁-C₈-alkoxy-C₂-C₆-alkyl, C₁-C₆-polyalkoxy-C₂-C₆-alkyl and C₁-C₈-alkylthio-C₂-C₆-alkyl, each of which is optionally substituted by halogen, or represents cycloalkyl having 3-7 ring atoms which can be interrupted by 1-2 oxygen and/or sulphur atoms,
B represents hydrogen or in each case straight-chain or branched C₁-C₁₀-alkyl or C₁-C₆-alkoxy-alkyl,
or in which
A and B together with the carbon atom to which they are bonded form a 3- to 7-membered saturated or unsaturated ring which can be substituted by C₁-C₆-alkyl, C₁-C₆-alkoxy, C₁-C₃-haloalkyl, C₁-C₃-haloalkoxy, fluorine or chlorine, and which can be interrupted by oxygen and/or sulphur,
R represents the groups 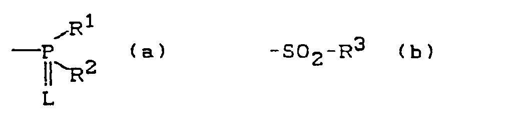 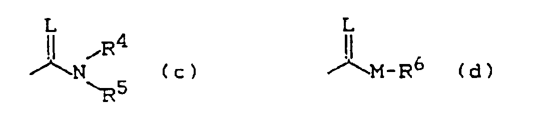 in which
Land M in each case represent oxygen or sulphur and where L and M do not simultaneously represent oxygen,
R¹, R² and R³ independently of one another represent C₁-C₆-alkyl, C₁-C₆-alkoxy, C₁-C₆-alkylamino, di-(C₁-C₆)-alkylamino, C₁-C₆-alkylthio, C₃-C₄-alkenylthio, C₂-C₄-alkinylthio and C₃-C₆-cycloalkylthio, each of which is optionally substituted by halogen, or represent phenyl, benzyl, phenoxy or phenylthio, each of which is optionally substituted by fluorine, chlorine, bromine, nitro, cyano, C₁-C₃-alkoxy, C₁-C₃-halogenoalkoxy, C₁-C₃-alkylthio, C₁-C₃-halogenoalkylthio, C₁-C₃-alkyl or C₁-C₃-halogenoalkyl,
R⁴ and R⁵ independently of one another represents Cᵢ-C₂ₒ-alkyl, C₁-C₂₀-alkoxy, C₂-C₈-alkenyl or C₁-C₂₀- alkoxy-C₁-C₂₀-alkyl, each of which is optionally substituted by halogen, or represents phenyl which is optionally substituted by halogen, C₁-C₅-halogenoalkyl, C₁-C₅-alkyl or C₁-C₅-alkoxy, or represents benzyl which is optionally substituted by halogen, C₁-C₅-alkyl, C₁-C₅-halogenoalkyl or C₁-C₅-alkoxy,
R⁶ represents C₁-C₂₀-alkyl which is optionally substituted by halogen and which can be interrupted by oxygen or represents phenyl which is optionally substituted by halogen, C₁-C₅-halogenoalkyl or C₁-C₅-alkoxy, or represents benzyl which is optionally substituted by halogen, C₁-C₅-halogenoalkyl or C₁-C₅-alkoxy,
and the pure enantiomeric forms of compounds of the formula (I).

4. Substituted 3-aryl-pyrrolidine-2,4-dione derivatives of the general formula (I) according to Claim 1, in which
X represents methyl, ethyl, propyl, i-propyl, fluorine, chlorine, bromine, methoxy and ethoxy,
Y represents hydrogen, methyl, ethyl, propyl, i-propyl, butyl, i-butyl, tert.-butyl, fluorine, chlorine, bromine, methoxy, ethoxy and trifluoromethyl,
Z represents methyl, ethyl, i-propyl, butyl, i-butyl, tert.-butyl, fluorine, chlorine, bromine, methoxy and ethoxy
n represents a number from 0-3,
A represents hydrogen, or represents in each case straight-chain or branched C₁-C₈-alkyl, C₃-C₄-alkenyl, C₃-C₄-alkinyl, Cᵢ-C₆-alkoxy-C₂-C₄-alkyl, C₁-C₄-polyalkoxy-C₂-C₄-alkyl and C₁-C₆-alkylthio-C₂-C₄-alkyl, each of which is optionally substituted by halogen, or represents cycloalkyl which has 3-6 ring atoms and which can be interrupted by 1-2 oxygen and/or sulphur atoms,
B represents hydrogen or in each case straight-chain or branched C₁-C₈-alkyl or C₁-C₄-alkoxy-alkyl,
or in which
A and B together with the carbon atom to which they are bonded form a 3- to 6-membered saturated or unsaturated ring which can be substituted by Ci-C4-alkyl, C₁-C₄-alkoxy, trifluoromethyl, trifluoromethoxy, fluorine or chlorine and which can be interrupted by oxygen and/or sulphur,
R represents the groups 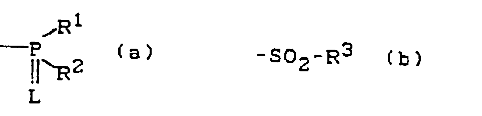 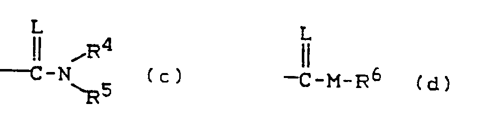 in which
Land M in each case represent oxygen or sulphur and where L and M do not simultaneously represent oxygen,
R¹, R² and R³ independently of each other represent C₁-C₄-alkyl, C₁-C₄-alkoxy, C₁-C₄-alkylamino, di-(C₁-C₄- alkyl)amino or C₁-C₄-alkylthio, each of which is optionally substituted by fluorine or chlorine, or represent phenyl, benzyl, phenoxy or phenylthio, each of which is optionally substituted by fluorine, chlorine, bromine, nitro, cyano, C₁-C₂-alkoxy, C₁-C₄-fluoroalkoxy, C₁-C₂-chloroalkoxy, C₁-C₂-alkylthio, C₁-C₂-fluoroalkylthio, C₁-C₂-chloroalkylthio or C₁-C₃-alkyl,
R⁴ and R⁵ independently of each other represents C₁-C₁₀-alkyl, C₁-C₁₀-alkoxy or C₁-C₁₀-alkoxy- (C₁-Cᵢₒ)alkyl, each of which is optionally substituted by fluorine, chlorine or bromine, or represents phenyl which is optionally substituted by fluorine, chlorine, bromine, C₁-C₂₀-halogenoalkyl, Cᵢ-C₂₀-alkyl or C₁-C₄-alkoxy, or represents benzyl which is optionally substituted by fluorine, chlorine, bromine, C₁-C₄-alkyl, C₁-C₄-halogenoalkyl or C₁-C₄-alkoxy,
R⁶ represents C₁-C₁₀-alkyl which is optionally substituted by fluorine, chlorine or bromine and which can be interrupted by oxygen or represents phenyl which is optionally substituted by fluorine, chlorine, bromine, C₁-C₄-halogenoalkyl or C₁-C₄-alkoxy, or represents benzyl which is optionally substituted by fluorine, chlorine, bromine, Ci-C4-halogenoalkyl or C₁-C₄-alkoxy,
and the pure enantiomeric forms of compounds of the formula (I).

5. Process for the preparation of substituted 3-aryl-pyrrolidine-2,4-dione derivatives of the general formula (I) according to Claim 1,
characterised in that, to obtain substituted 3-aryl-pyrrolidine-2,4-diones of the formula (la) 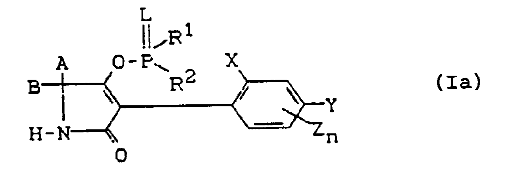 in which
A, B, L, X, Y, Z, R¹, R² and n have the meaning given in Claim 1,
A) 3-aryl-pyrrolidine-2,4-diones of the formula (II) or the enols thereof 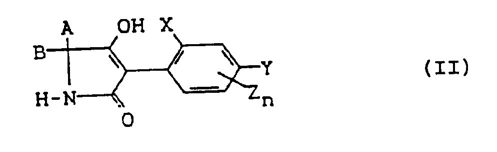 in which
A, B, X, Y, Z and n have the abovementioned meaning
are reacted with phosphorus compounds of the general formula (III) 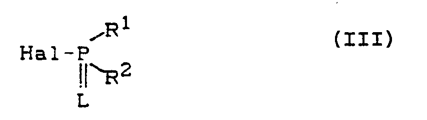 in which
L, R¹ and R² have the abovementioned meaning and
Hal represents halogen, in particular chlorine and bromine,
if appropriate in the presence of a diluent, if appropriate in the presence of an acid-binding agent and if appropriate in the presence of a phase-transfer catalyst,
B) or in that, to obtain compounds of the formula (Ib) 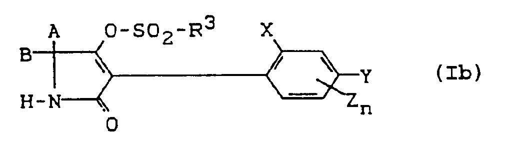 in which
A, B, X, Y, Z, R³ and n have the meaning given in Claim 1,
compounds of the formula (II) 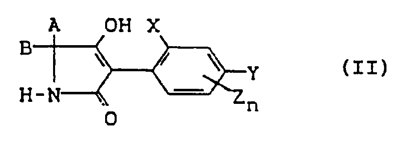 in which
A, B, X, Y, Z and n have the abovementioned meaning
are reacted with sulphonyl chlorides of the general formula (IV) 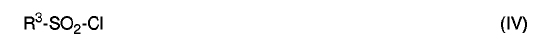 in which
R³ has the abovementioned meaning,
if appropriate in the presence of a diluent and if appropriate in the presence of an acid-binding agent,
C) or in that, to obtain compounds of the formula (Ic) 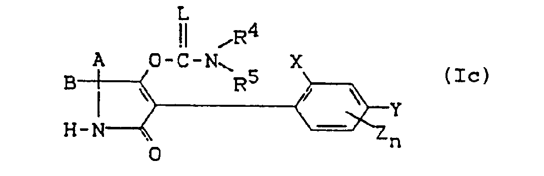 in which
A, B, L, X, Y, Z, R⁴, R⁵ and n have the meaning given in Claim 1,
compounds of the formula (II) 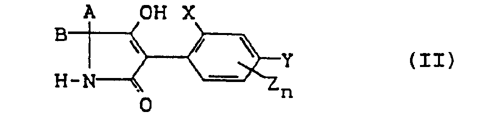 in which
A, B, X, Y, Z and n have the abovementioned meaning
are either reacted
a) with isocyanates of the general formula (V) 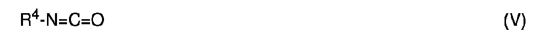 in which
R⁴ has the abovementioned meaning,
if appropriate in the presence of a diluent and if appropriate in the presence of a catalyst, or
β) with carbamic acid chlorides or thiocarbamic acid chlorides of the general formula (VI) 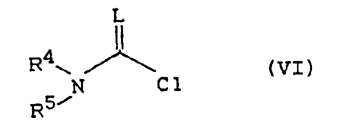 in which
L, R⁴ and R⁵ have the abovementioned meaning,
if appropriate in the presence of a diluent and if appropriate in the presence of an acid-binding agent,
D) or in that, to obtain compounds of the formula (Id) 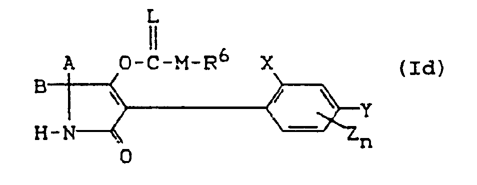 in which
A, B, L, M, R⁶, X, Y, Z and n have the meaning given in Claim 1,
compounds of the formula (II) 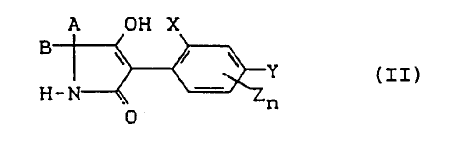 in which
A, B, X, Y, Z and n have the abovementioned meaning
are reacted either
a) with chloromonothioformic esters, chloroformic thioesters or chlorodithioformic esters of the general formula VII 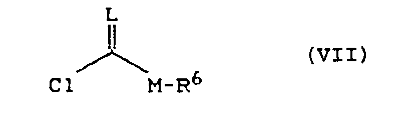 in which
L, M and R⁶ have the abovementioned meaning,
if appropriate in the presence of a diluent and if appropriate in the presence of an acid-binding agent, or
β) with carbon disuiphide and subsequently with alkyl halides of the general formula VIII 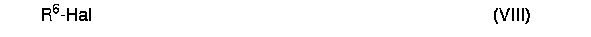 in which
R⁶ has the abovementioned meaning and
Hal represents chlorine, bromine or iodine.

6. Insecticidal, acaricidal and herbicidal agents, characterised in that they contain at least one 3-aryl-pyrrolidine-2,4-dione derivative of the formula (I).

7. Method of combating insects and/or arachnids and/or weeds, characterised in that 3-aryl-pyrrolidine-2,4-dione derivatives of the formula (I) are allowed to act on insects and/or arachnids and/or weeds and/or their environment.

8. Use of 3-aryl-pyrrolidine-2,4-dione derivatives of the formula (I) for combating insects and/or arachnids and/or weeds.

9. Process for the preparation of insecticidal and/or acaricidal and/or herbicidal agents, characterised in that 3-aryl- pyrrolidine-2,4-dione derivatives of the formula (I) are mixed with extenders and/or surface-active agents.

## Claims (Claims for the following Contracting State(s) : ES)

1. Process for the preparation of substituted 3-aryl-pyrrolidine-2,4-dione derivatives of the general formula (I) 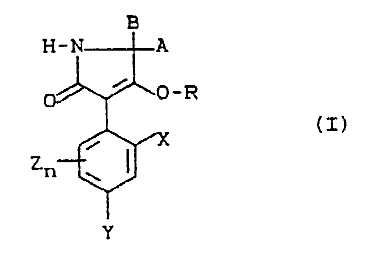 in which
X represents Ci-C6-alkyl, halogen or C₁-C₆-alkoxy,
Y represents hydrogen, Ci-C6-alkyl, halogen, Cᵢ-C₆-alkoxy or C₁-C₃-halogenoalkyl,
Z represents C₁-C₆-alkyl, halogen or C₁-C₆-alkoxy,
n represents a number from 0-3,
A represents hydrogen or in each case straight-chain or branched C₁-C₁₂-alkyl, C₃-C₈-alkenyl, C₃-C₈-alkinyl, C₁-C₁₀-alkoxy-C₂-C₈-alkyl, C₁-Cₛ-polyalkoxy-C₂-Cₛ-alkyl and C₁-C₁₀-alkylthio-C₂-C₈-alkyl, each of which is optionally substituted by halogen, or represents cycloalkyl which has 3-8 ring atoms and which can be interrupted by oxygen and/or sulphur,
B represents hydrogen or in each case straight-chain or branched C₁-C₁₂-alkyl or C₁-C₈-alkoxy-alkyl,
or in which
A and B together with the carbon atom to which they are bonded form a 3- to 8-membered saturated or unsaturated ring which can be interrupted by oxygen and/or sulphur and which can be substituted by in each case optionally halogenated alkyl, alkoxy or phenyl and halogen,
R represents the groups 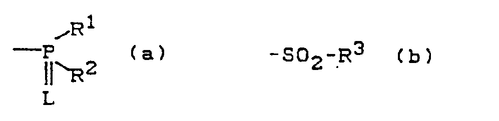 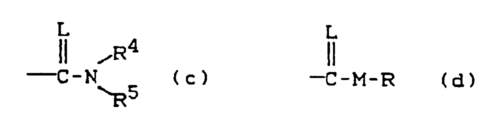 in which
Land M in each case represent oxygen or sulphur and where L and M do not simultaneously represent oxygen,
R¹, R² and R³ independently of each other represent C₁-C₈-alkyl, C₁-C₈-alkoxy, C₁-C₈-alkylamino, di-(C₁-C₈)-alkylamino, C₁-C₈-alkylthio, C₂-C₅-alkenylthio, C₂-C₅-alkinylthio and C₃-C₇-cycloalkylthio, each of which is optionally substituted by halogen, or represent phenyl, benzyl, phenoxy or phenylthio, each of which is optionally substituted by halogen, nitro, cyano, C₁-C₄-alkoxy, C₁-C₄-halog- enoalkoxy, C₁-C₄-alkylthio, C₁-C₄-halogenoalkylthio, C₁-C₄-alkyl or C₁-C₄-halogenoalkyl,
R⁴ and R⁵ independently of each other represents C₁-C₂₀-alkyl, C₁-C₂₀-alkoxy, C₂-C₈-alkenyl or C₁-C₂₀- alkoxy-C₁-C₂₀-alkyl, each of which is optionally substituted by halogen, or represents phenyl which is optionally substituted by halogen, C₁-C₂₀-halogenoalkyl, C₁-C₂₀-alkyl or C₁-C₂₀-alkoxy, or represents benzyl which is optionally substituted by halogen, C₁-C₂ₒ-alkyl, C₁-C₂₀-halogenoalkyl or C₁-C₂₀-alkoxy, or together represent a C₂-C₆-alkylene ring which is optionally interrupted by oxygen,
R⁶ represents C₁-C₂₀-alkyl which is optionally substituted by halogen and which can be interrupted by oxygen or represents phenyl which is optionally substituted by halogen, C₁-C₂₀-halogenoalkyl or C₁-C₂₀-alkoxy, or represents benzyl which is optionally substituted by halogen, C₁-C₂₀-halogenoalkyl or C₁-C₂₀-alkoxy, or represents C₂-C₈-alkenyl or C₂-C₅-alkinyl,
characterised in that, to obtain substituted 3-aryl-pyrrolidine-2,4-diones of the formula (Ia) 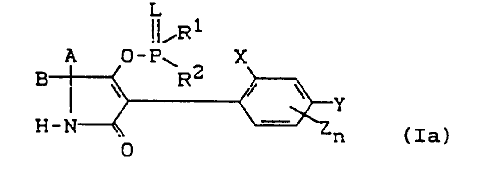 in which
A, B, L, X, Y, Z, R¹, R² and n have the meaning given in Claim 1,
A) 3-aryl-pyrrolidine-2,4-diones of the formula (II) or the enols thereof 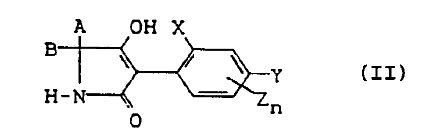 in which
A, B, X, Y, Z and n have the abovementioned meaning
are reacted with phosphorus compounds of the general formula (III) 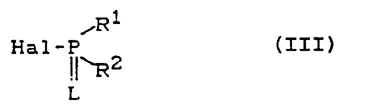 in which
L, R¹ and R² have the abovementioned meaning and
Hal represents halogen, in particular chlorine and bromine,
if appropriate in the presence of a diluent, if appropriate in the presence of an acid-binding agent and if appropriate in the presence of a phase-transfer catalyst,
B) or in that, to obtain compounds of the formula (Ib) 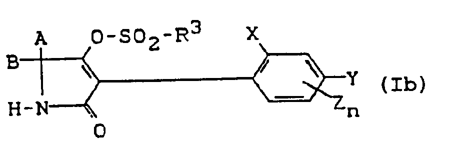 in which
A, B, X, Y, Z, R³ and n have the meaning given in Claim 1,
compounds of the formula (II) 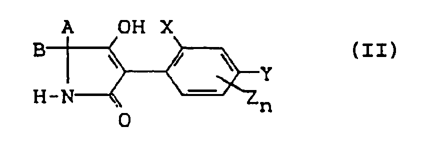 in which
A, B, X, Y, Z and n have the abovementioned meaning
are reacted with sulphonyl chlorides of the general formula (IV) 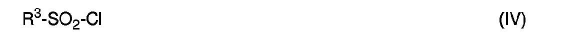 in which
R³ has the abovementioned meaning,
if appropriate in the presence of a diluent and if appropriate in the presence of an acid-binding agent,
C)' or in that, to obtain compounds of the formula (Ic) 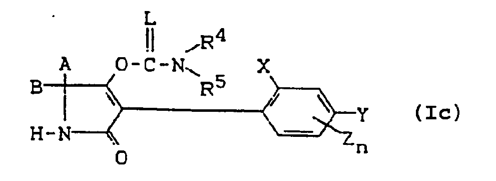 in which
A, B, L, X, Y, Z, R⁴, R⁵ and n have the meaning given in Claim 1,
compounds of the formula (II) 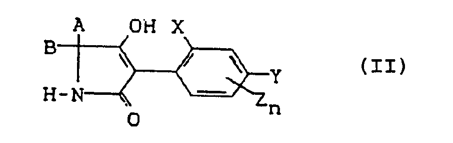 in which
A, B, X, Y, Z and n have the abovementioned meaning
are either reacted
a) with isocyanates of the general formula (V) 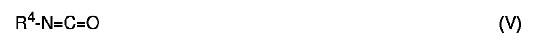 in which
R⁴ has the abovementioned meaning,
if appropriate in the presence of a diluent and if appropriate in the presence of a catalyst, or
β) with carbamic acid chlorides or thiocarbamic acid chlorides of the general formula (VI) 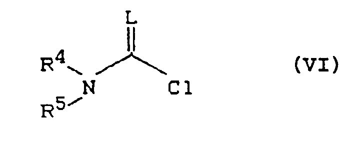 in which
L, R⁴ and R⁵ have the abovementioned meaning,
if appropriate in the presence of a diluent and if appropriate in the presence of an acid-binding agent,
D) or in that, to obtain compounds of the formula (id) 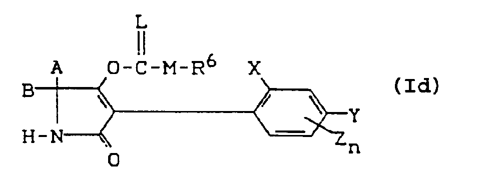 in which
A, B, L, M, R⁶, X, Y, Z and n have the meaning given in Claim 1,
compounds of the formula (II) 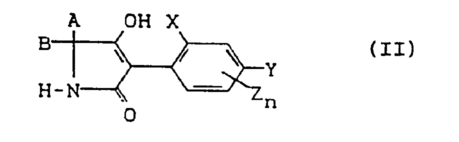 in which
A, B, X, Y, Z and n have the abovementioned meaning
are reacted either
a) with chloromonothioformic esters, chloroformic thicesters or chlorodithioformic esters of the general formula VII 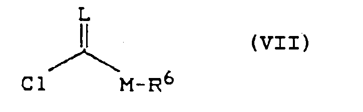 in which
L, M and R⁶ have the abovementioned meaning,
if appropriate in the presence of a diluent and if appropriate in the presence of an acid-binding agent, or
β) with carbon disulphide and subsequently with alkyl halides of the general formula VIII 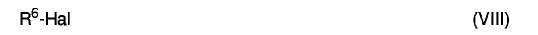 in which
R⁶ has the abovementioned meaning and
Hal represents chlorine, bromine or iodine.

2. Process for the preparation of substituted 3-aryl-pyrrolidine-2,4-dione derivatives according to Claim 1 to obtain compounds of the formulae (la) to (Id) 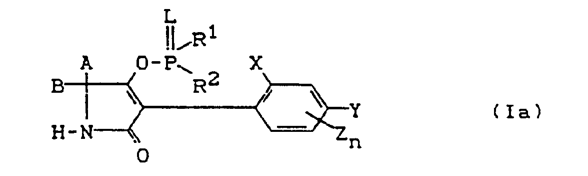 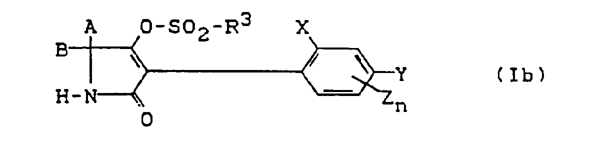 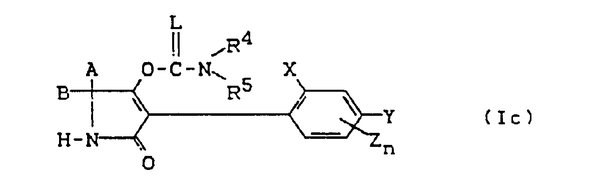 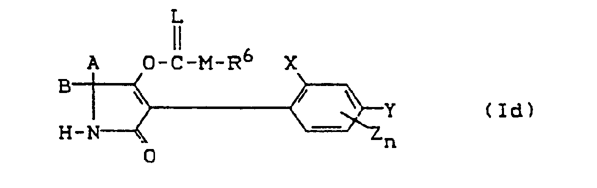 in which
A, B, L, M, X, Y, Zₙ, R¹, R², R³, R⁴, R⁵ and R⁶ have the meaning given in Claim 1.

3. Process for the preparation of substituted 3-aryl-pyrrolidine-2,4-dione derivatives according to Claim 1 to obtain compounds (I) according to Claim 1, where
X represents C₁-C₄-alkyl, halogen or Cᵢ-C₄-alkoxy,
Y represents hydrogen, C₁-C₆-alkyl, halogen, C₁-C₄-alkoxy or C₁-C₂-halogenoalkyl,
Z represents C₁-C₄-alkyl, halogen or C₁-C₄-alkoxy,
n represents a number from 0-3,
A represents hydrogen or in each case straight-chain or branched C₁-C₁₀-alkyl, C₃-C₆-alkenyl, C₃-C₆-alkinyl, C₁-C₈-alkoxy-C₂-C₆-alkyl, C₁-C₆-polyalkoxy-C₂-C₆-alkyl and C₁-C₈-alkylthio-C₂-C₆-alkyl, each of which is optionally substituted by halogen, or represents cycloalkyl having 3-7 ring atoms which can be interrupted by 1-2 oxygen and/or sulphur atoms,
B represents hydrogen or in each case straight-chain or branched C₁-C₁₀-alkyl or C₁-C₆-alkoxy-alkyl,
or in which
A and B together with the carbon atom to which they are bonded form a 3- to 7-membered saturated or unsaturated ring which can be substituted by C₁-C₆-alkyl, C₁-C₆-alkoxy, C₁-C₃-haloalkyl, C₁-C₃-haloalkoxy, fluorine or chlorine, and which can be interrupted by oxygen and/or sulphur,
R represents the groups 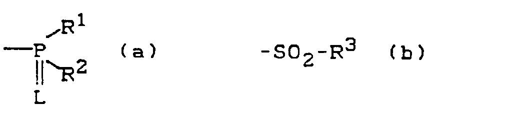 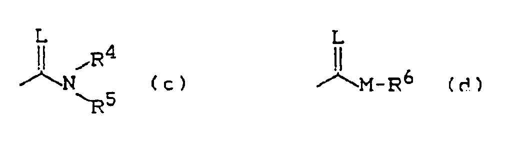 in which
Land M in each case represent oxygen or sulphur and where L and M do not simultaneously represent oxygen,
R¹, R² and R³ independently of one another represent C₁-C₆-alkyl, C₁-C₆-alkoxy, C₁-C₆-alkylamino, di-(C₁-C₆)-alkylamino, C₁-C₆-alkylthio, C₃-C₄-alkenylthio, C₂-C₄-alkinylthio and C₃-C₆-cycloalkylthio, each of which is optionally substituted by halogen, or represent phenyl, benzyl, phenoxy or phenylthio, each of which is optionally substituted by fluorine, chlorine, bromine, nitro, cyano, C₁-C₃-alkoxy, C₁-C₃-halogenoalkoxy, C₁-C₃-alkylthio, C₁-C₃-halogenoalkylthio, C₁-C₃-alkyl or C₁-C₃-halogenoalkyl,
R⁴ and R⁵ independently of one another represents C₁-C₂₀-alkyl, C₁-C₂₀-alkoxy, C₂-C₈-alkenyl or C₁-C₂₀- alkoxy-C₁-C₂₀-alkyl, each of which is optionally substituted by halogen, or represents phenyl which is optionally substituted by halogen, C₁-C₅-halogenoalkyl, C₁-C₅-alkyl or C₁-C₅-alkoxy, or represents benzyl which is optionally substituted by halogen, C₁-C₅-alkyl, C₁-C₅-halogenoalkyl or C₁-C₅-alkoxy,
R⁶ represents C₁-C₂₀-alkyl which is optionally substituted by halogen and which can be interrupted by oxygen or represents phenyl which is optionally substituted by halogen, C₁-C₅-halogenoalkyl or C₁-C₅-alkoxy, or represents benzyl which is optionally substituted by halogen, C₁-C₅-halogenoalkyl or C₁-C₅-alkoxy,
and the pure enantiomeric forms of compounds of the formula (I).

4. Insecticidal, acaricidal and herbicidal agents, characterised in that they contain at least one 3-aryl-pyrrolidine-2,4-dione derivative of the formula (I).

5. Method of combating insects and/or arachnids and/or weeds, characterised in that 3-aryl-pyrrolidine-2,4-dione derivatives of the formula (I) are allowed to act on insects and/or arachnids and/or weeds and/or their environment.

6. Use of 3-aryl-pyrrolidine-2,4-dione derivatives of the formula (I) for combating insects and/or arachnids and/or weeds.

7. Process for the preparation of insecticidal and/or acaricidal and/or herbicidal agents, characterised in that 3-aryl- pyrrolidine-2,4-dione derivatives of the formula (I) are mixed with extenders and/or surface-active agents.

## Revendications (Revendications pour l'(les) Etat(s) contractant(s) suivant(s) : BE, CH, DE, FR, GB, GR, IT, LI, NL)

1. Dérivés substitués de 3-arylpyrrolidine-2,4-diones de formule générale (I) 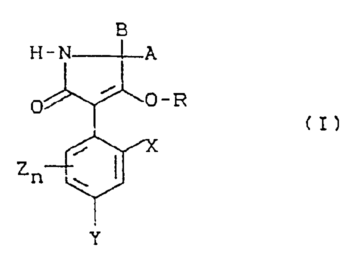 dans laquelle
X est un groupe alkyle en C₁ à C₆, un halogène, un groupe alkoxy en C₁ à C₆,
Y est de l'hydrogène, un groupe alkyle en C₁ à C₆, un halogène, un groupe alkoxy en C₁ à C₆, halogénalkyle en C₁ à C₃,
Z est un groupe alkyle en C₁ à C₆, un halogène, un groupe alkoxy en C₁ à C₆,
n est un nombre de 0 à 3,
A représente de l'hydrogène ou un groupe, éventuellement substitué par un halogène, alkyle en C₁ à C₁₂ linéaire ou ramifié, alcényle en C₃ à Cₛ, alcynyle en C₃ à C₈, (alkoxy en C₁ à C₁₀)-(alkyle en C₂ à C₈), (polyalkoxy en C₁ à C₈)-(alkyle en C₂ à C₈), (alkylthio en C1 à Cₗₒ)-(alkyle en C₂ à C₈), cycloalkyle à noyau de 3 à 8 atomes, qui peut être interrompu par de l'oxygène et/ou du soufre,
B est de l'hydrogène, un groupe alkyle en C₁ à C₁₂ linéaire ou ramifié, un groupe alkoxyalkyle en C₁ à C₈,
ou dans laquelle
A et B forment, conjointement avec l'atome de carbone auquel ils sont liés, un noyau saturé ou non saturé de 3 à 8 chaînons qui peut être interrompu par de l'oxygène et/ou du soufre et être substitué, le cas échéant, par un groupe alkyle éventuelleinent halogéné, alkoxy, phényle et un halogène,
R représente les groupes 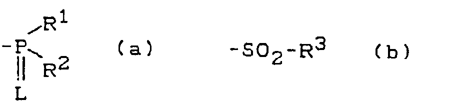 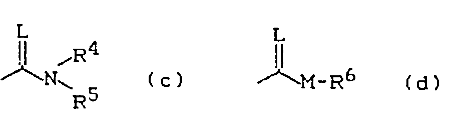 dans lesquels
L et M représentent chacun de l'oxygène ou du soufre, L et M ne pouvant pas représenter simultanément de l'oxygène,
R¹, R² et R³ représentent, indépendamment les uns des autres, un groupe, éventuellement substitué par un halogène, alkyle en C₁ à C₈, alkoxy en C₁ à C₈, alkylamino en C₁ à C₈, di-(alkyle en C₁ à C₈)-amino, alkylthio en C₁ à C₈, alcénylthio en C₂ à C₅, alcynylthio en C₂ à C₅, cycloalkylthio en C₃ à C₇, un groupe phényle, benzyle, phénoxy ou phénylthio éventuellement substitué par un radical halogéno, nitro, cyano, alkoxy en C₁ à C₄, halogénalkoxy en C₁ à C₄, alkylthio en C₁ à C₄, halogénalkylthio en C₁ à C₄, alkyle en C₁ à C₄, halogénalkyle en C₁ à C₄,
R⁴ et R⁵ représentent, indépendamment l'un de l'autre, un groupe, éventuellement substitué par un halogène, alkyle en C₁ à C₂₀, alkoxy en C₁ à C₂₀, alcényle en C₂ à C₈, (alkoxy en C₁ à C₂ₒ)-(alkyle en C₁ à C₂₀), un groupe phényle éventuellement substitué par un radical halogéno, halogénalkyle en C₁ à C₂₀, alkyle en C₁ à C₂₀ ou alkoxy en C₁ à C₂₀, un groupe benzyle éventuellement substitué par un radical halogéno, alkyle en C₁ à C₂₀, halogénalkyle en C₁ à C₂₀ ou alkoxy en C₁ à C₂₀ ou forment ensemble un noyau alkylène en C₂ à C₆ éventuellement interrompu par de l'oxygène,
R⁶ est un groupe alkyle en C₁ à C₂₀ éventuellement substitué par un halogène, qui peut être interrompu par de l'oxygène, un groupe phényle éventuellement substitué par un radical halogéno, halogénalkyle en C₁ à C₂₀, alkoxy en C₁ à C₂₀, un groupe benzyle éventuellement substitué par un radical halogéno, halogénalkyle en C₁ à C₂₀ ou alkoxy en C₁ à C₂₀, un groupe alcényle en C₂ à C₈ ou un groupe alcynyle en C₂ à C₅,
ainsi que les formes énantiomères pures de composés de formule (I).

2. Dérivés substitués de 3-arylpyrrolidine-2,4-diones de formules (la) à (Id) 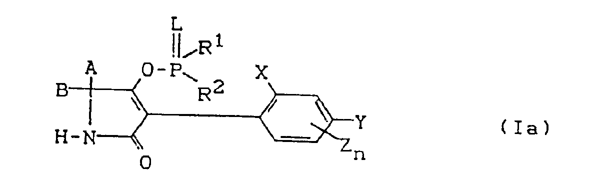 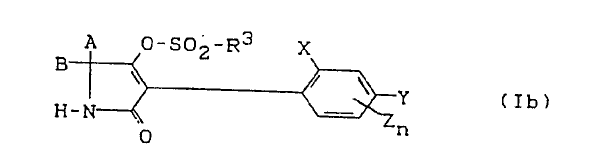 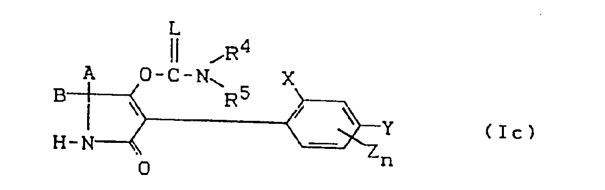 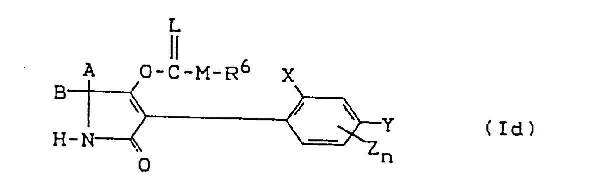 dans lesquelles
A, _{B}, _{L}, _{M}, _{X}, Y, Zₙ. R¹, R², R³, R⁴, R⁵ et R⁶ ont la définition indiquée dans la revendication 1.

3. Dérivés substitués de 3-arylpyrrolidine-2,4-diones de formule générale (I) suivant la revendication 1, dans laquelle
X est un groupe alkyle en C₁ à C₄ un halogène, un groupe alkox^{y} en C₁ à C₄,
Y est de l'hydrogène, un groupe alkyle en C₁ à C₆, un halogène, un groupe alkoxy en C₁ à C₄, halogénalkyle en C₁ ou C₂,
Z est un groupe alkyle en C₁ à C₄, un halogène, un groupe alkoxy en C₁ à C₄,
n est un nombre de 0 à 3,
A est de l'hydrogène ou un groupe, éventuellement substitué par un halogène, alkyle en C₁ à C₁₀ linéaire ou ramifié, alcényle en C₃ à C₆, alcynyle en C₃ à C₆, (alkoxy en C₁ à C₈)-(alkyle en C₂ à C₆), (polyalkoxy en C₁ à C₆)-(alkyle en C₂ à C₆), (alkylthio en C₁ à C₈)-(alkyle en C₂ à C₆), cycloalkyle à noyau de 3 à 7 atomes, qui peut être interrompu par 1 ou 2 atomes d'oxygène et/ou de soufre,
B est de l'hydrogène, un groupe alkyle en C₁ à C₁₀ linéaire ou ramifié, alkoxyalkyle en C₁ à C₆,
ou dans laquelle
A et B forment, conjointement avec l'atome de carbone auquel ils sont liés, un noyau saturé ou non saturé de 3 à 7 chaînons qui peut être substitué par un radical alkyle en C₁ à C₆, alkoxy en C₁ à C₆, halogénalkyle en C₁ à C₃, halogénalkoxy en C₁ à C₃, fluoro, chloro et qui peut être interrompu par de l'oxygène et/ou du soufre,
R représente les groupes 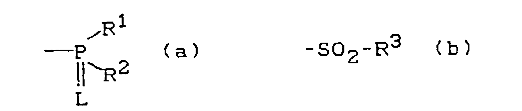 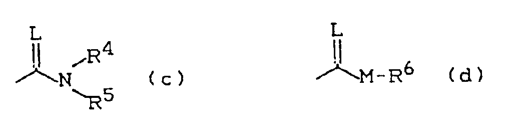 dans lesquels
L et M représentent chacun de l'oxygène ou du soufre, L et M ne représentant pas simultanément de l'oxygène,
R¹, R² et R³ représentent, indépendamment les uns des autres, un groupe, éventuellement substitué par un halogène, alkyle en C₁ à C₆, alkoxy en C₁ à C₆, alkylamino en C₁ à C₆, di-(alkyle en C₁ à C₆)-amino, alkylthio en C₁ à C₆, alcénylthio en C₃ ou C₄, alcynylthio en C₂ à C₄, cycloalkylthio en C₃ à C₆, un groupe phényle, benzyle, phénoxy ou phénylthio éventuellement substitué par un radical fluoro, chloro, bromo, nitro, cyano, alkoxy en C₁ à C₃, halogénalkoxy en C₁ à C₃, alkylthio en C₁ à C₃, halogénalkylthio en C₁ à C₃, alkyle en C₁ à C₃, halogénalkyle en C₁ à C₃,
R⁴ et R⁵ représentent, indépendamment l'un de l'autre, un groupe, éventuellement substitué par un halogène, alkyle en C₁ à C₂₀, alkoxy en C₁ à C₂₀, alcényle en C₂ à C₈, (alkoxy en C₁ à C₂ₒ)-(alkyle en C₁ à C₂₀), un groupe phényle éventuellement substitué par un radical halogéno, halogénalkyle en C₁ à C₅, alkyle en C₁ à C₅ ou alkoxy en C₁ à C₅, un groupe benzyle éventuellement substitué par un radical halogéno, alkyle en C₁ à C₅, halogénalkyle en C₁ à C₅ ou alkoxy en C₁ à C₅,
R⁶ est un groupe alkyle en C₁ à C₂₀ éventuellement substitué par un halogène, qui peut être interrompu par de l'oxygène, un groupe phényle éventuellement substitué par un radical halogéno, halogénalkyle en C₁ à C₅, alkoxy en C₁ à C₅, un groupe benzyle éventuellement substitué par un radical halogéno, halogénalkyle en C₁ à C₅ ou alkoxy en C₁ à C₅,
ainsi que les formes énantiomères pures de composés de formule (I).

4. Dérivés substitués de 3-arylpyrrolidine-2,4-diones de formule générale (I) suivant la revendication 1, dans laquelle
X représente un groupe méthyle, éthyle, propyle, isopropyle, du fluor, du chlore, du brome, un groupe méthoxy et un groupe éthoxy,
Y est de l'hydrogène, un groupe méthyle, éthyle, propyle, isopropyle, butyle, isobutyle, tertiobutyle, du fluor, du chlore, du brome, un groupe méthoxy, éthoxy et trifluorométhyle,
Z est un groupe méthyle, éthyle, isopropyle, butyle, isobutyle, tertio-butyle, du fluor, du chlore, du brome, un groupe méthoxy et un groupe éthoxy,
n est un nombre de 0 à 3,
A est de l'hydrogène, un groupe, éventuellement substitué par un halogène, alkyle en C₁ à C₈ linéaire ou ramifié, alcényle en C₃ ou C₄, alcynyle en C₃ ou C₄, (alkoxy en C₁ à C₆)-(alkyle en C₂ à C₄), (polyalkoxy en C₁ à C₄)-(alkyle en C₂ à C₄), (alkylthio en C₁ à C₆)-(alkyle en C₂ à C₄), cycloalkyle à noyau de 3 à 6 atomes, qui peut être interrompu par 1 ou 2 atomes d'oxygène et/ou de soufre,
B est de l'hydrogène, un groupe alkyle en C₁ à C₈ linéaire ou ramifié, alkoxyalkyle en C₁ à C₄,
ou dans laquelle
A et B forment conjointement avec l'atome de carbone auquel ils sont liés, un noyau saturé ou non saturé de 3 à 6 chaînons, qui peut être substitué par un radical alkyle en C₁ à C₄, alkoxy en C₁ à C₄, trifluorométhyle, trifluorométhoxy, fluoro, chloro et qui peut être interrompu par de l'oxygène et/ou du soufre,
R représente les groupes 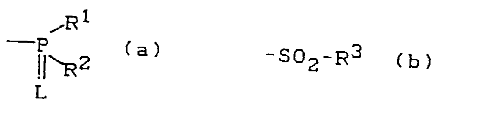 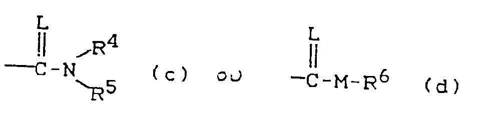 dans lesquels
L et M représentent chacun de l'oxygène ou du soufre, L et M ne représentant pas simultanément du soufre,
R¹, R² et R³ représentent, indépendamment les uns des autres, un groupe, éventuellement substitué par du fluor ou du chlore, alkyle en C₁ à C₄, alkoxy en C₁ à C₄, alkylamino en C₁ à C₄, di-(alkyle en C₁ à C₄)amino, alkylthio en C₁ à C₄, un groupe phényle, benzyle, phénoxy ou phénylthio éventuellement substitué par du fluor, du chlore, du brome, un radical nitro, cyano, alkoxy en C₁ ou C₂, fluo- ralkoxy en C₁ à C₄, chloralkoxy en C₁ ou C₂, alkylthio en C₁ ou C₂, fluoralkylthio en C₁ ou C₂, chloralkylthio en C₁ ou C₂, alkyle en C₁ à C₃,
R⁴ et R⁵ représentent, indépendamment l'un de l'autre, un groupe, éventuellement substitué par du fluor, du chlore, du brome, alkyle en C₁ à C₁₀, alkoxy en C₁ à C₁₀, (alkoxy en C₁ à C₁₀)-(alkyle en C₁ à C₁₀), un groupe phényle éventuellement substitué par un radical fluoro, chloro, bromo, halogénalkyle en C₁ à C₂₀, alkyle en C₁ à C₂₀ ou alkoxy en C₁ à C₄, un groupe benzyle éventuellement substitué par un radical fluoro, chloro, bromo, alkyle en C₁ à C₄, halogénalkyle en C₁ à C₄ ou alkoxy en Ci à C₄,
R⁶ représente un groupe alkyle en C₁ à C₁₀ éventuellement substitué par du fluor, du chlore ou du brome, qui peut être interrompu par de l'oxygène, un groupe phényle éventuellement substitué par du fluor, du chlore, du brome, un radical halogénalkyle en C₁ à C₄ ou alkoxy en C₁ à C₄, un groupe benzyle éventuellement substitué par du fluor, du chlore, du brome, un radical halogénalkyle en C₁ à C₄ ou alkoxy en C₁ à C₄,
ainsi que les formes énantiomères pures de composés de formule (I).

5. Procédé de production de dérivés substitués de 3-arylpyrrolidine-2,4-diones de formule générale (I) suivant la revendication 1, caractérisé en ce que, pour obtenir des 3-arylpyrrolidine-2,4-diones substituées de formule (la) 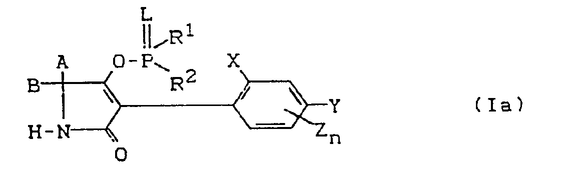 dans laquelle
A, B, L, X, Y, Z, R¹, R² et n ont la définition indiquée dans la revendication 1,
A) on fait réagir des 3-arylpyrrolidine-2,4-diones de formule (II) ou leurs énols 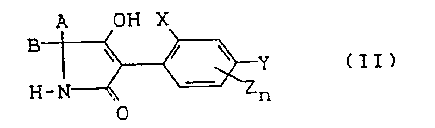 dans laquelle
A, B, X, Y, Z et n ont la définition indiquée ci-dessus,
avec des composés de phosphore de formule générale (III) 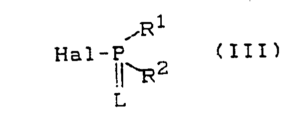 dans laquelle
L, R¹ et R² ont la définition indiquée ci-dessus et
Hal représente un halogène, en particulier le chlore et le brome,
le cas échéant en présence d'un diluant, éventuellement en présence d'un accepteur d'acide et, le cas échéant en présence d'un catalyseur de transfert de phase,
B) ou bien pour obtenir des composés de formule (Ib) 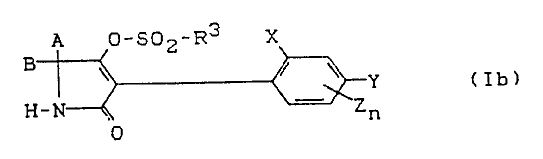 dans laquelle
A, B, X, Y, Z, R³ et n ont la définition indiquée dans la revendication 1,
on fait réagir des composés de formule (II) 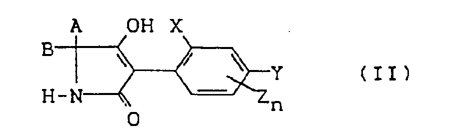 dans laquelle
A, B, X, Y, Z et n ont la définition indiquée ci-dessus
avec des chlorures d'acide sulfonique de formule générale (IV) 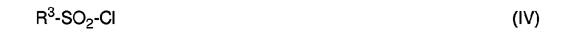 dans laquelle
R³ a la définition indiquée ci-dessus
le cas échéant en présence d'un diluant et en la présence éventuelle d'un accepteur d'acide,
C) ou bien pour obtenir des composés de formule (Ic) 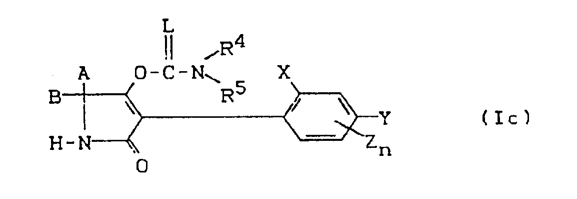 dans laquelle
A, B, L, X, Y, Z, R⁴, R⁵ et n ont la définition indiquée dans la revendication 1,
on fait réagir des composés de formule (II) 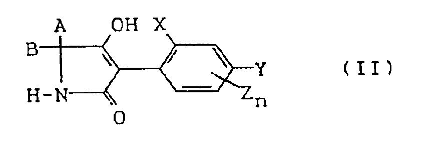 dans laquelle
A, B, X, Y, Z et n ont la définition indiquée ci-dessus
soit
a) avec des isocyanates de formule générale (V) 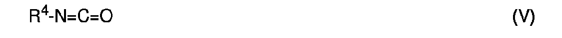 dans laquelle
R⁴ a la définition indiquée ci-dessus
le cas échéant en présence d'un diluant et en la présence éventuelle d'un catalyseur,
soit
β) avec des chlorures d'acide carbamique ou des chlorures d'acide thiocarbamique de formule générale (VI) 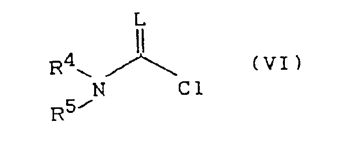 dans laquelle
L, R⁴ et R^{S} ont la définition indiquée ci-dessus, le cas échéant en présence d'un diluant et en la présence éventuelle d'un accepteur d'acide,
D) ou bien pour obtenir des composés de formule (Id) 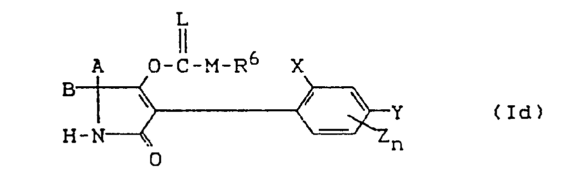 dans laquelle
A, B, L, M, R⁶, X, Y, Z et n ont la définition indiquée dans la revendication 1,
on fait réagir des composés de formule (II) 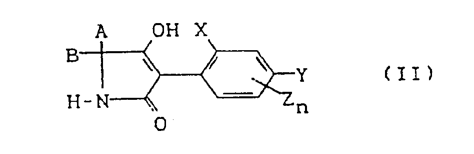 dans laquelle
A, B, X, Y, Z et n ont la définition indiquée ci-dessus, soit
a) avec des esters d'acide chloromonothioformique, des thioesters d'acide chloroformique ou des esters d'acide chlorodithioformique de formule générale (VII) 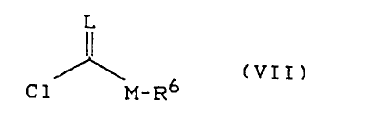 dans laquelle
L, M, R⁶ ont la définition indiquée ci-dessus,
le cas échéant en présence d'un diluant et en la présence éventuelle d'un accepteur d'acide,
soit
β) avec du sulfure de carbone, puis des halogénures d'alkyle de formule générale (VIII) 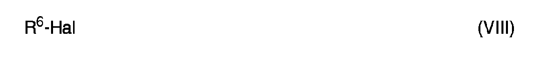 dans laquelle
R⁶ a la définition indiquée ci-dessus et
Hal représente du chlore, du brome, de l'iode.

6. Compositions insecticides, acaricides et herbicides, caractérisées par une teneur en au moins un dérivé de 3-aryl- pyrrolidine-2,4-dione de formule (1).

7. Procédé pour combattre des insectes et/ou des acariens et/ou des mauvaises herbes, caractérisé en ce qu'on fait agir des dérivés de 3-arylpyrrolidine-2,4-diones de formule (I) sur des insectes et/ou des acariens et/ou des mauvaises herbes et/ou leur milieu.

8. Utilisation de dérivés de 3-arylpyrrolidine-2,4-diones de formule (I) pour combattre des insectes et/ou des acariens et/ou des mauvaises herbes.

9. Procédé de préparation de compositions insecticides et/ou acaricides et/ou herbicides, caractérisé en ce qu'on mélange des dérivés de 3-arylpyrrolidine-2,4-diones de formule (I) avec des diluants et/ou des agents tensio-actifs.

## Revendications (Revendications pour l'(les) Etat(s) contractant(s) suivant(s) : ES)

1. Procédé de production de dérivés substitués de 3-arylpyrrolidine-2,4-diones de formule générale (I) 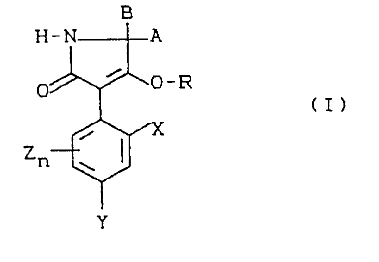 dans laquelle
X est un groupe alkyle en C₁ à C₆, un halogène, un groupe alkoxy en C₁ à C₆,
Y est de l'hydrogène, un groupe alkyle en C₁ à C₆, un halogène, un groupe alkoxy en C₁ à C₆, halogénalkyle en C₁ à C₃,
Z est un groupe alkyle en C₁ à C₆, un halogène, un groupe alkoxy en C₁ à C₆,
n est un nombre de 0 à 3,
A représente de l'hydrogène ou un groupe, éventuellement substitué par un halogène, alkyle en C₁ à C₁₂ linéaire ou ramifié, alcényle en C₃ à C₈, alcynyle en C₃ à C₈, (alkoxy en C₁ à Cᵢₒ)-(alkyle en C₂ à C₈), (polyalkoxy en C₁ à C₈)-(alkyle en C₂ à C₈), (alkylthio en C₁ à C₁₀)-(alkyle en C₂ à C₈), cycloalkyle à noyau de 3 à 8 atomes, qui peut être interrompu par de l'oxygène et/ou du soufre,
B est de l'hydrogène, un groupe alkyle en C₁ à C₁₂ linéaire ou ramifié, un groupe alkoxyalkyle en C₁ à C₈,
ou dans laquelle
A et B forment, conjointement avec l'atome de carbone auquel ils sont liés, un noyau saturé ou non saturé de 3 à 8 chaînons qui peut être interrompu par de l'oxygène et/ou du soufre et être substitué, le cas échéant, par un groupe alkyle éventuellement halogéné, alkoxy, phényle et un halogène,
R représente les groupes 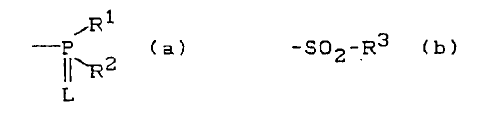 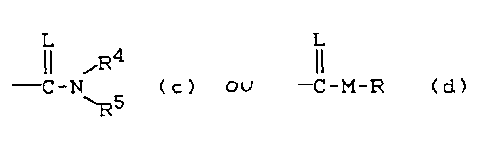 dans lesquels
L et M représentent chacun de l'oxygène ou du soufre, L et M ne pouvant pas représenter simultanément de l'oxygène,
R¹, R² et R³ représentent, indépendamment les uns des autres, un groupe, éventuellement substitué par un halogène, alkyle en C₁ à C₈, alkoxy en C₁ à C₈, alkylamino en C₁ à C₈, di-(alkyle en C₁ à C₈)-amino, alkylthio en C₁ à C₈, alcénylthio en C₂ à C₅, alcynylthio en C₂ à C₅, cycloalkylthio en C₃ à C₇, un groupe phényle, benzyle, phénoxy ou phénylthio éventuellement substitué par un radical halogéno, nitro, cyano, alkoxy en C₁ à C₄, halogénalkoxy en C₁ à C₄, alkylthio en C₁ à C₄, halogénalkylthio en C₁ à C₄, alkyle en C₁ à C₄, halogénalkyle en C₁ à C₄,
R⁴ et R⁵ représentent, indépendamment l'un de l'autre, un groupe, éventuellement substitué par un halogène, alkyle en C₁ à C₂₀, alkoxy en C₁ à C₂₀, alcényle en C₂ à C₈, (alkoxy en C₁ à C₂₀)-(alkyle en C₁ à C₂₀), un groupe phényle éventuellement substitué par un radical halogéno, halogénalkyle en C₁ à C₂₀, alkyle en C₁ à C₂₀ ou alkoxy en C₁ à C₂₀, un groupe benzyle éventuellement substitué par un radical halogéno, alkyle en C₁ à C₂ₒ, halogénalkyle en C₁ à C₂₀ ou alkoxy en C₁ à C₂₀ ou forment ensemble un noyau alkylène en C₂ à C₆ éventuellement interrompu par de l'oxygène,
R⁶ est un groupe alkyle en C₁ à C₂₀ éventuellement substitué par un halogène, qui peut être interrompu par de l'oxygène, un groupe phényle éventuellement substitué par un radical halogéno, halogénalkyle en C₁ à C₂₀, alkoxy en C₁ à C₂₀, un groupe benzyle éventuellement substitué par un radical halogéno, halogénalkyle en C₁ à C₂₀ ou alkoxy en C₁ à C₂₀, un groupe alcényle en C₂ à C₈ ou un groupe alcynyle en C₂ à C₅,
caractérisé en ce que, pour obtenir des 3-arylpyrrolidine-2,4-diones substituées de formule (la) 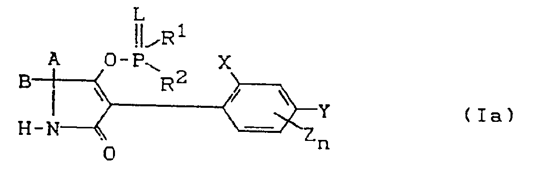 dans laquelle
A, B, L, X, Y, Z, R¹, R² et n ont la définition indiquée dans la revendication 1,
A) on fait réagir des 3-arylpyrrolidine-2,4₋diones de formule (II) ou leurs énols 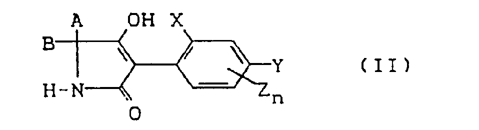 dans laquelle
A, B, X, Y, Z et n ont la définition indiquée ci-dessus,
avec des composés de phosphore de formule générale (III) 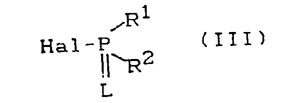 dans laquelle
L, R¹ et R² ont la définition indiquée ci-dessus et
Hal représente un halogène, en particulier le chlore et le brome,
le cas échéant en présence d'un diluant, éventuellement en présence d'un accepteur d'acide et, le cas échéant en présence d'un catalyseur de transfert de phase,
B) ou bien pour obtenir des composés de formule (Ib) 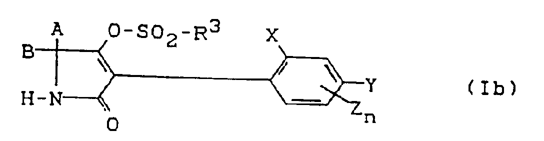 dans laquelle
A, B, X, Y, Z, R³ et n ont la définition indiquée dans la revendication 1,
on fait réagir des composés de formule (II) 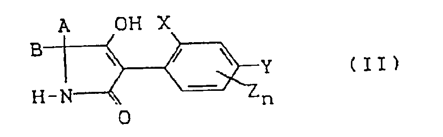 dans laquelle
A, B, X, Y, Z et n ont la définition indiquée ci-dessus
avec des chlorures d'acide sulfonique de formule générale (IV) 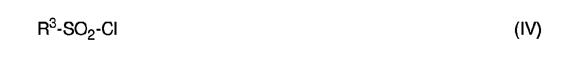 dans laquelle
R³ a la définition indiquée ci-dessus
le cas échéant en présence d'un diluant et en la présence éventuelle d'un accepteur d'acide,
C) ou bien pour obtenir des composés de formule (Ic) 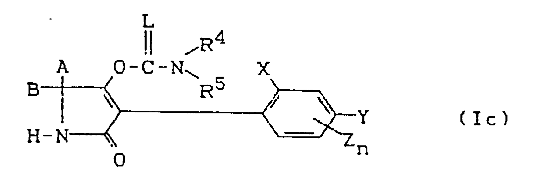 dans laquelle
A, B, L, X, Y, Z, R⁴, R⁵ et n ont la définition indiquée dans la revendication 1,
on fait réagir des composés de formule (II) 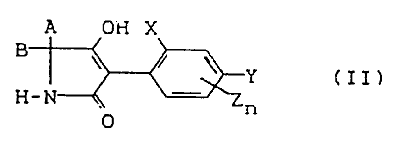 dans laquelle
A, B, X, Y, Z et n ont la définition indiquée ci-dessus
soit
a) avec des isocyanates de formule générale (V) 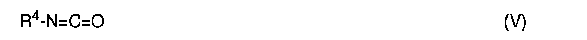 dans laquelle
R⁴ a la définition indiquée ci-dessus
le cas échéant en présence d'un diluant et en la présence éventuelle d'un catalyseur,
soit
ß) avec des chlorures d'acide carbamique ou des chlorures d'acide thiocarbamique de formule générale (VI) 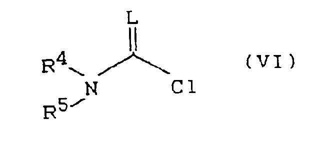 dans laquelle
L, R⁴ et R⁵ ont la définition indiquée ci-dessus,
le cas échéant en présence d'un diluant et en la présence éventuelle d'un accepteur d'acide,
D) ou bien pour obtenir des composés de formule (Id) 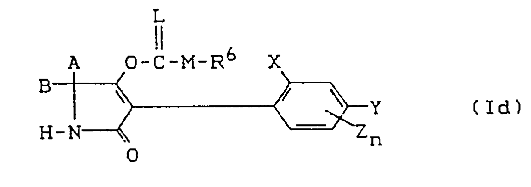 dans laquelle
A, B, L, M, R⁶, X, Y, Z et n ont la définition indiquée dans la revendication 1,
on fait réagir des composés de formule (II) 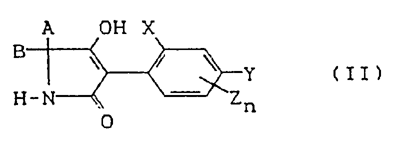 dans laquelle
A, B, X, Y, Z et n ont la définition indiquée ci-dessus,
soit
a) avec des esters d'acide chloromonothioformique, des thioesters d'acide chloroformique ou des esters d'acide chiorodithioformique de formule générale (VII) 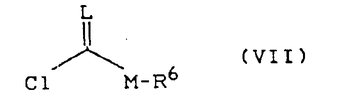 dans laquelle
L, M, R⁶ ont la définition indiquée ci-dessus,
le cas échéant en présence d'un diluant et en la présence éventuelle d'un accepteur d'acide, soit ß) avec du sulfure de carbone, puis des halogénures d'alkyle de formule générale (VIII) 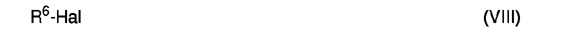 dans laquelle
R⁶ a la définition indiquée ci-dessus et
Hal représente du chlore, du brome, de l'iode.

2. Procédé de production de dérivés substitués de 3-arylpyrrolidine-2,4-diones suivant la revendication 1 pour l'obtention de composés de formules (la) à (Id) 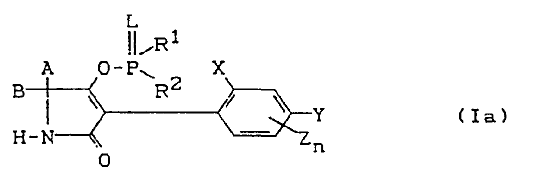 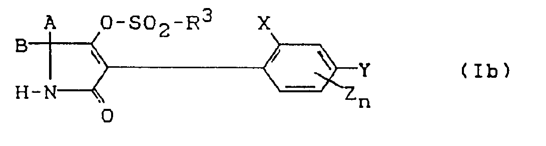 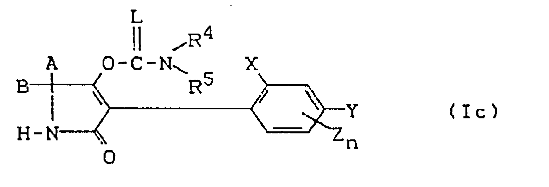 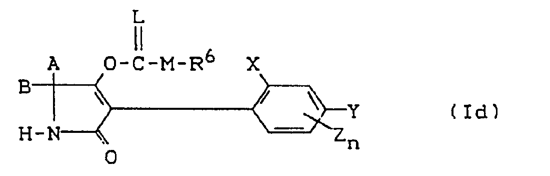 dans lesquelles
A, B, L, M, X, Y, Zₙ, R¹, R², R³, R⁴, R⁵ et R⁶ ont la définition indiquée dans la revendication 1.

3. Procédé de production de dérivés substitués de 3-arylpyrrolidine-2,4-diones suivant la revendication 1, pour l' obtention de composés de formule (I) suivant la revendication 1, dans laquelle
X est un groupe alkyle en C₁ à C₄, un halogène, un groupe alkoxy en C₁ à C₄,
Y est de l'hydrogène, un groupe alkyle en C₁ à C₆, un halogène, un groupe alkoxy en C₁ à C₄, halogénalkyle en C₁ ou C₂,
Z est un groupe alkyle en C₁ à C₄, un halogène, un groupe alkoxy en C₁ à C₄,
n est un nombre de 0 à 3,
A est de l'hydrogène ou un groupe, éventuellement substitué par un halogène, alkyle en C₁ à C₁₀ linéaire ou ramifié, alcényle en C₃ à C₆, alcynyle en C₃ à C₆, (alkoxy en C₁ à C₈)-(alkyle en C₂ à C₆), (polyalkoxy en C₁ à C₆)-(alkyle en C₂ à C₆), (alkylthio en C₁ à C₈)-(alkyle en C₂ à C₆), cycloalkyle à noyau de 3 à 7 atomes, qui peut être interrompu par 1 ou 2 atomes d'oxygène et/ou de soufre,
B est de l'hydrogène, un groupe alkyle en C₁ à C₁₀ linéaire ou ramifié, alkoxyalkyle en C₁ à C₆,
ou dans laquelle
A et B forment, conjointement avec l'atome de carbone auquel ils sont liés, un noyau saturé ou non saturé de 3 à 7 chaînons qui peut être substitué par un radical alkyle en C₁ à C₆, alkoxy en C₁ à C₆, halogénalkyle en C₁ à C₃, halogénalkoxy en C₁ à C₃, fluoro, chloro et qui peut être interrompu par de l'oxygène et/ou du soufre,
R représente les groupes 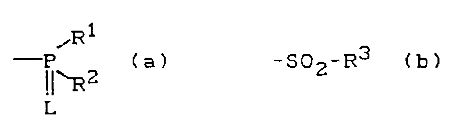 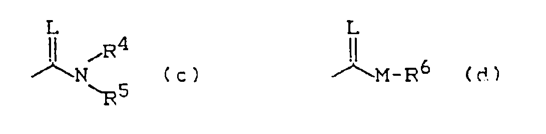 dans lesquels
L et M représentent chacun de l'oxygène ou du soufre, L et M ne représentant pas simultanément de l'oxygène,
R¹, R² et R³ représentent, indépendamment les uns des autres, un groupe, éventuellement substitué par un halogène, alkyle en C₁ à C₆, alkoxy en C₁ à C₆, alkylamino en C₁ à C₆, di-(alkyle en C₁ à C₆)-amino, alkylthio en C₁ à C₆, alcénylthio en C₃ ou C₄, alcynylthio en C₂ à C₄, cycloalkylthio en C₃ à C₆, un groupe phényle, benzyle, phénoxy ou phénylthio éventuellement substitué par un radical fluoro, chloro, bromo, nitro, cyano, alkoxy en C₁ à C₃, halogénalkoxy en C₁ à C₃, alkylthio en C₁ à C₃, halogénalkylthio en C₁ à C₃, alkyle en C₁ à C₃, halogénalkyle en C₁ à C₃,
R⁴ et R⁵ représentent, indépendamment l'un de l'autre, un groupe, éventuellement substitué par un halogène, alkyle en C₁ à C₂₀, alkoxy en C₁ à C₂₀, alcényle en C₂ à C₈, (alkoxy en C₁ à C₂ₒ)-(alkyle en Ci à C₂₀), un groupe phényle éventuellement substitué par un radical halogéno, halogénalkyle en Ci à C₅, alkyle en C₁ à C₅ ou alkoxy en C₁ à C₅, un groupe benzyle éventuellement substitué par un radical halogéno, alkyle en C₁ à C₅, halogénalkyle en C₁ à C₅ ou alkoxy en C₁ à C₅,
R⁶ est un groupe alkyle en C₁ à C₂₀ éventuellement substitué par un halogène, qui peut être interrompu par de l'oxygène, un groupe phényle éventuellement substitué par un radical halogéno, halogénalkyle en C₁ à C₅, alkoxy en C₁ à C₅, un groupe benzyle éventuellement substitué par un radical halogéno, halogénalkyle en C₁ à C₅ ou alkoxy en Ci à C₅,
ainsi que les formes énantiomères pures de composés de formule (I).

4. Compositions insecticides, acaricides et herbicides, caractérisées par une teneur en au moins un dérivé de 3-aryl- pyrrolidine-2,4-dione de formule (I).

5. Procédé pour combattre des insectes et/ou des acariens et/ou des mauvaises herbes, caractérisé en ce qu'on fait agir des dérivés de 3-arylpyrrolidine-2,4-diones de formule (I) sur des insectes et/ou des acariens et/ou des mauvaises herbes et/ou leur milieu.

6. Utilisation de dérivés de 3-arylpyrrolidine-2,4-diones de formule (I) pour combattre des insectes et/ou des acariens et/ou des mauvaises herbes.

7. Procédé de préparation de compositions insecticides et/ou acaricides et/ou herbicides, caractérisé en ce qu'on mélange des dérivés de 3-arylpyrrolidine-2,4-diones de formule (I) avec des diluants et/ou des agents tensio-actifs.
